# EUROPEAN PATENT SPECIFICATION

(11) **EP 2 548 452 B1**
(45) Date of publication and mention of the grant of the patent: **14.05.2014**
(21) Application number: 12005961.3
(22) Date of filing: 19.11.2007
(51) Int. Cl.: A23L 1/236, A23G 4/20

(54) **Confectionery compositions uncluding an elstomeric component and a saccharide component**
Konfektzusammensetzung mit Elastomerbestandteil und einem Saccharidbestandteil
Compositions de confiserie incluant un composant élastomère et un composant saccharide

(30) Priority: 29.11.2006 US 867690 P
(43) Date of publication of application: 23.01.2013
(62) Divisional of application: 07864591.8
(73) Proprietor: Intercontinental Great Brands LLC, East Hanover, NJ 07936 (US)
(72) Inventor: Kabse, Kishor, Morris Plains New Jersey 07950 (US); Allison, Scott B., Stamford CT Connecticut 06903 (US); Kramer Colleen M., Ho-ho-kus New Jersey 07423 (US); McCormick, Demetrius Torino, Clinton New Jersey 08809 (US); Cotton, Gerald, Sparta New Jersey 07871 (US); Boghani, Navroz, Flanders New Jersey 07836 (US); Gebreselassie, Petros, Whitehouse Station NJ 08889 (US); Robinson, Mary, Sparta New Jersey 07871 (US); Schmitz, Kristen A., Jersey City New Jersey 07302 (US); Cooper, Janle M., Lafayette New Jersey 07848 (US); Constance, Patricia Reinhart, Wyckoff New Jersey 07481-3017 (US); Amarista, Jose, Morristown New Jersey 07960 (US)
(74) Representative: Wilson Gunn

(56) References cited:
- EP-A2- 0 370 296
- EP-A2- 1 707 058
- US-A- 2 460 698
- US-A- 4 741 905
- US-A- 5 376 389
- US-B2- 6 866 876

## Description

### BACKGROUND OF THE INVENTION

### FIELD OF THE INVENTION

The present invention relates to confectionery compositions including a saccharide component and a chewing gum base.

### DESCRIPTION OF THE RELATED ART

The present invention relates to the manufacture of confectionery compositions including saccharide including common sugars, such as sucrose, glucose etc, and/or polyols such as maltitol, erythritol, and isomalt, and a chewing gum base. The compositions may have or may provide long lasting characteristics and/or variable textures. Optionally, components that create multi-modal effects are included in different portions of confectionery compositions.

Some confectionery compositions where the finished product is formed by combining saccharide syrups with chewing gum bases are known. For example, United States Patent Number 4,741,905 discloses a chewing confectionery candy confection product produced from a process that combines a cooked sorbitol syrup with confectionery base. However, these compositions result in confectionery products that lack long lasting sensory characteristics and that have a narrow range of texture characteristics. Furthermore, these compositions have not been used to create multi-modal effects. Therefore, a need exists for confectionery compositions including saccharide syrups and elasomeric components that demonstrate long lasting sensory attributes, offer a range of texture attributes, and/or provide multi-modal effects.

### SUMMARY OF THE INVENTION

It has been discovered that the mixture of a saccharide component and chewing gum having a number of advantages in terms of compositional flexibility, uses, and processing not described previously.

Accordingly, one embodiment of the present invention is to provide a method of manufacturing an edible composition comprising a saccharide portion and a chewing gum base. The composition may also include a high intensity sweetener, other actives and/or functional components in various portions of the composition, and which may be encapsulated.

In one embodiment of the invention, it has also been discovered that confectioneries composed of a mixed product having a gum base portion and a saccharide portion are not limited in the amount of coating that can be applied. Therefore, in one embodiment, a coated confectionery composed of a mixed product having a gum base portion and a saccharide component has a coating in an amount such that at least all or a part of the confectionery is coated in an amount of 0.5% by weight. In another embodiment, coated confectionery composed of a mixed product having a gum base portion and a saccharide component can be coated in an amount of at least 50% by weight.

In another embodiment of the invention, it has been discovered that there is no particular limit to which gum base can be used in candy gum confectionery compositions and, in particular, that an optimal range of gum base is from 8% - 20% by weight while still presenting the consumer with a suitable product that delivers a satisfying chew experience at a significant cost savings on a per piece basis.

In another embodiment of the invention, it has been found that confectioneries containing a mixture of gum base and saccharide (e.g., hard and/or soft candies) are easily manipulatable for forming a variety of shaped objects with the confectionery while providing the consumer with the pleasure of a chewing gum experience. Accordingly, in one aspect of the present invention is to provide a shaped edible composition comprising a saccharide component with a chewing gum base.

In another embodiment of the invention, it has been found that center edible cores can be increased substantially compared to traditional center fill gums. This product as the advantage of providing more core material that is desired by the consumer. Also, coupled with the unique organoleptic properties of the outer candy-gum, this new product provides multiple sensory effects, such as crunch, chew and bursts of flavor.

In another embodiment of the invention, it has been found that confectioneries composed of a mixture of gum base with a saccharide component can be simply made into granulated form without the need of expensive and time-consuming processes such as cryogenics. Accordingly, one aspect of the present invention is a granulated composition having a mixture of gum base with a saccharide component. Another aspect of the present invention is a method of manufacturing such a composition by mixing the gum base with a saccharide component; and after the product has cooled, pulverizing the composition into granules.

In another embodiment of the invention, a confectionery in which chewing gum base is mixed with a saccharide component can be gasified and provides the additional advantage of providing a pleasing chewing experience coupled with the sensation of gas release from the matrix of the confectionery.

Furthermore, in some embodiments, there is provided a packaged gum product that includes an edible composition comprising a saccharide portion and a chewing gum base, said package having indicia placed on an outer surface, said indicia being indicative of the product contained therein.

In some embodiments, there is provided a packaged gum product that includes a coated confectionery composed of a mixed product having a gum base portion and a saccharide component has a coating in an amount such that at least all or a part of the confectionery is coated in an amount of 0.5% by weight, said package having indicia placed on an outer surface, said indicia being indicative of the product contained therein.

In another embodiment, there is provided a packaged gum product that includes coated confectionery composed of a mixed product having a gum base portion and a saccharide component has a coating in an amount such that at least all or a part of the confectionery is coated in an amount of at least 50% by weight, said package having indicia placed on an outer surface, said indicia being indicative of the product contained therein.

In another embodiment, there is provided a packaged gum product that includes a confectionery composed of a mixed product having a gum base portion and a saccharide component gum base is from 8% - 20% by weight, said package having indicia placed on an outer surface, said indicia being indicative of the product contained therein.

In another embodiment, there is provided a packaged gum product that includes a confectionery containing a mixture of gum base and saccharide (e.g., hard and/or soft candies) formed into at least one regular or irregular shape, said package having indicia placed on an outer surface, said indicia being indicative of the product contained therein.

In another embodiment, there is provided a packaged gum product that includes a confectionery containing a mixture of gum base and a saccharide component further including a center-fill, said package having indicia placed on an outer surface, said indicia being indicative of the center-filled product contained therein.

In another embodiment, a confectionery containing a mixture of gum base and a saccharide component further including a center-fill, said confectionery providing multiple sensory effects, such as crunch, chew and bursts of flavor, said package having indicia placed on an outer surface, said indicia being indicative of the multiple sensory effects provided by the product contained therein.

In another embodiment, a granulated confectionery containing a mixture of gum base and a saccharide component, said package having indicia placed on an outer surface, said indicia being indicative of the granulated confectionery contained therein.

In another embodiment, an edible composition containing a granulated confectionery composed of mixture of gum base and a saccharide component, wherein the granulated confectionery coats at least a part of the edible composition, said package having indicia placed on an outer surface, said indicia being indicative of the product contained therein.

In another embodiment, an edible composition containing a granulated confectionery composed of mixture of gum base and a saccharide component, wherein the granulated confectionery being partly or wholly contained in the edible composition, said package having indicia placed on an outer surface, said indicia being indicative of the product contained therein.

In another embodiment, an edible composition containing a gasified confectionery composed of mixture of gum base and a saccharide component, said package having indicia placed on an outer surface, said indicia being indicative of the gasified product contained therein.

### BRIEF DESCRIPTION OF THE DRAWINGS

A more complete appreciation of the invention and many of the attendant advantages thereof will be readily obtained as the same becomes better understood by reference to the following detailed description when considered in connection with the accompanying drawings, wherein:
Figure 1 is one embodiment for manufacturing a confectionery composition according to the present invention.
Figure 2 is a second embodiment for manufacturing a confectionery composition according to the present invention.
Figure 3 is a third embodiment for manufacturing a confectionery composition according to the present invention.
Figure 4 is a fourth embodiment for manufacturing a confectionery composition according to the present invention.
Figure 5 is a fifth embodiment for manufacturing a confectionery composition according to the present invention.
Figure 6 is a sixth embodiment for manufacturing a confectionery composition according to the present invention.
Figure 7 is a seventh embodiment for manufacturing a confectionery composition according to the present invention.
Figure 8 is an eighth embodiment for manufacturing a confectionery composition according to the present invention.
Figure 9 is a ninth embodiment for manufacturing a confectionery composition according to the present invention.
Figure 10 is a tenth embodiment for manufacturing a confectionery composition according to the present invention.
Figure 11 is an eleventh embodiment for manufacturing a confectionery composition according to the present invention.
Figure 12 is a twelfth embodiment for manufacturing a confectionery composition according to the present invention.
Figure 13 is a thirteenth embodiment for manufacturing a confectionery composition according to the present invention.
Figure 14 is fourteenth embodiment for manufacturing a confectionery composition according to the present invention.
Figure 15 is a perspective view of a first embodiment of the packaging assembly of the present invention.
Figure 16 is a perspective view of a second embodiment of the packaging assembly of the present invention.
Figure 17 is a perspective view of a third embodiment of a package assembly of the present invention.

Like reference symbols in various drawings indicate like elements.

### DETAILED DESCRIPTION OF THE PREFERRED EMBODIMENTS

The unique ability to combine various confectionery and gum products into a single product provides a number of advantages in terms of manufacturing flexibility, product design, as well as consumer experience when using the product. In particular, by coating the types of confectioneries described herein, one can control the color and/or gloss, provide a product that has longer lasting consuming experience and differing textures while the consumer is enjoying the product. Additionally, the placement of some or all of certain flavor and/or sweeteners in the coating can result in a faster release of the flavor sensed by the consumer, e.g., providing an initial burst of flavor and/or sweetener.

In addition, in certain embodiments, the combination of saccharide component with gum base has flow properties similar to candies and may have glass transistion temperatures greater than 38°C. Thus, the confectioneries of the present invention have the added advantage of being more stable than traditional chewing gums, e.g., in a car, pocket or beach without significantly softening.

In another advantageous aspect of the present invention, the addition of gum base to the saccharide portion can act as a barrier layer and/or control graining of the saccharide portion (e.g., sugar crystallization). In addition, the gum base component can act to protect temperature sensitive ingredients that could be introduced into the confectionery via the gum base composition at a lower temperature than is used to make the saccharide portion.

In another advantageous aspect of the present invention, the saccharide portion can improve the processiblity of the gum base portion by imparting a less sticky, less plastic and/or more rigid texture when compared to a gum base not mixed with the saccharide portions.

Confectionery products are often consumed and enjoyed for their sensory characteristics including taste and texture attributes. Confectionery products can also be used to provide multi-modal effects and to deliver functional ingredients that provide consumer desired benefits. A prized attribute of confectionery products often is long lasting taste. Another desirable attribute is the product's texture profile including initial bite and hardness/softness over time. It can be desirable to provide consumers with interesting textures including those that provide a variety of textures such as an initial crispy texture followed by a soft chewing texture. Similarly, it can be desirable to provide consumers with products that provide a texture change or transformation such as textures that have an initial hardness similar to hard candy but then change to a chewy texture similar to chewing gum. However, economically producing confectionery products with interesting textures and long lasting sensory attributes remains a challenge because the technologies can be cost prohibitive. It has been found that confectionery compositions and processes allowing the use of confectionery equipment can alleviate the cost constraints through lower capital investment requirements thus making inclusion of long lasting sensory technologies possible. The result can be economically viable confectionery products with interesting textures and acceptable long lasting technology. A further finding has been that the long lasting technologies needed for confectionery compositions and processes that use confectionery equipment must be tailored to the demands of those compositions and processes. Yet another finding has been that incorporation of components in different portions of the confection can provide multi-modal effects.

As used herein, "confectionery composition" (sometimes refered to herein as a "candy gum") includes, but are not limited to, a mixture of a saccharide portion (sometimes referred to as a "candy portion," "candy component," "candy composition," saccharide component," and "saccharide composition") and a gum base portion. In other embodiments, which does not include saccharide and gum base portions, the "confectionery composition" is understood according to the knowledge in this field.

As used herein, the saccharide component includes sugars, monosaccharides, disaccharides, trisaccharides, polysaccharides, polyols, hydrogenated sugars as well as their combinations. In this saccharide component, the moisture content of the saccharide component or first portion of the candy gum or confectionery can be manipulated by either heat treating the saccharide component to drive off moisture and/or by adding solids to lower the moisture level.

In some embodiments, confectionery compositions can include a saccharide including sucrose, glucose and other sugars, and/or polyols such as erythritol, maltitol, lactitol, galactitol, isomalt, and combinations thereof and may also contain together with or separately from the saccharide sugar that is combined together or separately with the saccharide. The sugar used herein is those that are commonly used in the confectionery field and, in particular, for making hard and soft or chewy candies. For example, sucrose, glucose, fructose and maltose can be used. Various combinations of these can also be used.

In other embodiments, confectionery compositions can also include additional components such as sweeteners, functional ingredients, and combinations thereof. In still other embodiments, such confectionery compositions with additional components can include encapsulated additional components, unencapsulated additional components, or both. The encapsulated and unencapsulated additional components can be included in the saccharide portion, the elastomeric or gum base component, or both.

Additionally, in some embodiments, confectionery compositions include delivery systems. Such delivery systems can be included in the saccharide portion, the Elastomeric or gum base component, or both. In some embodiments, the delivery systems can have tensile strengths of at least 6,500 psi. In some embodiments, the delivery sytems can have water retentions of less than 15%.

In some embodiments, confectionery compositions can include texture modifying components. Such texture modifying components can include, but are not limited to, particulate materials, hydrophilic materials, flavoring materials, or combinations thereof.

The individual pieces may form a variety of shapes including pellet, tablet, ball, pillow, chunk, stick, lollipop, and slab, among others. Further, in some embodiments, a confectionery composition can be in a particulate form. For example, in some embodiments, grinding the confectionery composition can create a particulate form. In still other embodiments, the grinding operation proceeds under ambient conditions. In some embodiments, a confectionery composition in particulate form is in a compressible form. In some embodiments, the particulate form of the confectionery composition can be used as a coating while in other embodiments the compressed particulate form can be coated.

As used herein, the terms "first portion" and "saccharide" or "candy portion" are used interchangeably to refer to the portion of the compositions comprising sugars, and/or saccharides and other optional ingredients.

As used herein, the terms "second portion" and "elastomeric portion" are used interchangeably to refer to a portion of the compositions comprising water insoluble polymers and other optional ingredients. In some embodiments, the second portion may contain, but is not limited to, elastomers, bulking agents, waxes, elastomer solvents, emulsifiers, plasticizers, fillers, and mixtures thereof.

As used herein, the term "gum base" refers to water insoluble material(s) and can include, but is not limited to, elastomers, bulking agents, waxes, elastomer solvents, emulsifiers, plasticizers, fillers, and mixtures thereof.

As used herein, the term "confectionery composition" and "confection" and "candy gum" are used interchangeably to refer to the combination of the at least two portions of elastomer and saccharide portions discussed above.

As used herein, the term "delivery system" includes an encapsulating material and at least one ingredient encapsulated with the encapsulating material. In some embodiments, a delivery system may include multiple ingredients, multiples layers or levels of encapsulation, and/or one or more other additives. A delivery system may be an ingredient or component in a confectionery composition. In some embodiments, the one or more ingredients and an encapsulating material in the delivery system may form a matrix. In some embodiments, the encapsulating material may completely coat or cover the one or more ingredients or form a partial or complete shell, cover, or coating around the one or more ingredients.

As used herein, the term "tensile strength" includes the maximum stress a material subjected to a stretching load can withstand without tearing. A standard method for measuring tensile strength of a given substance is defined by the American Society of Testing Materials in method number ASTM-D638.

As used herein, the term "encapsulating material" includes any one or more water insoluble polymers, co-polymers, or other materials capable of forming a coating, shell, or film as a protective barrier or layer around one or more ingredients and/or capable of forming a matrix with the one or more ingredients. In some embodiments, the encapsulating material may completely surround, coat, cover, or enclose an ingredient. In other embodiments, the encapsulating material may only partially surround, coat, cover, or enclose an ingredient.

As used herein the transitional term "comprising," (also "comprises," etc.) which is synonymous with "including," "containing," or "characterized by," is inclusive or openended and does not exclude additional, unrecited elements or method steps, regardless of its use in the preamble or the body of a claim.

As used herein, the terms "bubble gum" and "chewing gum" are used interchangeably and are both meant to include any confectionery compositions.

As used herein, the term "ingredient" and the term "component" are used interchangeably to describe any additive, fixing, substance, material, agent, active, element, or part that may be included in the confectionery compositions of some embodiments.

As used herein, the term "duality" or "dual perception" refers to the perception by an individual of two characteristics that are complementary to each other, opposed to each other, i.e., distinct, or different in intensity from each other. The dual characteristics may be flavors, sensations, tastes or functionalities. Flavors, sensates, tastants and functional agents also may include compounds that potentiate each of these types of components.

The term "multi-modality" refers to the perception by an individual of at least two characteristics that are complementary, opposed, i.e., distinct, or different in intensity from one another. The multi-modal characteristics may be flavors, sensations, tastes, functionalities or combinations thereof. Flavors, sensates, tastants and functional agents also may include compounds that potentiate each of these types of components. The term "multi-modality" is broader than and encompasses the term "duality" in that it includes embodiments that have a dual perception, as well as embodiments that have more than one dual perception. For example, multi-modality may encompass two different dualities in one confectionery composition, such as dual flavors and dual tastes.

The term "complementary" refers to components that are in the same or similar family, for example, components that are in the same or similar flavor family, such as the mint family or the fruit family; components that are in the same or similar sensation family, such as the cooling family, the warming family or the tingling family; components that are in the same or similar taste family, such as the sweetener family, the sour family, the bitter/astringent family, the salty family, the umami family or the kokumi family; and components that are in the same or similar functional family, such as the breath freshening family or other functional families provided in Table 2 herein. The terms "family" and "type" are used interchangeably herein when referring to multi-modality components.

The term "opposed" means distinctly different components, for example, components that are from different families, such as a component in the flavor family and a component in the taste family.

The term "different in intensity" means that the at least two components that form the duality or multi-modality may be the same component but create the duality or multi-modality by being present in different amounts or by being encapsulated thereby providing a different intensity from one another. This different intensity can be formed by the component being in different amounts from one portion of the confectionery to another, or from being released at one rate in one portion versus being released at another rate in another portion. The different intensity can also be formed by the component interacting with the composition of a portion to provide a different intensity such as when a component has a low affinity for a portion's composition and therefore releases fully to provide a higher intensity at an amount lower than the amount needed to provide that same intensity from a portion where the component has a greater affinity for the portion's composition and is therefore less fully released.

### Saccharide Portion

The saccharide portion can include starches, fats, and hydrocolloids. As described in more detail below in the "Texture Modification" section, in some embodiments, the composition of this portion is influenced by the composition of the elastomeric portion.

In some embodiments, the saccharide poritions include saccharides with low hygroscopicity and low tendency to crystallize such that when combined with elastomeric, the resultant chewing confectionery products demonstrate desired shelf life stability. Examples of such saccharide poritions include sugar/corn syrup blends, isomalt, erythritol, maltitol, and combinations of these saccharides. In some embodiments, the tendency of the saccharides to crystallize is exploited by seeding the saccharide portion so that it will crystallize over time to adjust the texture from a harder texture during manufacture to a softer texture at the time of consumption.

In some embodiments, a saccharide portion can itself include confectionery compositions. Such confectionery compositions can include, but are not limited to, chocolate, compound coating, carob coating, cocoa butter, butter fat, hydrogenated vegetable fat, illipe butter, fondant including fondant-based cremes, fudge, frappe, caramel, nougat, compressed tablet, candy floss (also known as cotton candy), marzipan, hard boiled candy, gummy candy, jelly beans, toffees, taffy, jellies including pectin-based gels, jams, preserves, butterscotch, nut brittles or croquant, candied fruit, marshmallow, pastilles, pralines or nougats, flour or starch confectionery, truffles, nonpareils, bon bons, after-dinner mints, fourres, nut pastes, peanut butter, chewing gum, kisses, angel kisses, montelimart, nougatine, fruit chews, Turkish delight, hard gummies, soft gummies, starch jellies, gelatin jellies, agar jellies, persipan, coconut paste, coconut ice, lozenges, cachous, crème paste, dragees, sugared nuts, sugared almonds, comfits, aniseed balls, licorice, licorice paste, chocolate spreads, chocolate crumb, and combinations thereof.

In some embodiments, the saccharide portion may contain ingredients well known in the confectionery arts, such as flavoring agents, sweetening agents, and the like, and mixtures thereof, as described above. In addition to confectionery additives, the saccharide portion may also contain pharmaceutical additives such as medicaments, breath fresheners, vitamins, minerals, caffeine, phytochemicals, nutraceuticals, fruit juices, and the like, and mixtures thereof. The confectionery and pharmaceutical agents may be used in many distinct physical forms well known in the art to provide an initial burst of sweetness and flavor and/or therapeutic activity or a prolonged sensation of sweetness and flavor and/or therapeutic activity. Without being limited thereto, such physical forms include free forms, such as spray dried, powdered, and beaded forms, and encapsulated forms, and mixtures thereof. Specific examples of suitable additional components include taurine, guarana, Echinacea, ginseng, green tea, white tea, vitamins, Actizol^{™}, chlorophyll, Recaldent^{™} tooth remineralization technology, and Retsyn^{™} breath freshening technology.

The following are some examples of various compositions which can be used as the component including saccharide. All values are % by weight of the total composition.

### Hard Candy Formulas

### Sugar formula

| **Ingredient** | **Concentration** |
|---|---|
| Bulk Sweeteners (Sucrose, Dextrose, Corn Syrup, | 80-100 |
| etc...) | |
| Fat | 0-15 |
| Emulsifier | 0-5 |
| Flavor | 0-5 |
| Acid | 0-10 |
| Intense sweetener | 0-1 |

### Sugar Free formula

| **Ingredient** | **Concentration** |
|---|---|
| Bulk Sweeteners (Sorbitol, Xylitol, Isomalt, Maltitol syrup, etc...) | 80-100 |
| Fat | 0-15 |
| Emulsifier | 0-5 |
| Flavor | 0-5 |
| Acid | 0-10 |
| Intense sweetener | 0-1 |

The ratios of chewing gum or gum base to candy could be in the range: Chewing gum or gum base (25-95%) versus hard candy (75-5%).

### Nougat Formulas

### Sugar formula

| **Ingredient** | **Concentration** |
|---|---|
| Bulk Sweeteners (Sucrose, Dextrose, Corn Syrup, etc...) | 30-90 |
| Starch | 0-75 |
| Fat | 0.1-25 |
| Emulsifier | 0-10 |
| Flavor | 0-20 |
| Acid | 0-25 |
| Intense sweetener | 0-1 |
| Gelling Agent (gelatin, hydrocolloids, etc...) | 0-10 |

### Sugar Free formula

| | |
|---|---|
| | |

| **Ingredient** | **Concentration** |
|---|---|
| Bulk Sweeteners (Sorbitol, Xylitol, Isomalt, Maltitol syrup, etc... | 30-90 |
| Starch | 0-75 |
| Fat | 0.1-25 |
| Emulsifier | 0-10 |
| Flavor | 0-20 |
| Acid | 0-25 |
| Intense sweetener | 0-1 |
| Gelling Agent (gelatin, hydrocolloids, etc...) | 0-10 |

### Chewy Candy Formulas

### Sugar formula

| **Ingredient** | **Concentration** |
|---|---|
| Bulk Sweeteners (Sucrose, Dextrose, Corn Syrup, etc...) | 30-90 |
| Starch | 0-75 |
| Fat | 0.1-25 |
| Emulsifier | 0-10 |
| Flavor | 0-20 |
| Acid | 0-25 |
| Intense sweetener | 0-1 |
| Gelling Agent (gelatin, hydrocolloids, etc...) | 0-10 |

### Sugar Free formula

| **Ingredient** | **Concentration** |
|---|---|
| Bulk Sweeteners (Sorbitol, Xylitol, Isomalt, Maltitol syrup, etc... | 30-90 |
| Starch | 0-75 |
| Fat | 0.1-25 |
| Emulsifier | 0-10 |
| Flavor | 0-20 |
| Acid | 0-25 |
| Intense sweetener | 0-1 |

Nougat formulas and chewy candy formulas differ mainly in the type of gelling agent used. For nougat it is more important to whip the candy instead of forming a gel. Chewy candy can be more of a gel. Therefore in the nougat type formula we used a non-bloom gelatin to create air cells in the candy. The chewy candy used a 200-bloom gelatin which provided a nice gel. The cooking temperature of the nougat and chewy candy are the same.

The ratios of chewing gum or gum base to candy could be in the range: Chewing gum or gum base (50-95%) versus chewy candy/nougat (50-5%).

### Elastomeric Portion

The elastomeric portion, also referred to as a second portion, may include at least one modified release component, as discussed in more detail below. Moreover, in some embodiments, the elastomeric portion may include a component that exhibits modified release properties in combination with the same component in its free, or unmodified, form.

The elastomeric portion may be varied to provide a range of characteristics. For example, in some embodiments, an elastomeric portion can include a level of mineral adjuvant or filler that provides a desired chewing texture and is higher than an elastomeric portion with a lesser amount of filler. In other embodiments, an elastomeric portion can include low melting point fats that provides an unctuous mouthfeel and indulgent chewing experience.

The elastomeric portion may include a gum base and/or other elastomeric materials. The gum base or elastomeric materials may include any component known in the chewing gum art. For example, the elastomeric portion may include elastomers, bulking agents, waxes, elastomer solvents, emulsifiers, plasticizers, fillers and mixtures thereof. The elastomeric portion may comprise from about 5% to about 95%, including from about 30% to about 70% by weight of the confectionery composition piece, such as about 50%.

As discussed herein, in some embodiments, it has been discovered that there a low cost confectionery can be produced while providing the dual sensations of candy and chewing gum consumption. Accordingly, in certain embodiements, the elastomeric or gum base portion may comprise from about 8 to about 20% by weight of the confectionery composition piece including 9, 10, 11, 12, 13, 14, 15, 16, 17, 18, 19 % by weight and all values and ranges therebetween.

The amount of the gum base or elastomeric material which is present in the elastomeric portion may also vary. In some embodiments, the gum base or elastomeric materials may be included in the elastomeric portion in an amount from about 25% to about 100% by weight of the elastomeric portion. A more specific range of gum base or elastomeric materials in some embodiments may be from about 30% to about 75% by weight of the elastomeric portion. Even more specifically, the range may be from about 35% to about 65% or from about 40% to about 50% in some embodiments.

The elastomers (rubbers) employed in the elastomeric portion will vary greatly depending upon various factors such as the type of elastomeric portion desired, the consistency of elastomeric portion desired and the other components used in the elastomeric portion to make the final confectionery product. The elastomer may be any water-insoluble polymer known in the art, and includes those polymers utilized for chewing gums and bubble gums. Illustrative examples of suitable polymers in gum bases include both natural and synthetic elastomers. For example, those polymers which are suitable in elastomeric portion compositions include, without limitation, natural substances (of vegetable origin) such as chicle, natural rubber, crown gum, nispero, rosidinha, jelutong, perillo, niger gutta, tunu, balata, guttapercha, lechi capsi, sorva, gutta kay, and the like, and combinations thereof. Examples of synthetic elastomers include, without limitation, styrene-butadiene copolymers (SBR), polyisobutylene, isobutylene-isoprene copolymers, polyethylene, polyvinyl acetate and the like, and combinations thereof.

Additional useful polymers include: crosslinked polyvinyl pyrrolidone, polymethylmethacrylate; copolymers of lactic acid, polyhydroxyalkanoates, plasticized ethylcellulose, polyvinyl acetatephthalate and combinations thereof.

The amount of elastomer employed in the elastomeric portion may vary depending upon various factors such as the type of elastomer used, the consistency of the elastomeric portion desired and the other components used in the elastomeric portion to make the final confectionery product. In general, the elastomer will be present in the elastomeric portion in an amount from about 10% to about 60% by weight of the elastomeric portion, desirably from about 35% to about 40% by weight.

In some embodiments, the elastomeric portion may include wax. It softens the polymeric mixture and improves the elasticity of the elastomeric portion. When present, the waxes employed will have a melting point below about 60°C, and preferably between about 45°C and about 55°C. The low melting wax may be a paraffin wax. The wax may be present in the elastomeric portion in an amount from about 6% to about 10%, and preferably from about 7% to about 9.5%, by weight of the elastomeric portion.

In addition to the low melting point waxes, waxes having a higher melting point may be used in the elastomeric portion in amounts up to about 5%, by weight of the elastomeric portion. Such high melting waxes include beeswax, vegetable wax, candelilla wax, carnuba wax, most petroleum waxes, and the like, and mixtures thereof.

In addition to the components set out above, the elastomeric portion may include a variety of other ingredients, such as components selected from elastomer solvents, emulsifiers, plasticizers, fillers, and mixtures thereof.

The elastomeric portion may contain elastomer solvents to aid in softening the elastomeric materials. Such elastomer solvents may include those elastomer solvents known in the art, for example, terpinene resins such as polymers of alpha-pinene or beta-pinene, methyl, glycerol and pentaerythritol esters of rosins and modified rosins and gums such as hydrogenated, dimerized and polymerized rosins, and mixtures thereof. Examples of elastomer solvents suitable for use herein may include the pentaerythritol ester of partially hydrogenated wood and gum rosin, the pentaerythritol ester of wood and gum rosin, the glycerol ester of wood rosin, the glycerol ester of partially dimerized wood and gum rosin, the glycerol ester of polymerized wood and gum rosin, the glycerol ester of tall oil rosin, the glycerol ester of wood and gum rosin and the partially hydrogenated wood and gum rosin and the partially hydrogenated methyl ester of wood and rosin, and the like, and mixtures thereof. The elastomer solvent may be employed in the elastomeric portion in amounts from about 2% to about 15%, and preferably from about 7% to about 11%, by weight of the elastomeric portion.

The elastomeric portion may also include emulsifiers which aid in dispersing the immiscible components into a single stable system. The emulsifiers useful in this invention include glyceryl monostearate, lecithin, fatty acid monoglycerides, diglycerides, propylene glycol monostearate, and the like, and mixtures thereof. The emulsifier may be employed in amounts from about 2% to about 15%, and more specifically, from about 7% to about 11%, by weight of the elastomeric portion.

The elastomeric portion may also include plasticizers or softeners to provide a variety of desirable textures and consistency properties. Because of the low molecular weight of these ingredients, the plasticizers and softeners are able to penetrate the fundamental structure of the elastomeric portion making it plastic and less viscous. Useful plasticizers and softeners include lanolin, palmitic acid, oleic acid, stearic acid, sodium stearate, potassium stearate, glyceryl triacetate, glyceryl lecithin, glyceryl monostearate, propylene glycol monostearate, acetylated monoglyceride, glycerine, and the like, and mixtures thereof. Waxes, for example, natural and synthetic waxes, hydrogenated vegetable oils, petroleum waxes such as polyurethane waxes, polyethylene waxes, paraffin waxes, microcrystalline waxes, fatty waxes, sorbitan monostearate, tallow, propylene glycol, mixtures thereof, and the like, may also be incorporated into the elastomeric portion. The plasticizers and softeners are generally employed in the elastomeric portion in amounts up to about 20% by weight of the elastomeric portion, and more specifically in amounts from about 9% to about 17%, by weight of the elastomeric portion.

Plasticizers also include are the hydrogenated vegetable oils and include soybean oil and cottonseed oil which may be employed alone or in combination. These plasticizers provide the elastomeric portion with good texture and soft chew characteristics. These plasticizers and softeners are generally employed in amounts from about 5% to about 14%, and more specifically in amounts from about 5% to about 13.5%, by weight of the elastomeric portion.

Anhydrous glycerin may also be employed as a softening agent, such as the commercially available United States Pharmacopeia (USP) grade. Glycerin is a syrupy liquid with a sweet warm taste and has a sweetness of about 60% of that of cane sugar. Because glycerin is hygroscopic, the anhydrous glycerin may be maintained under anhydrous conditions throughout the preparation of the confectionery composition.

In some embodiments, the elastomeric portion of this invention may also include effective amounts of bulking agents such as mineral adjuvants which may serve as fillers and textural agents. Useful mineral adjuvants include calcium carbonate, magnesium carbonate, alumina, aluminum hydroxide, aluminum silicate, talc, tricalcium phosphate, dicalcium phosphate, calcium sulfate and the like, and mixtures thereof. These fillers or adjuvants may be used in the elastomeric portion in various amounts. The amount of filler, may be present in an amount from about zero to about 40%, and more specifically from about zero to about 30%, by weight of the elastomeric portion. In some embodiments, the amount of filler will be from about zero to about 15%, more specifically from about 3% to about 11%.

A variety of traditional ingredients may be optionally included in the elastomeric portion in effective amounts such as coloring agents, antioxidants, preservatives, flavoring agents, high intensity sweeteners, and the like. For example, titanium dioxide and other dyes suitable for food, drug and cosmetic applications, known as F. D. & C. dyes, may be utilized. An anti-oxidant such as butylated hydroxytoluene (BHT), butylated hydroxyanisole (BHA), propyl gallate, and mixtures thereof, may also be included. Other conventional confectionery additives known to one having ordinary skill in the confectionery art may also be used in the elastomeric portion.

Some embodiments extend to methods of making the confectionery compositions. The manner in which the elastomeric portion components can be performed using standard techniques and apparatus known to those skilled in the art. In a typical method, an elastomer is admixed with an elastomer solvent and/or a plasticizer and/or an emulsifier and agitated for a period of from 1 to 30 minutes. The remaining ingredients, such as the low melting point wax, are then admixed, either in bulk or incrementally, while the elastomeric portion mixture is blended again for 1 to 30 minutes.

The elastomeric portion may include amounts of conventional additives selected from the group consisting of sweetening agents (sweeteners), plasticizers, softeners, emulsifiers, waxes, fillers, bulking agents (carriers, extenders, bulk sweeteners), mineral adjuvants, flavoring agents (flavors, flavorings), coloring agents (colorants, colorings), antioxidants, acidulants, thickeners, medicaments, and the like, and mixtures thereof. Some of these additives may serve more than one purpose. For example, in sugarless confectionery compositions, a sweetener, such as maltitol or other sugar alcohol, may also function as a bulking agent.

The plasticizers, softening agents, mineral adjuvants, waxes and antioxidants discussed above, as being suitable for use in the elastomeric portion, may also be used in the confectionery composition. Examples of other conventional additives which may be used include emulsifiers, such as lecithin and glyceryl monostearate, thickeners, used alone or in combination with other softeners, such as methyl cellulose, alginates, carrageenan, xanthan gum, gelatin, carob, tragacanth, locust bean gum, pectin, alginates, galactomannans such as guar gum, carob bean gum, glucomannan, gelatin, starch, starch derivatives, dextrins and cellulose derivatives such as carboxy methyl cellulose, acidulants such as malic acid, adipic acid, citric acid, tartaric acid, fumaric acid, and mixtures thereof, and fillers, such as those discussed above under the category of mineral adjuvants.

In some embodiments, the elastomeric portion may also contain a bulking agent. Suitable bulking agents may be water-soluble and include sweetening agents selected from, but not limited to, monosaccharides, disaccharides, polysaccharides, sugar alcohols, and mixtures thereof; randomly bonded glucose polymers such as those polymers distributed under the tradename Litesse^{™} which is the brand name for polydextrose and is manufactured by Danisco Sweeteners, Ltd. of 41-51 Brighton Road, Redhill, Surryey, RH1 6YS, United Kingdom; isomalt (a racemic mixture of alpha-D-glucopyranosyl-1,6-mannitol and alpha-D-glucopyranosyl-1,6-sorbitol manufactured under the tradename PALATINIT™ by Palatinit Sussungsmittel GmbH of Gotlieb-Daimler-Strause 12 a, 68165 Mannheim, Germany); maltodextrins; hydrogenated starch hydrolysates; hydrogenated hexoses; hydrogenated disaccharides; minerals, such as calcium carbonate, talc, titanium dioxide, dicalcium phosphate; celluloses; and mixtures thereof.

Suitable sugar bulking agents include monosaccharides, disaccharides and polysaccharides such as xylose, ribulose, glucose (dextrose), lactose, mannose, galactose, fructose (levulose), sucrose (sugar), maltose, invert sugar, partially hydrolyzed starch and corn syrup solids, and mixtures thereof.

Suitable sugar alcohol bulking agents include sorbitol, xylitol, mannitol, galactitol, lactitol, maltitol, erythritol, isomalt and mixtures thereof. Suitable hydrogenated starch hydrolysates include those disclosed in U.S. Pat. No. 4,279,931 and various hydrogenated glucose syrups and/or powders which contain sorbitol, maltitol, hydrogenated disaccharides, hydrogenated higher polysaccharides, or mixtures thereof. Hydrogenated starch hydrolysates are primarily prepared by the controlled catalytic hydrogenation of corn syrups. The resulting hydrogenated starch hydrolysates are mixtures of monomeric, dimeric, and polymeric saccharides. The ratios of these different saccharides give different hydrogenated starch hydrolysates different properties. Mixtures of hydrogenated starch hydrolysates, such as LYCASIN^{®}, a commercially available product manufactured by Roquette Freres of France, and HYSTAR^{®}, a commercially available product manufactured by SPI Polyols, Inc. of New Castle, Delaware, are also useful.

The sweetening agents which may be included in the compositions of some embodiments may be any of a variety of sweeteners known in the art. These are described in more detail in the "Additional Components" section herein below and may be used in many distinct physical forms well-known in the art to provide an initial burst of sweetness and/or a prolonged sensation of sweetness. Without being limited thereto, such physical forms include free forms, such as spray dried, powdered, beaded forms, encapsulated forms, and mixtures thereof.

Desirably, the sweetener is a high intensity sweetener such as aspartame, neotame, sucralose, monatin, and acesulfame potassium (Ace-K).

In general, an effective amount of sweetener may be utilized to provide the level of sweetness desired, and this amount may vary with the sweetener selected. In some embodiments the amount of sweetener may be present in amounts from about 0.001% to about 3%, by weight of the confectionery composition, depending upon the sweetener or combination of sweeteners used. The exact range of amounts for each type of sweetener may be selected by those skilled in the art.

In some embodiments, particularly confectionery composition embodiments, the elastomeric portion may include a specific polyol composition including at least one polyol which is from about 30% to about 80% by weight of said elastomeric portion, and specifically from 50% to about 60%. In some confectionery composition embodiments, such elastomeric portion compositions may have low hygroscopicity. The polyol composition may include any polyol known in the art including, but not limited to maltitol, sorbitol, erythritol, xylitol, mannitol, isomalt, lactitol and combinations thereof. Lycasin^{™} which is a hydrogenated starch hydrolysate including sorbitol and maltitol, may also be used.

The amount of the polyol composition or combination of polyols used in the elastomeric portion will depend on many factors including the type of elastomers used in the elastomeric portion and the particular polyols used. For example, wherein the total amount of the polyol composition is in the range of about 40% to about 65% based on the weight of the elastomeric portion, the amount of isomalt may be from about 40% to about 60% in addition to an amount of sorbitol from about 0 up to about 10%, more specifically, an amount of isomalt may be from about 45% to about 55% in combination with sorbitol from about 5% to about 10% based on the weight of the elastomeric portion.

The polyol composition which may include one or more different polyols which may be derived from a genetically modified organism ("GMO") or GMO free source. For example, the maltitol may be GMO free maltitol or provided by a hydrogenated starch hydrolysate. For the purposes of this invention, the term "GMO-free" refers to a composition that has been derived from process in which genetically modified organisms are not utilized.

Coloring agents may be used in amounts effective to produce the desired color. The coloring agents may include pigments which may be incorporated in amounts up to about 6%, by weight of the confectionery composition. For example, titanium dioxide may be incorporated in amounts up to about 2%, and preferably less than about 1%, by weight of the confectionery composition. The colorants may also include natural food colors and dyes suitable for food, drug and cosmetic applications. These colorants are known as F.D.& C. dyes and lakes. The materials acceptable for the foregoing uses are preferably water-soluble. Illustrative nonlimiting examples include the indigoid dye known as F.D.& C. Blue No.2, which is the disodium salt of 5,5-indigotindisulfonic acid. Similarly, the dye known as F.D.& C. Green No.1 comprises a triphenylmethane dye and is the monosodium salt of 4-[4-(N-ethyl-p-sulfoniumbenzylamino) diphenylmethylene]-[1-(N-ethyl -N-p-sulfoniumbenzyl)-delta-2,5-cyclohexadieneimine]. A full recitation of all F.D.& C. colorants and their corresponding chemical structures may be found in the Kirk-Othmer Encyclopedia of Chemical Technology, 3rd Edition, in volume 5 at pages 857-884, which text is incorporated herein by reference. Additional coloring components are described in the "Additional Components" section hereinbelow.

Suitable oils and fats usable in confectionery compositions include partially hydrogenated vegetable or animal fats, such as coconut oil, palm kernel oil, beef tallow, and lard, among others. These ingredients when used are generally present in amounts up to about 7%, and preferably up to about 3.5%, by weight of the confectionery composition.

Some embodiments may include a method for preparing the improved compositions for the elastomeric portion, including elastomeric materials for both chewing gum and bubble gum compositions. The elastomeric portion compositions may be prepared using standard techniques and equipment known to those skilled in the art. The apparatus useful in accordance with some embodiments comprises mixing and heating apparatus well known in the confectionery manufacturing arts, and therefore the selection of the specific apparatus will be apparent to the artisan.

In some embodiments, the edible composition also contains a high intensity sweetener that can be added to the saccharide portion, the gum base portion or both. In still further embodiments, the high intensity sweetener can be in an encapsulated form, a free form, or both. In some embodiments, the edible composition can include a delivery system as described herein. In some embodiments, the edible composition comprises a texture modifying component. In some embodiments, the texture modifying component can include sorbitol, fat, flavor, or combinations thereof.

In some embodiments, the edible composition can include a center-fill.

In some embodiments, the edible composition can include at least one sensate. In other embodiments, at least one portion of the at least one sensate can be mixed with the saccharide portion, the gum base portion, or both. In still other embodiments, the at least one sensate can be in encapsulated form, in free form, or both:

In some embodiments, the edible composition can include at least one flavor. In other embodiments, at least one portion of the at least one flavor can be mixed with either or both portions. In still other embodiments, the at least one flavor can be in encapsulated form, in free form, or both.

In some embodiments, the edible composition can include at least one functional ingredient. In other embodiments, at least one portion of the at least one functional ingredient can be mixed with either or both portions. In still other embodiments, the at least one functional ingredient can be in encapsulated form, in free form, or both.

In some embodiments, the edible composition can include at least one sweetener. In other embodiments, at least one portion of the at least one sweetener can be mixed with either or both portions. In still other embodiments, the at least one sweetener can be in encapsulated form, in free form, or both.

In some embodiments, the at least two portions that make up the confectionery are visually distinct. In some embodiments, the at least two portions of the confectionery are visually indistinct.

In some embodiments, there is provided an edible composition comprising a saccharide component, a gum base component; a multiple encapsulation sucralose composition, wherein said multiple encapsulation sucralose composition further comprises sucralose, a first encapsulation forming a first layer, and a second encapsulation forming a second layer; wherein the first layer encapsulates the sucralose and the second layer encapsulates the first layer; wherein the first encapsulation coprises polyvinyl acetate and the second encapsulation is selected from the group consisting of gum arabic, gelatin, or combinations thereof; and wherein the multiple encapsulation sucralose is added to the gum base component.

In some embodiments, there is provided an edible composition comprising a saccharide component; a gum base component; and a functional ingredient. In some embodiments, the functional ingredient can be added to the saccharide component or to the gum base component, or to both. In still other embodiments, the saccharide component includes isomalt.

In some embodiments, there is provided an edible composition comprising a saccharide component; a gum base component; a first flavor component; and a second flavor component. In some embodiments, the first flavor component can be added to the saccharide component while the second flavor component can be added to the gum base component.

In some embodiments, the saccharide portion has a moisture content of no more than 2% w/w, and wherein along with the gum base are designed to withstand vigorous mixing without the incorporation of air into the mixture such that a homogeneous mixture of the two portions results. In some embodiments, the chewing gum base is 10% - 90% w/w of the edible composition while in other embodiments, the saccharide is 10% - 90% w/w of the edible composition. In some embodiments, the amounts are selected to provide a desired texture.

In some embodiments, the edible composition has an initial crunch that is the same as the initial crunch of a hard panned confection as measured by sensory testing techniques. In some embodiments, the composition has a surface gloss appearance that is the same as the surface gloss appearance of a hard panned product as measured by optometric equipment.

In some embodiments, at least a portion of the edible composition is in a ground particulate form. In other embodiments, at least a portion of the particulate composition is in compressible form.

In some embodiments, the edible composition includes a first ingredient in the gum base and a second ingredient in the saccharide portion. In some embodiments, the first ingredient is the same as the second ingredient while in other embodiments, the first ingredient is different than the second ingredient. In still other embodiments, the first ingredient and the second ingredient have different intensities as measured by sensory evaluation techniques.The ingredients can include flavor, cooling agent, sensate, sweetener, food acid, functional ingredient, sweetener potentiator, modified release agent, and other ingredients/additives as described herein and/or commonly used in the confectionery and chewing gum fields.

In some embodiments, the gum base portion and the saccharide portion are adjusted to be visually different.

In some embodiments, the first or second functional ingredient is selected from the group comprising breath fresheners, dental care components, actives, herbals, effervescing systems, appetite suppressors, vitamins, micronutrients, mouth moistening components, throat care components, energy boosting agents, concentration boosting agents, and combinations thereof.

In some embodiments, the modified release component includes at least one ingredient selected from the group comprising flavors, sweeteners, sensates, breath fresheners, dental care components, actives, herbals, effervescing systems, appetite suppressors, potentiators, food acids, micronutrients, mouth moistening components, throat care components, and combinations thereof.

In some embodiments, as the saccharide portions are generally more efficient than gum base at delivering the flavor, sweetener and/or other functional ingredients as described herein, it may be particularly advantageous to incorporate such ingredients into the saccharide component instead of the gum base component. This has the particular advantage for high cost and/or low availability ingredients because less of these ingredients can be used to deliver the same amount of ingredient when compared to the amount delivered by an elastomer-containing gum base.

### Dualities and Multi-Modalities

As described above, coated and/or center-filled products in addition to the at least two components of gum base and saccharide create dualities and multi-modalities. Thus, at least two, three, four, five or even more combinations of ingredients can be used to provide such modalities. In some embodiments, the at least two components may be opposed to each other, i.e., distinctly different components. For example, two opposed flavors, such as strawberry and kiwi, may be employed. In some embodiments, the at least two components may be complementary to one another. For example, two mint oils that complement each other, such as peppermint and spearmint, may be employed. In some embodiments, the at least two components may differ in intensity from one another. For example, a single mint oil may be used, but in different amounts or intensities such that an intensity difference exists between the two portions of the mint oil. In some embodiments, the release of the at least two components can be such that a lesser amount can produce a higher intensity. For example, mint oil included in a saccharide portion at an amount lower than a mint oil amount included in an elastomeric portion can produce a higher intensity due to an increased release from the saccharide portion. Combinations of these various modalities can be included in a single product.

The components that create the duality, or multi-modality, may be included in different portions of the confectionery composition and/or in the same portion.

Non-limiting examples of some of the possible physical combinations for providing a duality in a confectionery composition are indicated in Table 1 below. In particular, Table 1 identifies a number of different physical combinations of components that may be employed involving dualities among: (1) distinct components; (2) complementary components; and (3) intensity differences between a single component.

For example, in one embodiment, an edible composition is provided with at least one sensate, at least one functional ingredient, at least one flavor ingredient, at least one sweetening ingredient, or a combination thereof, optionally encapsulated, where in at least a portion of the gum base is a higher lower compared to the saccharide portion.

In another embodiment, an edible composition is provided with at least one sensate, at least one functional ingredient, at least one flavor ingredient, at least one sweetening ingredient, or a combination thereof, optionally encapsulated, where in at least a portion of the gum base in a lower amount compared to the saccharide portion

As referred to in Table 1 and as defined above, the coating composition refers to an outermost portion of the confection, the center-fill composition refers to an innermost portion of the confection, the elastomeric portion composition refers to the water insoluble polymer ingredients and the saccharide portion refers to the saccharide and other optional ingredients. As discussed herein, the center-fill may or may not be completely surrounded by the outer layer and the coating may coat a portion or all of the composition to which it is applied. Table 1 sets forth non-limiting examples of how different ingredients can be distributed in the confection to achieve dualities and/or multi-modalities and should be recognized that in addition to these particular regions of the confection, other ways of achieving modalities can be use, such as ingredients pressed into the outer surface of the product, other ingredients, encapsulated or not, added to the candy portion, the gum portion, and/or during the processing of the candy and gum portions together, multiple enrobings or outer layers, multiple center-fills, multiple laminates or layers, etc.

As used in Table 1, A represents a first component and B represents a second component, which is distinct from the first component. A' represents a second component that is complementary to the first component. 1/n is used to indicate a fractional portion of component A. 1/m is used to indicate a fractional portion of component A that is different from fractional portion 1/n. n*A is used to indicate a multiplicative portion of component A, and m*A indicates a multiplicative portion of component A that is different from multiplicative portion n*A.

**TABLE 1**

| Coating and/or Center-fill Composition | Elastomeric Portion Composition | Saccharide Portion Composition |
|---|---|---|
| (1) Dualities based on differences between separate and distinct components: | | |
| A | B | |
| A | | B |
| | A | B |
| B | A | |
| B | | A |
| | B | A |
| | | |
| 1/n A | B | 1/n A |
| 1/n A | 1/n A | B |
| B | 1/n A | 1/n A |
| | | |
| 1/n A | B | 1/m A |
| 1/n A | 1/m A | B |
| B | 1/n A | 1/m A |

| (2) Dualities based on complementary components: | | |
|---|---|---|
| A | A' | |
| A | | A' |
| | A | A' |
| A' A | | |
| A' | | A |
| | A' | A |
| | | |
| 1/n A | A' | 1/n A |
| 1/n A | 1/n A | A' |
| A' | 1/n A | 1/n A |
| | | |
| 1/n A | A' | 1/m A |
| 1/n A | 1/m A | A' |
| A' | 1/n A | 1/m A |

| (3) Dualities based on intensity differences of a single component: | | |
|---|---|---|
| n*A | A | |
| n*A | | A |

| Coating and/or Center-fill Composition | Elastomeric Portion Composition | Saccharide Portion Composition |
|---|---|---|
| | n*A | A |
| A | n*A | |
| A | | n*A |
| | A | n*A |
| | | |
| n*A | A | n*A |
| n*A | n*A | A |
| A | n*A | n*A |
| | | |
| n*A | A | m*A |
| n*A | m*A | A |
| A | n*A | m*A |

Table 1, above, provides examples of a variety of different physical combinations of two components used to impart a duality to a confection. In some embodiments, more than one combination might be included.

Dualities based on distinct flavors may include, but are not limited to, the following combinations: a mint flavor and a fruit flavor; a mint flavor and a spicy flavor; a mint flavor and a savory flavor; a mint flavor and an indulgent flavor; a fruit flavor and a spicy flavor; a fruit flavor and a savory flavor; a fruit flavor and an indulgent flavor; a spicy flavor and a savory flavor; a spicy flavor and an indulgent flavor; and a savory flavor and an indulgent flavor.

Some of the duality combinations set forth above include an indulgent flavor. As used herein, "indulgent" refers to a type of flavor associated with a creamy or decadent taste. Sometimes these flavors are referred to as "sweet/brown" in the art. Examples of suitable indulgent flavors include, but are not limited to, maple, cola, chocolate, dulce de leche, raisin, vanilla, caramel, dairy flavors, such as cream, butter, milk and yogurt, butterscotch, peanut butter, fruit cream flavors, such as strawberry cream, and combinations thereof.

In some embodiments, an indulgent flavor is included in a texture modifying agent as discussed below to provide an unctuous mouthfeel along with the indulgent flavor perception. In some embodiments, the indulgent flavor and unctuous mouthfeel provide an eating experience similar to high caloric confections such as chocolate without delivering the calories.

In one embodiment, the duality or modalities can provide a pleasing odor excreted by the consumer's skin after it is consumed, or reduce and/or neutralize a body odor after it is consumed.

In some embodiments, at least two flavors that are complementary may be employed. In some embodiments, the complementary flavors may be the same type of flavor, e.g., two different mint flavors. In some other embodiments, a first flavor, e.g., a fruit flavor, may be provided, and the second flavor may be complementary by enhancing the first flavor, e.g., a fruit potentiator. More specifically, dualities based on complementary flavors may include, but are not limited to, the following combinations: a mint flavor and a mint potentiator; a fruit flavor and a fruit potentiator; a spicy flavor and a spice potentiator; a savory flavor and a savory potentiator; a mint flavor and a different mint flavor; a fruit flavor and a different fruit flavor; a spicy flavor and a different spicy flavor; a savory flavor and a different savory flavor; and an indulgent flavor and a different indulgent flavor.

In some embodiments, the duality may be based on at least two portions of a flavor that differ in intensity. For example, any of the following types of flavors may be used in at least two portions, each of which contains a different amount or intensity of the flavor: mint flavor; fruit flavor; spicy flavor; savory flavor; and indulgent flavor. For example, one of the portions of the confectionery composition may include a first amount or intensity of a flavor and a separate portion may include a second amount or intensity of the same flavor. The second amount or intensity may be greater than the first amount or intensity of the flavor, thereby creating an intensity differential in the flavor impact. It further may be desirable, in some embodiments, to include a third portion of the same flavor in the remaining portion of the confection, which is different in amount or intensity than the first and/or second portion.

In some embodiments, the amount of flavor used to create a desired intensity is determined by the portion to which the flavor is added. For example, the amount of flavor added to the saccharide portion to create a desired intensity can be lower than the amount of flavor added to the elastomeric portion to create the same intensity. Therefore, in some embodiments, a desired confectionery composition flavor intensity can be created using an amount of flavor lower than would be needed to create the same flavor intensity in a confectionery composition without the saccharide portion.

A variety of exemplary flavors, such as mint, fruit, spicy, savory and indulgent flavors are provided in Table 2 herein. Specific flavors may be selected from Table 2 and combined in various manners as described herein.

Further, in some embodiments, at least one of the flavors may have a modified release profile. As described in more detail below, components may be at least partially encapsulated to provide a modified release profile. Suitable encapsulating materials and methods of encapsulation are provided in more detail below in the section entitled "Additional Components." One or all of the flavors used in the confectionery composition may be at least partially encapsulated. Further, in some embodiments, at least one of the flavors may include a mixture of the flavor in its encapsulated and unencapsulated (sometimes referred to as "free") forms. Encapsulated and unencapsulated forms of a flavor may be included in any of the portions of the confectionery composition in the same or different amounts.

Some embodiments described herein extend to methods of preparing multi-modality confectionery products, which include at least one flavor duality. In particular, a confectionery composition including any of the flavor dualities described above may first be provided. The confectionery composition may include a saccharide portion, an elastomeric portion and optionally a third portion, which may be a coating or shell or a center-fill. One of the confectionery composition portions may include at least one first flavor and at least a second portion of the confectionery composition portions may include at least one second flavor. The second flavor may be distinct from, complementary to or different in intensity from the first flavor. Individual confectionery composition pieces then may be formed from the confectionery composition.

Some confectionery compositions may include a duality based on sensations, such as coolness, warmth and tingling sensations. Such sensations may be provided by sensates, such as cooling agents, warming agents and tingling agents, respectively. In some embodiments, one of the portions of the confectionery composition may include a first sensate and at least a second of the portions may include at least a second sensate. The second sensate may be distinct from, complementary to or different in intensity from the first sensate.

A variety of sensates may be used in any of these or other combinations to impart different dualities. More specifically, in some embodiments, at least two sensates that are distinct may be employed. Dualities based on distinct sensates may include, but are not limited to, the following combinations: a cooling agent and a warming agent; a cooling agent and a tingling agent; and a warming agent and a tingling agent.

In some embodiments, at least two sensates that are complementary may be employed. In particular, the complementary sensates may be the same type of sensate, such as, two different cooling agents, two different warming agents or two different tingling agents.

In some embodiments, the duality may be based on at least two portions of a sensate that differ in intensity. Any of the following types of sensates may be used in at least two portions, each of which contains a different amount or delivers a different intensity of the sensate: cooling agents, warming agents or tingling agents. For example, one of the portions of the confectionery composition may include a first amount or intensity of a sensate and a separate portion may include a second amount or intensity of the same sensate. The second amount or intensity may be greater than the first amount or intensity of the sensate, thereby creating an intensity differential in the sensation. It further may be desirable, in some embodiments, to include a third portion or intensity of the same sensate in the remaining portion of the confectionery composition, which is different in amount or intensity than the first and/or second portion or intensity of the sensate.

As with the flavor ingredients described above, the amounts of sensates added to the various portions of a confectionery composition can depend on the composition of that portion and how the sensate interacts with that portion. For example, in some embodiments, sensates with an affinity for the polymers in elastomeric are used in lower amounts to deliver a desired sensation intensity when they are included in portions such as the saccharide, coating, or center-fill portions than when those sensates with an affinity for elastomeric materials are included in the elastomeric portion. Therefore, in some embodiments, the overall level of sensates needed to deliver a desired sensation can be manipulated and lowered by including the sensate in one portion versus another.

A variety of exemplary sensates, such as cooling, warming and tingling agents are provided in Table 2 herein. Specific sensates may be selected from Table 2 and combined in various manners as described herein.

Further, in some embodiments, at least one of the sensates may have a modified release profile. As described in more detail below, components may be at least partially encapsulated to provide a modified release profile. Suitable encapsulating materials and methods of encapsulation are provided in more detail below in the section entitled "Additional Components." One or all of the sensates used in the confectionery composition may be at least partially encapsulated. Further, in some embodiments, at least one of the sensates may include a mixture of the sensate in its encapsulated and unencapsulated (sometimes referred to as "free") forms. Encapsulated and unencapsulated forms of a sensate may be included in any of the portions of the confectionery compositions in the same or different amounts.

Some embodiments described herein extend to methods of preparing multi-modality confectionery products, which include at least one sensation duality. In particular, a confectionery composition including any of the sensation dualities described above may first be provided. One of the confectionery composition portions may include at least one first sensate and at least a second of the confectionery composition portions may include at least one second sensate. The second sensate may be distinct from, complementary to or different in intensity from the first sensate. Individual confectionery composition pieces then may be formed from the confectionery composition. Methods of forming individual confectionery pieces from confectionery compositions are described in more detail below in the section entitled "Processing."

In some embodiments, methods of imparting a dual sensation perception are provided. In accordance therewith, a confectionery product prepared as described above may be provided. The confectionery product may include a saccharide portion, an elastomeric portion, and optionally a third portion, which may be a coating or center-fill. One of the confectionery composition portions may include at least one first sensate and at least a second of the confectionery composition portions may include at least one second sensate. The second sensate may be distinct from, complementary to or different in intensity from the first sensate. The confectionery product may be applied into the oral cavity of an individual. As the individual chews the product and saliva mixes therewith, the at least one first sensate and the at least one second sensate may be released from the confection. The individual may experience a dual sensation perception as the first and second sensates are released and combine in the oral cavity.

Some confectionery compositions may include a duality based on tastes, such as, bitter, salty, sweet, sour, uma.

In one embodiment, the duality or modalities can provide a pleasing odor excreted by the consumer's skin after it is consumed. As with the other sensory and/or functional ingredients described herein such odoriferous components may be combined in a single component of the confectionery or in different portions thereof. In certain embodiments, the selection of odoriferous components to include is not based on their initial odor but on its odor after being excreted by the body and, in particular, its action with other components in the confectionery. In one embodiment, the odoriferous components can further act with one or more flavorants and/or sweeteners to provide the consumer with a unique taste sensation based on the interaction of a person's senses of smell and taste. Non-limiting examples of such odiferous ingredients include geraniol, linalool, nerol, nerolidal, citronellol, alliaceous compounds, heliotropine, methyl cyclopentelone, ethyl vanillin, maltol, ethyl maltol, furaneol, rose type compounds such as phenethanol, phenylacetic acid, nerol, linalyl esters, jasmine, sandlewood, patchouli, and cedarwood.

In certain embodiments, the odiferous ingredients can be selected and/or their release controlled using the delivery systems described herein or other delayed and/or long release technologies known in the art. By controlling the release of the odoriferous ingredient, one can influence the timing before the smell is noticed on the skin, the strength of the smell, the length at which the smell lasts after the composition is injested as well as the onset of excretion.

A variety of tastants may be used in any of these or other combinations to impart different dualities. More specifically, in some embodiments, at least two tastants that are distinct may be employed. Dualities based on distinct tastes may include, but are not limited to, the following combinations: a sweet tastant and a sour tastant; a sweet tastant and a salty tastant; a sweet tastant and a bitter tastant; a sweet tastant and an astringent tastant; a sweet tastant and an umami tastant; a sweet tastant and a kokumi tastant; a sour tastant and a salty tastant; a sour tastant and a bitter tastant; a sour tastant and an astringent tastant; a sour tastant and an umami tastant; a sour tastant and a kokumi tastant; a salty tastant and a bitter tastant; a salty tastant and an astringent tastant; a salty tastant and an umami tastant; a salty tastant and a kokumi tastant; a bitter tastant and an astringent tastant; a bitter tastant and an umami tastant; and a bitter tastant and a kokumi tastant.

In some embodiments, at least two tastants that are complementary may be employed. In particular, the complementary tastants may be the same type of tastant, such as, two different bitter agents; two different sour agents, two different sweeteners; two different salts; two different umami agents; or two different kokumi agents.

In some embodiments, the duality may be based on at least two portions of a tastant that differ in intensity. Any of the following types of tastants may be used in at least two portions, each of which contains a different amount or provides a different intensity of the tastant: bitter agents; two different sour agents, two different sweeteners; two different salts; two different umami agents; or two different kokumi agents. For example, one of the portions of the confectionery composition may include a first amount of a tastant and a separate portion may include a second amount of the same tastant. The second amount may be greater than the first amount of the tastant, thereby creating an intensity differential in the taste. Alternatively, the tastant may provide a greater intensity at a lower amount due to the tastant's interaction with the portion. It further may be desirable, in some embodiments, to include a third portion of the same tastant in the remaining portion of the chewing confectionery, which is different in amount or intensity than the first and/or second portion of the tastant.

Some of the duality combinations set forth above include an umami tastant. "Umami" refers to a taste that is savory, or the taste of glutamate.

Some of the duality combinations set forth above include a kokumi tastant. "Kokumi" refers to materials that impart "mouthfulness" and "good body," as disclosed in U.S. Patent No. 5,679,397 to Kuroda et al.

A variety of exemplary tastants, such as bitter, salty, sweet, sour, umami and kokumi tastants are provided in Table 2 herein. Specific tastants may be selected from Table 2 and combined in various manners as described herein.

Further, in some embodiments, at least one of the tastants may have a modified release profile.

Some confectionery compositions may include a duality based on functionalities. Functionalities include, for example, teeth whitening and breath freshening, among others, and may be provided by various functional agents. In some embodiments, one of the portions of the confectionery composition may include a first functional agent and at least a second of the portions may include at least a second functional agent. The second functional agent may be distinct from, complementary to or different in intensity from the first functional agent. For example, the saccharide portion may include the first functional agent and the elastomeric portion may include the second functional agent.

In some embodiments, the saccharide portion may include the first functional agent, the elastomeric portion the second functional agent and the coating or center-fill may include a third functional agent. The coating or center-fill functional agent, in some embodiments, may be the same as the elastomeric portion functional agent. In such embodiments, the saccharide portion functional agent may be distinct from, complementary to or different in intensity from both the coating or center-fill and elastomeric portion functional agents. In other embodiments, the coating or center-fill functional agent may be complementary to the elastomeric portion functional agent, but distinct from the saccharide portion functional agent. For example, the elastomeric portion and coating or center-fill functional agents may be two different anti-plaque agents, such as, chlorhexidine and triclosan. The saccharide portion functional agent may be distinct from the anti-plaque agents, such as, for example, a remineralization agent. Alternatively, the coating or center-fill functional agent may be the same as the saccharide portion functional agent. In such embodiments, the elastomeric portion functional agent may be distinct from, complementary to or different in intensity from both the coating or center-fill and saccharide portion functional agents. In other embodiments, the coating or center-fill functional agent may be complementary to the saccharide portion functional agent, but distinct from the elastomeric portion functional agent.

A variety of functional agents may be used in any of these or other combinations to impart different dualities. More specifically, in some embodiments, at least two functional agents that are distinct may be employed. Dualities based on distinct functional agents may include, but are not limited to, the following combinations: a vitamin and a mineral; a breath freshening agent and a tooth whitening agent; a breath freshening agent and a remineralization agent; a breath freshening agent and an antimicrobial agent; a tooth whitening agent and a stain prevention agent; a remineralization agent and a demineralization agent; an appetite suppressant and a stress relieving agent; an energy boosting agent and a stress relieving agent; and a concentration enhancing agent and a focus enhancing agent.

In some embodiments, at least two functional agents that are complementary may be employed. In particular, the complementary functional agents may be the same type of functional agent, such as, two different surfactants, two different breath freshening agents, two different anti-microbial agents, two different antibacterial agents, two different anti-calculus agents, two different anti-plaque agents, two different fluoride compounds, two different quaternary ammonium compounds, two different remineralization agents, two different demineralization agents, two different pharmaceutical actives, two different micronutrients, two different throat care actives, two different tooth whitening agents, two different stain removing agents, two different energy boosting agents, two different concentration boosting agents, two different focus enhancing agents and two different appetite suppressants.

In some embodiments, the duality may be based on at least two portions of a functional agent that differ in intensity. Any of the types of functional agents set forth above in the description of complementary functional agents may be used in at least two portions, each of which contains a different amount of the functional agent. For example, one of the portions of the confectionery composition may include a first amount of a functional agent and a separate portion may include a second amount of the same functional agent. The second amount may be greater than the first amount of the functional agent, thereby creating an intensity differential in the functionality. Additionally, the difference in intensity may arise from the composition of the portion and interaction between the portion and the functional agent. Therefore, in some embodiments, lower amounts can provide higher intensities of functional agents when they are more completely released from a given portion. It further may be desirable, in some embodiments, to include a third portion of the same functional agent in the remaining portion of the confectionery composition, which is different in amount or intensity than the first and/or second portion of the functional agent.

A variety of exemplary functional agents are provided in Table 2 herein. Specific functional agents may be selected from Table 2 and combined in various manners as described herein.

Further, in some embodiments, at least one of the functional agents may have a modified release profile. As described in more detail below, components may be at least partially encapsulated to provide a modified release profile. Suitable encapsulating materials and methods of encapsulation are provided in more detail below in the section entitled "Additional Components." One or all of the functional agents used in the confectionery compositions may be at least partially encapsulated. Further, in some embodiments, at least one of the functional agents may include a mixture of the functional agent in its encapsulated and unencapsulated (sometimes referred to as "free") forms. Encapsulated and unencapsulated forms of a functional agent may be included in any of the portions of the confectionery composition in the same or different amounts or intensities.

As mentioned above, specific flavors, sensates, tastants and functional agents may be selected from the exemplary listing of multi-modality components provided in Table 2 below and combined to create any of the different dualities described above. Suitable amounts for a multi-modality component when it is selected for use in any of the three portions are set forth in Table 2. Table 2 also provides a listing of basic components that can be included in three portions of a confectionery composition. Suitable amounts for the basic components also are set forth in Table 2. The amounts provided for the basic and multi-modality components are based on the specified portion in which the component is contained.

Further, the amounts provided for the multi-modality components in Table 2 generally apply to a component as it may be added to the specified portion of the confectionery composition in a free form, i.e., unencapsulated. In some embodiments, where the selected multi-modality component is provided in an encapsulated form, an amount greater than those amounts as set forth in Table 2 may be used due to the modified release profile of the component. Also, because a multi-modality component is selected in a specific embodiment to create a specific duality, the amounts provided in Table 2 represent amounts used only when the component is selected for inclusion in the composition. In other words, the lower limit of 0% is not included even though the multi-modality component may not be present.

Any of the multi-modality components listed in Table 2, below, which are selected to create a specific duality or multi-modality in a confectionery composition may be added to any portion of the confectionery composition in their encapsulated and/or unencapsulated forms.

In some embodiments, the two or more ingredients are kept separate in the confectionery and are not mixed until consumption (e.g., an acid and a base).

As described above, Table 2 provides a list of multi-modality components that optionally may be present in one or more portions of the confectionery product. Suitable amounts that may be present in the coating or center-fill, saccharide portion or elastomeric portion are provided in the table. The amounts in Table 2 are provided as ppm or weight % in a portion of the confectionery product. Table 2 is only representative and is not to be construed to limit the ingredients that can be included in the confectionery composition portions in any way.

**TABLE 2**

| Components | Coating and/or center fill | Saccharide Portion | Elastomeric Portion |
|---|---|---|---|
| Basic Components | | | |
| Sugar | 0-100% | 0-95% | 20-80% |
| Polyol | 0-100% | 0-95% | 20-80% |
| Glycerin | 0-90% | 1-70% | 0-7% |
| Natural or synthetic confectionery | | 0-1% | |
| Elastomer | | | 10-70% |
| Bulking agent/Filler | 0-20% | 0-12% | 0-30% |
| Plasticizer/Softening agent | | | 0-10% |
| Mineral adjuvants | 0-20% | 0-20% | 0-12% |
| Wax | | | 0-3.0% |
| Emulsifier/Thickener | 0-3% | 0-5% | 0-1% |
| Texture Modifying Component | 0-10% | 2-25% | 0-30% |
| | | | |
| Multi-Modality Components | | | |

| I. Sensates | | | |
|---|---|---|---|
| A. Cooling agents | | | |
| Menthol | 10-500 ppm | 10-500 ppm | 500 - 20,000 ppm |
| Xylitol | 5-100% | 5-95% | 5-80% |

| Components | Coating and/or center fill | Saccharide Portion | Elastomeric Portion |
|---|---|---|---|
| Erythritol | 5-100% | 5-95% | 5-80% |
| Menthane | 10-500 ppm | 10-500 ppm | 500 - 20,000 ppm |
| Menthone | 10-500 ppm | 10-500 ppm | 500 - 20,000 ppm |
| Menthyl acetate | 10-500 ppm | 10-500 ppm | 500 - 20,000 ppm |
| Menthyl salicylate | 10-500 ppm | 10-500 ppm | 500 - 20,000 ppm |
| WS-23 | 10-500 ppm | 10-500 ppm | 500 - 20,000 ppm |
| WS-3 | 10-500 ppm | 10-500 ppm | 500 - 20,000 ppm |
| Menthyl succinate (and its alkaline earth metal salts) | 10-500 ppm | 10-500 ppm | 500 - 20,000 ppm |
| 3,1-menthoxypropane 1,2-diol | 10-500 ppm | 10-500 ppm | 500 - 20,000 ppm |
| Glutarate esters | 10-500 ppm | 10-500 ppm | 500 - 20,000 ppm |
| dextrose | 10-500 ppm | 10-500 ppm | 500 - 20,000 ppm |
| sorbitol | 10-500 ppm | 10-500 ppm | 500 - 20,000 ppm |
| ketals | 10-500 ppm | 10-500 ppm | 500 - 20,000 ppm |
| menthone ketals | 10-500 ppm | 10-500 ppm | 500 - 20,000 ppm |
| menthone glycerol ketals | 10-500 ppm | 10-500 ppm | 500 - 20,000 ppm |
| substituted p-menthanes | 10-500 ppm | 10-500 ppm | 500 - 20,000 ppm |
| acyclic carboxamides | 10-500 ppm | 10-500 ppm | 500 - 20,000 ppm |
| mono menthyl glutarate | 10-500 ppm | 10-500 ppm | 500 - 20,000 ppm |
| substituted cyclohexanamides | 10-500 ppm | 10-500 ppm | 500 - 20,000 ppm |
| substituted cyclohexane carboxamides | 10-500 ppm | 10-500 ppm | 500 - 20,000 ppm |
| substituted ureas and sulfonamides | 10-500 ppm | 10-500 ppm | 500 - 20,000 ppm |
| substituted menthanols | 10-500 ppm | 10-500 ppm | 500 - 20,000 ppm |
| hydroxymethyl | 10-500 ppm | 10-500 ppm | 500 - 20,000 ppm |
| hydroxymethyl derivatives of p-menthane | 10-500 ppm | 10-500 ppm | 500 - 20,000 ppm |
| 2-mercapto-cyclo-decanone | 10-500 ppm | 10-500 ppm | 500 - 20,000 ppm |
| hydroxycarboxylic acids with 2-6 carbon atoms | 10-500 ppm | 10-500 ppm | 500 - 20,000 ppm |
| cyclohexanamides | 10-500 ppm | 10-500 ppm | 500 - 20,000 ppm |
| 1-isopulegol | 10-500 ppm | 10-500 ppm | 500 - 20,000 ppm |
| 3-(1-menthoxy)-2-methylpropane-1,2-diol | 10-500 ppm | 10-500 ppm | 500 - 20,000 ppm |
| p-menthane-2,3-diol | 10-500 ppm | 10-500 ppm | 500 - 20,000 ppm |
| p-menthane-3,8-diol | 10-500 ppm | 10-500 ppm | 500 - 20,000 ppm |
| 6-isopropyl-9-methyl-1,4-dioxaspiro[4,5]decane-2-methanol | 10-500 ppm | 10-500 ppm | 500 - 20,000 ppm |
| trimethylcyclohexanol | 10-500 ppm | 10-500 ppm | 500 - 20,000 ppm |
| N-ethyl-2-isopropyl-5-methylcyclohexanecarboxamide | 10-500 ppm | 10-500 ppm | 500 - 20,000 ppm |
| Japanese mint oil | 10-500 ppm | 10-500 ppm | 500 - 20,000 ppm |
| Peppermint oil | 10-500 ppm | 10-500 ppm | 500 - 20,000 ppm |
| 3-(1-menthoxy)ethan-1-ol | 10-500 ppm | 10-500 ppm | 500 - 20,000 ppm |
| 3-(1-menthoxy)propan-1-ol | 10-500 ppm | 10-500 ppm | 500 - 20,000 ppm |
| 3-(1-menthoxy)butan-1-ol | 10-500 ppm | 10-500 ppm | 500 - 20,000 ppm |
| 1-menthylacetic acid N-ethylamide | 10-500 ppm | 10-500 ppm | 500 - 20,000 ppm |
| 1-menthyl-4-hydroxypentanoate | 10-500 ppm | 10-500 ppm | 500 - 20,000 ppm |
| 1-menthyl-3-hydroxybutyrate | 10-500 ppm | 10-500 ppm | 500 - 20,000 ppm |
| N,2,3-trimethyl-2-(1-methylethyl)-butanamide | 10-500 ppm | 10-500 ppm | 500 - 20,000 ppm |
| n-ethyl-t-2-c-6 nonadienamide | 10-500 ppm | 10-500 ppm | 500 - 20,000 ppm |
| N,N-dimethyl menthyl succinamide | 10-500 ppm | 10-500 ppm | 500 - 20,000 ppm |
| substituted p-menthane-carboxamides | 10-500 ppm | 10-500 ppm | 500 - 20,000 ppm |
| 2-isopropanyl-5-methylcyclohexanol | 10-500 ppm | 10-500 ppm | 500 - 20,000 ppm |
| menthyl lactate | 10-500 ppm | 10-500 ppm | 500 - 20,000 ppm |
| WS-30 | 10-500 ppm | 10-500 ppm | 500 - 20,000 ppm |
| WS-14 | 10-500 ppm | 10-500 ppm | 500 - 20,000 ppm |
| Eucalyptus extract | 10-500 ppm | 10-500 ppm | 500 - 20,000 ppm |
| Menthol PG carbonate | 10-500 ppm | 10-500 ppm | 500 - 20,000 ppm |
| Menthol EG carbonate | 10-500 ppm | 10-500 ppm | 500 - 20,000 ppm |
| Menthol glyceryl ether | 10-500 ppm | 10-500 ppm | 500 - 20,000 ppm |
| N-tertbutyl-p-menthane-3-carboxamide | 10-500 ppm | 10-500 ppm | 500 - 20,000 ppm |
| P-menthane-3-carboxylic acid glycerol ester | 10-500 ppm | 10-500 ppm | 500 - 20,000 ppm |
| Methyl-2-isopryl-bicyclo (2.2.1) | 10-500 ppm | 10-500 ppm | 500 - 20,000 ppm |
| Heptane-2-carboxamide | 10-500 ppm | 10-500 ppm | 500 - 20,000 ppm |
| Menthol methyl ether | 10-500 ppm | 10-500 ppm | 500 - 20,000 ppm |
| Methyl glutarate | 10-500 ppm | 10-500 ppm | 500 - 20,000 ppm |
| menthyl pyrrolidone carboxylate | 10-500 ppm | 10-500 ppm | 500 - 20,000 ppm |
| WS-5 | 10-500 ppm | 10-500 ppm | 500 - 20,000 ppm |
| WS-15 | 10-500 ppm | 10-500 ppm | 500 - 20,000 ppm |
| | | | |

| B. Warming agents | | | |
|---|---|---|---|
| vanillyl alcohol n-butylether | 1-1000 ppm | 1-1500 ppm | 10-8000 ppm |
| vanillyl alcohol n-propylether | 1-1000 ppm | 1-1500 ppm | 10-8000 ppm |
| vanillyl alcohol isopropylether | 1-1000 ppm | 1-1500 ppm | 10-8000 ppm |
| vanillyl alcohol isobutylether | 1-1000 ppm | 1-1500 ppm | 10-8000 ppm |
| vanillyl alcohol n-aminoether | 1-1000 ppm | 1-1500 ppm | 10-8000 ppm |
| vanillyl alcohol isoamylether | 1-1000 ppm | 1-1500 ppm | 10-8000 ppm |
| vanillyl alcohol n-hexylether | 1-1000 ppm | 1-1500 ppm | 10-8000 ppm |
| vanillyl alcohol methylether | 1-1000 ppm | 1-1500 ppm | 10-8000 ppm |
| vanillyl alcohol ethylether | 1-1000 ppm | 1-1500 ppm | 10-8000 ppm |
| gingerol | 1-1000 ppm | 1-1500 ppm | 10-8000 ppm |
| shogaol | 1-1000 ppm | 1-1500 ppm | 10-8000 ppm |

| Components | Coating and/or center fill | Saccharide Portion | Elastomeric Portion |
|---|---|---|---|
| paradol | 1-1000 ppm | 1-1500 ppm | 10-8000 ppm |
| zingerone | 1-1000 ppm | 1-1500 ppm | 10-8000 ppm |
| capsaicin | 1-1000 ppm | 1-1500 ppm | 10-8000 ppm |
| dihydrocapsaicin | 1-1000 ppm | 1-1500 ppm | 10-8000 ppm |
| nordihydrocapsaicin | 1-1000 ppm | 1-1500 ppm | 10-8000 ppm |
| homocapsaicin | 1-1000 ppm | 1-1500 ppm | 10-8000 ppm |
| homodihydrocapsaicin | 1-1000 ppm | 1-1500 ppm | 10-8000 ppm |
| ethanol | 1-1000 ppm | 1-1500 ppm | 10-8000 ppm |
| isopropyl alcohol | 1-1000 ppm | 1-1500 ppm | 10-8000 ppm |
| iso-amylalcohol | 1-1000 ppm | 1-1500 ppm | 10-8000 ppm |
| benzyl alcohol | 1-1000 ppm | 1-1500 ppm | 10-8000 ppm |
| glycerine | 1-1000 ppm | 1-1500 ppm | 10-8000 ppm |
| chloroform | 1-1000 ppm | 1-1500 ppm | 10-8000 ppm |
| eugenol | 1-1000 ppm | 1-1500 ppm | 10-8000 ppm |
| cinnamon oil | 1-1000 ppm | 1-1500 ppm | 10-8000 ppm |
| cinnamic aldehyde | 1-1000 ppm | 1-1500 ppm | 10-8000 ppm |
| | | | |

| C. Tingling agents | | | |
|---|---|---|---|
| Jambu Oleoresin or para cress | 5-500 ppm | 5-500 ppm | 50-5000 ppm |
| Japanese pepper extract | 5-500 ppm | 5-500 ppm | 50-5000 ppm |
| black pepper extract | 5-500 ppm | 5-500 ppm | 50-5000 ppm |
| Echinacea extract | 5-500 ppm | 5-500 ppm | 50-5000 ppm |
| Northern Prickly Ash extract | 5-500 ppm | 5-500 ppm | 50-5000 ppm |
| red pepper oleoresin | 5-500 ppm | 5-500 ppm | 50-5000 ppm |
| effervescing agents | 5-500 ppm | 5-500 ppm | 50-5000 ppm |
| Spilanthol | 5-500 ppm | 5-500 ppm | 50-5000 ppm |
| Sanshool | 5-500 ppm | 5-500 ppm | 50-5000 ppm |
| | | | |

| II. Flavors | | | |
|---|---|---|---|
| spearmint oil | 0.01 - 10.0% | 0.01 - 10.0 % | 0.5 - 30.0 % |
| cinnamon oil | 0.01 - 10.0% | 0.01 - 10.0 % | 0.5 - 30.0 % |
| oil of wintergreen | 0.01 - 10.0% | 0.01 - 10.0 % | 0.5 - 30.0 % |
| peppermint oil | 0.01 - 10.0% | 0.01 - 10.0 % | 0.5 - 30.0 % |
| clove oil | 0.01 - 10.0% | 0.01 - 10.0 % | 0.5 - 30.0 % |
| bay oil | 0.01 - 10.0% | 0.01 - 10.0 % | 0.5 - 30.0 % |
| anise oil | 0.01 - 10.0% | 0.01 - 10.0 % | 0.5 - 30.0 % |
| eucalyptus oil | 0.01 - 10.0% | 0.01 - 10.0 % | 0.5 - 30.0 % |
| thyme oil | 0.01 - 10.0% | 0.01 - 10.0 % | 0.5 - 30.0 % |
| cedar leaf oil | 0.01 - 10.0% | 0.01 - 10.0 % | 0.5 - 30.0 % |
| oil of nutmeg | 0.01 - 10.0% | 0.01 - 10.0 % | 0.5 - 30.0 % |
| allspice | 0.01 - 10.0% | 0.01 - 10.0 % | 0.5 - 30.0 % |
| oil of sage | 0.01 - 10.0% | 0.01 - 10.0 % | 0.5 - 30.0 % |
| mace | 0.01 - 10.0% | 0.01 - 10.0 % | 0.5 - 30.0 % |
| oil of bitter almonds | 0.01 - 10.0% | 0.01 - 10.0 % | 0.5 - 30.0 % |
| cassia oil | 0.01 - 10.0% | 0.01 - 10.0 % | 0.5 - 30.0 % |
| vanilla | 0.01 - 10.0% | 0.01 - 10.0 % | 0.5 - 30.0 % |

| Components | Coating and/or center fill | Saccharide Portion | Elastomeric Portion |
|---|---|---|---|
| lemon | 0.01 - 10.0% | 0.01 - 10.0 % | 0.5 - 30.0 % |
| range | 0.01 - 10.0% | 0.01 - 10.0 % | 0.5 - 30.0 % |
| lime | 0.01 - 10.0% | 0.01 - 10.0 % | 0.5 - 30.0 % |
| grapefruit | 0.01 - 10.0% | 0.01 - 10.0 % | 0.5 - 30.0 % |
| apple | 0.01 - 10.0% | 0.01 - 10.0 % | 0.5 - 30.0 % |
| pear | 0.01 - 10.0% | 0.01 - 10.0 % | 0.5 - 30.0 % |
| peach | 0.01 - 10.0% | 0.01 - 10.0 % | 0.5 - 30.0 % |
| grape | 0.01 - 10.0% | 0.01 - 10.0 % | 0.5 - 30.0 % |
| strawberry | 0.01 - 10.0% | 0.01 - 10.0 % | 0.5 - 30.0 % |
| raspberry | 0.01 - 10.0% | 0.01 - 10.0 % | 0.5 - 30.0 % |
| cherry | 0.01 - 10.0% | 0.01 - 10.0 % | 0.5 - 30.0 % |
| plum | 0.01 - 10.0% | 0.01 - 10.0 % | 0.5 - 30.0 % |
| pineapple | 0.01 - 10.0% | 0.01 - 10.0 % | 0.5 - 30.0 % |
| apricot | 0.01 - 10.0% | 0.01 - 10.0 % | 0.5 - 30.0 % |
| watermelon | 0.01 - 10.0% | 0.01 - 10.0 % | 0.5 - 30.0 % |
| chocolate | 0.01 - 10.0% | 0.01 - 10.0 % | 0.5 - 30.0 % |
| cola | 0.01 - 10.0% | 0.01 - 10.0 % | 0.5 - 30.0 % |
| maple | 0.01 - 10.0% | 0.01 - 10.0 % | 0.5 - 30.0 % |
| dulce de leche | 0.01 - 10.0% | 0.01 - 10.0 % | 0.5 - 30.0 % |
| raisin | 0.01 - 10.0% | 0.01 - 10.0 % | 0.5 - 30.0 % |
| caramel | 0.01 - 10.0% | 0.01 - 10.0 % | 0.5 - 30.0 % |
| cinnamyl acetate | 0.01 - 10.0% | 0.01 - 10.0 % | 0.5 - 30.0 % |
| cinnamaldehyde | 0.01 - 10.0% | 0.01 - 10.0 % | 0.5 - 30.0 % |
| citral diethylacetal | 0.01 - 10.0% | 0.01 - 10.0 % | 0.5 - 30.0 % |
| dihydrocarvyl acetate | 0.01 - 10.0% | 0.01 - 10.0 % | 0.5 - 30.0 % |
| eugenyl formate | 0.01 - 10.0% | 0.01 - 10.0 % | 0.5 - 30.0 % |
| p-methylamisol | 0.01 - 10.0% | 0.01 - 10.0 % | 0.5 - 30.0 % |
| acetaldehyde | 0.01 - 10.0% | 0.01 - 10.0 % | 0.5 - 30.0 % |
| benzaldehyde | 0.01 - 10.0% | 0.01 - 10.0 % | 0.5 - 30.0 % |
| anisic aldehyde | 0.01 - 10.0% | 0.01 - 10.0 % | 0.5 - 30.0 % |
| cinnamic aldehyde | 0.01 - 10.0% | 0.01 - 10.0 % | 0.5 - 30.0 % |
| citral | 0.01 - 10.0% | 0.01 - 10.0 % | 0.5 - 30.0 % |
| neral | 0.01 - 10.0% | 0.01 - 10.0 % | 0.5 - 30.0 % |
| decanal | 0.01 - 10.0% | 0.01 - 10.0 % | 0.5 - 30.0 % |
| ethyl vanillin | 0.01 - 10.0% | 0.01 - 10.0 % | 0.5 - 30.0 % |
| heliotrope | 0.01 - 10.0% | 0.01 - 10.0 % | 0.5 - 30.0 % |
| vanillin | 0.01 - 10.0% | 0.01 - 10.0 % | 0.5 - 30.0 % |
| alpha-amyl cinnamaldehyde | 0.01 - 10.0% | 0.01 - 10.0 % | 0.5 - 30.0 % |
| butyraldehyde | 0.01 - 10.0% | 0.01 - 10.0 % | 0.5 - 30.0 % |
| valeraldehyde | 0.01 - 10.0% | 0.01 - 10.0 % | 0.5 - 30.0 % |
| citronellal | 0.01 - 10.0% | 0.01 - 10.0 % | 0.5 - 30.0 % |
| decanal | 0.01 - 10.0% | 0.01 - 10.0 % | 0.5 - 30.0 % |
| aldehyde C-8 | 0.01 - 10.0% | 0.01 - 10.0 % | 0.5 - 30.0 % |
| aldehyde C-9 | 0.01 - 10.0% | 0.01 - 10.0 % | 0.5 - 30.0 % |
| aldehyde C-12 | 0.01 - 10.0% | 0.01 - 10.0 % | 0.5 - 30.0 % |
| 2-ethyl butyraldehyde | 0.01 - 10.0% | 0.01 - 10.0 % | 0.5 - 30.0 % |
| hexenal | 0.01 - 10.0% | 0.01 - 10.0 % | 0.5 - 30.0 % |
| tolyl aldehyde | 0.01 - 10.0% | 0.01 - 10.0 % | 0.5 - 30.0 % |
| veratraldehyde | 0.01 - 10.0% | 0.01 - 10.0 % | 0.5 - 30.0 % |
| 2,6-dimethyl-5-heptenal | 0.01 - 10.0% | 0.01 - 10.0 % | 0.5 - 30.0 % |
| 2,6-dimethyloctanal | 0.01 - 10.0% | 0.01 - 10.0 % | 0.5 - 30.0 % |
| 2-dodecenal | 0.01 - 10.0% | 0.01 - 10.0 % | 0.5 - 30.0 % |
| strawberry shortcake | 0.01 - 10.0% | 0.01 - 10.0 % | 0.5 - 30.0 % |
| pomegranate | 0.01 - 10.0% | 0.01 - 10.0 % | 0.5 - 30.0 % |
| beef | 0.01 - 10.0% | 0.01 - 10.0 % | 0.5 - 30.0 % |
| chicken | 0.01 - 10.0% | 0.01 - 10.0 % | 0.5 - 30.0 % |
| cheese | 0.01 - 10.0% | 0.01 - 10.0 % | 0.5 - 30.0 % |
| onion | 0.01 - 10.0% | 0.01 - 10.0 % | 0.5 - 30.0 % |
| geraniol | 0.01 - 10.0% | 0.01 - 10.0 % | 0.5 - 30.0 % |
| linalool | 0.01 - 10.0% | 0.01 - 10.0 % | 0.5 - 30.0 % |
| nerol | 0.01 - 10.0% | 0.01 - 10.0 % | 0.5 - 30.0 % |
| nerolidal | 0.01 - 10.0% | 0.01 - 10.0 % | 0.5 - 30.0 % |
| citronellol | 0.01 - 10.0% | 0.01 - 10.0 % | 0.5 - 30.0 % |
| heliotropine | 0.01 - 10.0% | 0.01 - 10.0 % | 0.5 - 30.0 % |
| methyl cyclopentelone | 0.01 - 10.0% | 0.01 - 10.0 % | 0.5 - 30.0 % |
| ethyl maltol | 0.01 - 10.0% | 0.01 - 10.0 % | 0.5 - 30.0 % |
| furaneol | 0.01 - 10.0% | 0.01 - 10.0 % | 0.5 - 30.0 % |
| alliaceous compounds | 0.01 - 10.0% | 0.01 - 10.0 % | 0.5 - 30.0 % |
| rose type compounds such as phenethanol, phenylacetic acid, nerol, linalyl esters | 0.01 - 10.0% | 0.01 - 10.0 % | 0.5 - 30.0 % |
| jasmine | 0.01 - 10.0% | 0.01 - 10.0 % | 0.5 - 30.0 % |
| sandlewood | 0.01 - 10.0% | 0.01 - 10.0 % | 0.5 - 30.0 % |
| patchouli | 0.01 - 10.0% | 0.01 - 10.0 % | 0.5 - 30.0 % |
| cedarwood | 0.01 - 10.0% | 0.01 - 10.0 % | 0.5 - 30.0 % |
| | | | |

| III. Tastes | | | |
|---|---|---|---|
| A. Sweeteners | | | |
| sucrose | 5-100% | 5-100% | 5-80% |
| dextrose | 5-100% | 5-100% | 5-80% |
| maltose | 5-100% | 5-100% | 5-80% |
| dextrin | 5-100% | 5-100% | 5-80% |
| xylose | 5-100% | 5-100% | 5-80% |
| ribose | 5-100% | 5-100% | 5-80% |
| glucose | 5-100% | 5-100% | 5-80% |
| mannose | 5-100% | 5-100% | 5-80% |
| galactose | 5-100% | 5-100% | 5-80% |
| fructose | 5-100% | 5-100% | 5-80% |
| invert sugar | 5-100% | 5-100% | 5-80% |
| fructo oligo saccharide syrups | 5-100% | 5-100% | 5-80% |
| partially hydrolyzed starch | 5-100% | 5-100% | 5-80% |
| corn syrup solids | 5-100% | 5-100% | 5-80% |

| Components | Coating and/or center fill | Saccharide Portion | Elastomeric Portion |
|---|---|---|---|
| sorbitol | 5-100% | 5-100% | 5-80% |
| xylitol | 5-100% | 5-100% | 5-80% |
| mannitol | 5-100% | 5-100% | 5-80% |
| galactitol | 5-100% | 5-100% | 5-80% |
| maltitol | 5-100% | 5-100% | 5-80% |
| Isomalt | 5-100% | 5-100% | 5-80% |
| lactitol | 5-100% | 5-100% | 5-80% |
| erythritol | 5-100% | 5-100% | 5-80% |
| hydrogenated starch hydrolysate | 5-100% | 5-100% | 5-80% |
| stevia | 10 - 20,000 ppm | 10 - 20,000 ppm | 10 - 20,000 ppm |
| dihydrochalcones | 10 - 20,000 ppm | 10 - 20,000 ppm | 10 - 20,000 ppm |
| monellin | 10 - 20,000 ppm | 10 - 20,000 ppm | 10 - 20,000 ppm |
| steviosides | 10 - 20,000 ppm | 10 - 20,000 ppm | 10 - 20,000 ppm |
| glycyrrhizin | 10 - 20,000 ppm | 10 - 20,000 ppm | 10 - 20,000 ppm |
| dihydroflavenol | 10 - 20,000 ppm | 10 - 20,000 ppm | 10 - 20,000 ppm |
| L-aminodicarboxylic acid aminoalkenoic acid ester amides | 10 - 20,000 ppm | 10 - 20,000 ppm | 10 - 20,000 ppm |
| sodium or calcium saccharin salts | 10 - 20,000 ppm | 10 - 20,000 ppm | 10 - 20,000 ppm |
| cyclamate salts | 10 - 20,000 ppm | 10 - 20,000 ppm | 10 - 20,000 ppm |
| sodium, ammonium or calcium salt of 3,4-dihydro-6-methyl-1,2,3-oxathiazine-4-one-2,2-dioxide | 10 - 20,000 ppm | 10 - 20,000 ppm | 10 - 20,000 ppm |
| Acesulfame-K | 10 - 20,000 ppm | 10 - 20,000 ppm | 10 - 20,000 ppm |
| free acid form of saccharin | 10 - 20,000 ppm | 10 - 20,000 ppm | 10 - 20,000 ppm |
| Aspartame | 10 - 20,000 ppm | 10 - 20,000 ppm | 10 - 20,000 ppm |
| Alitame | 10 - 20,000 ppm | 10 - 20,000 ppm | 10 - 20,000 ppm |
| Neotame | 10 - 20,000 ppm | 10 - 20,000 ppm | 10 - 20,000 ppm |
| methyl esters of L-aspartyl-L-phenylglycerine and L-aspartyl-L-2,5-dihydrophenyl-glycine | 10 - 20,000 ppm | 10 - 20,000 ppm | 10 - 20,000 ppm |
| L-aspartyl-2,5-dihydro-L-phenylalanine | 10 - 20,000 ppm | 10 - 20,000 ppm | 10 - 20,000 ppm |
| L-aspartyl-L-(1-cyclohexen)-alanine | 10 - 20,000 ppm | 10 - 20,000 ppm | 10 - 20,000 ppm |
| Sucralose | 10 - 20,000 ppm | 10 - 20,000 ppm | 10 - 20,000 ppm |
| 1-chloro-1'-deoxysucrose | 10 - 20,000 ppm | 10 - 20,000 ppm | 10 - 20,000 ppm |
| 4-chloro-4-deoxy-alpha-D-galactopyranosyl-alpha-D-fructofuranoside | 10 - 20,000 ppm | 10 - 20,000 ppm | 10 - 20,000 ppm |
| 4-chloro-4-deoxygalactosucrose | 10 - 20,000 ppm | 10 - 20,000 ppm | 10 - 20,000 ppm |
| 4-chloro-4-deoxy-alpha-D-galactopyranosyl-1-chloro-1-deoxy-beta-D-fructo-f uranoside | 10 - 20,000 ppm | 10 - 20,000 ppm | 10 - 20,000 ppm |
| 4,1'-dichloro-4,1'-dideoxyflalactosucrose | 10 - 20,000 ppm | 10 - 20,000 ppm | 10 - 20,000 ppm |
| 1',6'-dichloro1',6'-dideoxysucrose | 10 - 20,000 ppm | 10 - 20,000 ppm | 10 - 20,000 ppm |
| 4-chloro-4-deoxy-alpha-D-galactopyranosyl-1,6-dichloro-1,6-dideoxy-beta-D-fructofuranoside | 10 - 20,000 ppm | 10 - 20,000 ppm | 10 - 20,000 ppm |
| 4,1',6'-trichloro-4,1',6'-trideoxygalactosucrose | 10 - 20,000 ppm | 10 - 20,000 ppm | 10 - 20,000 ppm |
| 4,6-dichloro-4,6-dideoxy-alpha-D-galactopyranosyl-6-chloro-6-deoxy-beta-D- fructofuranoside | 10 - 20,000 ppm | 10 - 20,000 ppm | 10 - 20,000 ppm |
| 4,6,6'-trichloro-4,6,6'-trideoxygalactosucrose | 10 - 20,000 ppm | 10 - 20,000 ppm | 10 - 20,000 ppm |
| 6,1',6'-trichloro-6,1',6'-trideoxysucrose | 10 - 20,000 ppm | 10 - 20,000 ppm | 10 - 20,000 ppm |
| 4,6-dichloro-4,6-dideoxy-alpha-D-galacto-pyranosyl-1,6-dichloro-1,6-dideox y-beta-D-fructofuranoside | 10 - 20,000 ppm | 10 - 20,000 ppm | 10 - 20,000 ppm |
| 4,6,1',6'-tetrachloro4,6,1',6'-tetradeoxygalacto-sucrose | 10 - 20,000 ppm | 10 - 20,000 ppm | 10 - 20,000 ppm |
| 4,6,1',6'-tetradeoxy-sucrose | 10 - 20,000 ppm | 10 - 20,000 ppm | 10 - 20,000 ppm |
| Thaumatin I and II | 10 - 20,000 ppm | 10 - 20,000 ppm | 10 - 20,000 ppm |
| Monatin | 10 - 20,000 ppm | 10 - 20,000 ppm | 10 - 20,000 ppm |
| | | | |

| B. Sour | | | |
|---|---|---|---|
| acetic acid | 0.00005 - 10% | 0.00005 - 10% | 0.00005 - 10% |
| adipic acid | 0.00005 - 10% | 0.00005 - 10% | 0.00005 - 10% |
| ascorbic acid | 0.00005 - 10% | 0.00005 - 10% | 0.00005 - 10% |
| butyric acid | 0.00005 - 10% | 0.00005 - 10% | 0.00005 - 10% |
| citric acid | 0.00005 - 10% | 0.00005 - 10% | 0.00005 - 10% |
| formic acid | 0.00005 - 10% | 0.00005 - 10% | 0.00005 - 10% |
| fumaric acid | 0.00005 - 10% | 0.00005 - 10% | 0.00005 - 10% |
| glyconic acid | 0.00005 - 10% | 0.00005 - 10% | 0.00005 - 10% |
| lactic acid | 0.00005 - 10% | 0.00005 - 10% | 0.00005 - 10% |
| phosphoric acid | 0.00005 - 10% | 0.00005 - 10% | 0.00005 - 10% |
| malic acid | 0.00005 - 10% | 0.00005 - 10% | 0.00005 - 10% |
| oxalic acid | 0.00005 - 10% | 0.00005 - 10% | 0.00005 - 10% |
| succinic acid | 0.00005 - 10% | 0.00005 - 10% | 0.00005 - 10% |
| tartaric acid | 0.00005 - 10% | 0.00005 - 10% | 0.00005 - 10% |

| Components | Coating and/or center fill | Saccharide Portion | Elastomeric Portion |
|---|---|---|---|
| C. Bitter/Astringent | | | |
| quinine | 0.01 - 100 ppm | 0.01 - 100 ppm | 0.01 - 100 ppm |
| naringin | 0.01 - 100 ppm | 0.01 - 100 ppm | 0.01 - 100 ppm |
| quassia | 0.01 - 100 ppm | 0.01 - 100 ppm | 0.01 - 100 ppm |
| phenyl thiocarbamide (PTC) | 0.01 - 100 ppm | 0.01 - 100 ppm | 0.01 - 100 ppm |
| 6-n-propylthiouracil (Prop) | 0.01 - 100 ppm | 0.01 - 100 ppm | 0.01 - 100 ppm |
| alum | 0.01 - 100 ppm | 0.01 - 100 ppm | 0.01 - 100 ppm |
| salicin | 0.01 - 100 ppm | 0.01 - 100 ppm | 0.01 - 100 ppm |
| caffeine | 0.01 - 100 ppm | 0.01 - 100 ppm | 0.01 - 100 ppm |
| Epigallocatechingallate | 0.01 - 100 ppm | 0.01 - 100 ppm | 0.01 - 100 ppm |
| | | | |

| D. Salty | | | |
|---|---|---|---|
| sodium chloride | 0.01 - 1% | 0.01 - 1% | 0.01 - 1% |
| calcium chloride | 0.01 - 1% | 0.01 - 1% | 0.01 - 1% |
| potassium chloride | 0.01 - 1% | 0.01 - 1% | 0.01 - 1% |
| 1-lysine | 0.01 - 1% | 0.01 - 1% | 0.01 - 1% |
| | | | |

| IV. Functional agents | | | |
|---|---|---|---|
| A. Surfactants | | | |
| salts of fatty acids selected from the group consisting of C₈-C₂₄ | 0.001 - 5% | 0.001 - 5% | 0.001 - 2% |
| palmitoleic acid | 0.001 - 5% | 0.001 - 5% | 0.001 - 2% |
| oleic acid | 0.001 - 5% | 0.001 - 5% | 0.001 - 2% |
| eleosteric acid | 0.001 - 5% | 0.001 - 5% | 0.001 - 2% |
| butyric acid | 0.001 - 5% | 0.001 - 5% | 0.001 - 2% |
| caproic acid | 0.001 - 5% | 0.001 - 5% | 0.001 - 2% |
| caprylic acid | 0.001 - 5% | 0.001 - 5% | 0.001 - 2% |
| capric acid | 0.001 - 5% | 0.001 - 5% | 0.001 - 2% |
| lauric acid | 0.001 - 5% | 0.001 - 5% | 0.001 - 2% |
| myristic acid | 0.001 - 5% | 0.001 - 5% | 0.001 - 2% |
| palmitic acid | 0.001 - 5% | 0.001 - 5% | 0.001 - 2% |
| stearic acid | 0.001 - 5% | 0.001 - 5% | 0.001 - 2% |
| ricinoleic acid | 0.001 - 5% | 0.001 - 5% | 0.001 - 2% |
| arachidic acid | 0.001 - 5% | 0.001 - 5% | 0.001 - 2% |
| behenic acid | 0.001 - 5% | 0.001 - 5% | 0.001 - 2% |
| lignoceric acid | 0.001 - 5% | 0.001 - 5% | 0.001 - 2% |
| cerotic acid | 0.001 - 5% | 0.001 - 5% | 0.001 - 2% |
| sulfated butyl oleate | 0.001 - 5% | 0.001 - 5% | 0.001 - 2% |
| medium and long chain fatty acid esters | 0.001 - 2% | 0.001 - 2% | 0.001 - 2% |
| sodium oleate | 0.001 - 2% | 0.001 - 2% | 0.001 - 2% |
| salts of fumaric acid | 0.001 - 2% | 0.001 - 2% | 0.001 - 2% |
| potassium glomate | 0.001 - 2% | 0.001 - 2% | 0.001 - 2% |
| organic acid esters of mono-and diglycerides | 0.001 - 2% | 0.001 - 2% | 0.001 - 2% |

| Components | Coating and/or center fill | Saccharide Portion | Elastomeric Portion |
|---|---|---|---|
| stearyl monoglyceridyl citrate | 0.001 - 2% | 0.001 - 2% | 0.001-2% |
| succistearin | 0.001 - 2% | 0.001 - 2% | 0.001 - 2% |
| dioctyl sodium sulfosuccinate | 0.001 - 2% | 0.001 - 2% | 0.001 - 2% |
| glycerol tristearate | 0.001 - 2% | 0.001 - 2% | 0.001 - 2% |
| lecithin | 0.001 - 2% | 0.001 - 2% | 0.001 - 2% |
| hydroxylated lecithin | 0.001 - 2% | 0.001 - 2% | 0.001 - 2% |
| sodium lauryl sulfate | 0.001 - 2% | 0.001 - 2% | 0.001 - 2% |
| acetylated monoglycerides | 0.001 - 2% | 0.001 - 2% | 0.001 - 2% |
| succinylated monoglycerides | 0.001 - 2% | 0.001 - 2% | 0.001 - 2% |
| monoglyceride citrate | 0.001 - 2% | 0.001 - 2% | 0.001 - 2% |
| ethoxylated mono- and diglycerides | 0.001 - 2% | 0.001 - 2% | 0.001 - 2% |
| sorbitan monostearate | 0.001 - 2% | 0.001 - 2% | 0.001 - 2% |
| calcium stearyl-2-lactylate | 0.001 - 2% | 0.001 - 2% | 0.001 - 2% |
| sodium stearyl lactylate | 0.001 - 2% | 0.001 - 2% | 0.001 - 2% |
| lactylated fatty acid esters of glycerol and propylene | 0.001 - 2% | 0.001 - 2% | 0.001 - 2% |
| glycerol-lactoesters of C8-C24 fatty acids | 0.001 - 2% | 0.001 - 2% | 0.001 - 2% |
| polyglycerol esters of C8-C24 fatty acids | 0.001 - 2% | 0.001 - 2% | 0.001 - 2% |
| propylene glycol alginate | 0.001 - 2% | 0.001 - 2% | 0.001 - 2% |
| sucrose C8-C24 fatty acid esters | 0.001 - 2% | 0.001 - 2% | 0.001 - 2% |
| diacetyl tartaric and citric acid esters of mono- and diglycerides | 0.001 - 2% | 0.001 - 2% | 0.001 - 2% |
| triacetin | 0.001 - 2% | 0.001 - 2% | 0.001 - 2% |
| sarcosinate surfactants | 0.001 - 2% | 0.001 - 2% | 0.001 - 2% |
| isethionate surfactants | 0.001 - 2% | 0.001 - 2% | 0.001 - 2% |
| tautate surfactants | 0.001 - 2% | 0.001 - 2% | 0.001 - 2% |
| pluronics | 0.001 - 2% | 0.001 - 2% | 0.001 - 2% |
| polyethylene oxide condensates of alkyl phenols | 0.001 - 2% | 0.001 - 2% | 0.001 - 2% |
| products derived from the condensation of ethylene oxide with the reaction product of propylene oxide and ethylene diamine | 0.001 - 2% | 0.001 - 2% | 0.001 - 2% |
| ethylene oxide condensates of aliphatic alcohols | 0.001 - 2% | 0.001 - 2% | 0.001 - 2% |
| long chain tertiary amine oxides | 0.001 - 2% | 0.001 - 2% | 0.001 - 2% |
| long chain tertiary phosphine oxides | 0.001 - 2% | 0.001 - 2% | 0.001 - 2% |
| long chain dialkyl sulfoxides | 0.001 - 2% | 0.001 - 2% | 0.001 - 2% |

| B. Breath freshening agents | | | |
|---|---|---|---|
| spearmint oil | 0.001 - 10% | 0.001 - 10% | 0.001 - 10% |
| peppermint oil | 0.001 - 10% | 0.001 - 10% | 0.001 - 10% |
| wintergreen oil | 0.001 - 10% | 0.001 - 10% | 0.001 - 10% |
| sassafras oil | 0.001 - 10% | 0.001 - 10% | 0.001 - 10% |
| chlorophyll oil | 0.001 - 10% | 0.001 - 10% | 0.001 - 10% |
| citral oil | 0.001 - 10% | 0.001 - 10% | 0.001 - 10% |
| geraniol oil | 0.001 - 10% | 0.001 - 10% | 0.001 - 10% |
| linalool | 0.001 - 10% | 0.001 - 10% | 0.001 - 10% |
| cardamom oil | 0.001 - 10% | 0.001 - 10% | 0.001 - 10% |
| clove oil | 0.001 - 10% | 0.001 - 10% | 0.001 - 10% |
| sage oil | 0.001 - 10% | 0.001 - 10% | 0.001 - 10% |
| carvacrol oil | 0.001 - 10% | 0.001 - 10% | 0.001 - 10% |
| eucalyptus oil | 0.001 - 10% | 0.001 - 10% | 0.001 - 10% |
| cardamom oil | 0.001 - 10% | 0.001 - 10% | 0.001 - 10% |
| magnolia bark extract oil | 0.001 - 10% | 0.001 - 10% | 0.001 - 10% |
| marjoram oil | 0.001 - 10% | 0.001 - 10% | 0.001 - 10% |
| cinnamon oil | 0.001 - 10% | 0.001 - 10% | 0.001 - 10% |
| lemon oil | 0.001 - 10% | 0.001 - 10% | 0.001 - 10% |
| lime oil | 0.001 - 10% | 0.001 - 10% | 0.001 - 10% |
| grapefruit oil | 0.001 - 10% | 0.001 - 10% | 0.001 - 10% |
| orange oil | 0.001 - 10% | 0.001 - 10% | 0.001 - 10% |
| cinnamic aldehyde | 0.001 - 10% | 0.001 - 10% | 0.001 - 10% |
| salicylaldehyde | 0.001 - 10% | 0.001 - 10% | 0.001 - 10% |
| menthol | 0.001 - 10% | 0.001 - 10% | 0.001 - 10% |
| carvone | 0.001 - 10% | 0.001 - 10% | 0.001 - 10% |
| iso-garrigol | 0.001 - 10% | 0.001 - 10% | 0.001 - 10% |
| anethole | 0.001 - 10% | 0.001 - 10% | 0.001 - 10% |
| zinc citrate | 0.01 - 25% | 0.01 - 25% | 0.1 - 15% |
| zinc acetate | 0.01 - 25% | 0.01 - 25% | 0.1 - 15% |
| zinc fluoride | 0.01 - 25% | 0.01 - 25% | 0.1 - 15% |
| zinc ammonium sulfate | 0.01 - 25% | 0.01 - 25% | 0.1 - 15% |
| zinc bromide | 0.01 - 25% | 0.01 - 25% | 0.1 - 15% |
| zinc iodide | 0.01 - 25% | 0.01 - 25% | 0.1 - 15% |
| zinc chloride | 0.01 - 25% | 0.01 - 25% | 0.1 - 15% |
| zinc nitrate | 0.01 - 25% | 0.01 - 25% | 0.1 - 15% |
| zinc flurosilicate | 0.01 - 25% | 0.01 - 25% | 0.1 - 15% |
| zinc gluconate | 0.01 - 25% | 0.01 - 25% | 0.1 - 15% |
| zinc tartarate | 0.01 - 25% | 0.01 - 25% | 0.1 - 15% |
| zinc succinate | 0.01 - 25% | 0.01 - 25% | 0.1 - 15% |
| zinc formate | 0.01 - 25% | 0.01 - 25% | 0.1 - 15% |
| zinc chromate | 0.01 - 25% | 0.01 - 25% | 0.1 - 15°C |
| zinc phenol sulfonate | 0.01 - 25% | 0.01 - 25% | 0.1 - 15% |
| zinc dithionate | 0.01 - 25% | 0.01 - 25% | 0.1 - 15% |
| zinc sulfate | 0.01 - 25% | 0.01 - 25% | 0.1 - 15% |
| silver nitrate | 0.01 - 25% | 0.01 - 25% | 0.1 - 15% |
| zinc salicylate | 0.01 - 25% | 0.01 - 25% | 0.1 - 15% |
| zinc glycerophosphate | 0.01 - 25% | 0.01 - 25% | 0.1 - 15% |
| copper nitrate | 0.01 - 25% | 0.01 - 25% | 0.1 - 15% |
| chlorophyll | 0.01 - 25% | 0.01 - 25% | 0.1 - 15% |
| copper chlorophyll | 0.01 - 25% | 0.01 - 25% | 0.1 - 15% |
| chlorophyllin | 0.01 - 25% | 0.01 - 25% | 0.1 - 15% |
| hydrogenated cottonseed oil | 0.5 - 5% | 0.5 - 70% | 0.5 - 15% |
| chlorine dioxide | 0.025 - 0.50 % | 0.025 - 0.50 % | 0.025 - 0.50 % |
| beta cyclodextrin | 0.1 - 5% | 0.1 - 5% | 0.1 - 5% |
| zeolite | 0.1 - 5% | 0.1 - 5% | 0.1 - 5% |
| silica-based materials | 0.1 - 5% | 0.1 - 5% | 0.1 - 5% |
| carbon-based materials | 0.1 - 5% | 0.1 - 5% | 0.1 - 5% |
| enzymes such as laccase, papain, krillase, amylase, glucose oxidase | 0.1 - 5% | 0.1 - 5% | 0.1 - 5% |
| geraniol | 0.001 - 10% | 0.001 - 10% | 0.001 - 10% |
| linalool | 0.001 - 10% | 0.001 - 10% | 0.001 - 10% |
| nerol | 0.001 - 10% | 0.001 - 10% | 0.001 - 10% |
| nerolidal | 0.001 - 10% | 0.001 - 10% | 0.001 - 10% |
| citronellol | 0.001 - 10% | 0.001 - 10% | 0.001 - 10% |
| heliotropine | 0.001 - 10% | 0.001 - 10% | 0.001 - 10% |
| methyl cyclopentelone | 0.001 - 10% | 0.001 - 10% | 0.001 - 10% |
| ethyl vanillin | 0.001 - 10% | 0.001 - 10% | 0.001 - 10% |
| ethyl maltol | 0.001 - 10% | 0.001 - 10% | 0.001 - 10% |
| furaneol | 0.00 - 10% | 0.001 - 10% | 0.001 - 10% |
| alliaceous compounds | 0.001 - 10% | 0.001 - 10% | 0.001 - 10% |
| rose type compounds such as phenethanol, phenylacetic acid, nerol, linalyl esters | 0.001 - 10% | 0.001 - 10% | 0.001 - 10% |
| jasmine | 0.001 - 10% | 0.001 - 10% | 0.001 - 10% |
| sandlewood | 0.001 - 10% | 0.001 - 10% | 0.001 - 10% |
| patchouli | 0.001 - 10% | 0.001 - 10% | 0.001 - 10% |
| cedarwood | 0.001 - 10% | 0.001 - 10% | 0.001 - 10% |
| | | | |

| C. Anti-microbial agents | | | |
|---|---|---|---|
| cetylpyridinium chloride | 0.01 - 1% | 0.01 - 1% | 0.01 - 1% |
| zinc compounds | 0.01 - 25% | 0.01 - 25% | 0.1 - 15% |
| copper compounds | 0.01 - 25% | 0.01 - 25% | 0.1 - 15% |
| | | | |

| D. Antibacterial agents | | | |
|---|---|---|---|
| chlorhexidine | 0.0025-2% | 0.0025-2% | 0.0025-2% |
| alexidine | 0.0025-2% | 0.0025-2% | 0.0025-2% |
| quaternary ammonium salts | 0.0025-2% | 0.0025-2% | 0.0025-2% |
| benzethonium chloride | 0.0025-2% | 0.0025-2% | 0.0025-2% |
| cetyl pyridinium chloride | 0.0025-2% | 0.0025-2% | 0.0025-2% |

| Components | Coating and/or center fill | Saccharide Portion | Elastomeric Portion |
|---|---|---|---|
| 2,4,4'-trichloro-2'-hydroxydiphenyl ether (triclosan) | 0.0025-2% | 0.0025-2% | 0.0025-2% |
| | | | |

| E. Anti-calculus agents | | | |
|---|---|---|---|
| pyrophosphates | 1 - 6% | 1-6% | 1 - 6% |
| triphosphates | 0.1 - 10% | 0.1 - 10% | 0.1 - 10% |
| polyphosphates | 0.1 - 10% | 0.1 - 10% | 0.1 - 10% |
| polyphosphonates | 0.1 - 10% | 0.1 - 10% | 0.1 - 10% |
| dialkali metal pyrophosphate salt | 1 - 6% | 1-6% | 1 - 6% |
| tetra alkali polyphosphate salt | 0.1 - 10% | 0.1 - 10% | 0.1 - 10% |
| tetrasodium pyrophosphate | 1 - 6% | 1 - 6% | 1 - 6% |
| tetrapotassium pyrophosphate | 1 - 6% | 1-6% | 1 - 6% |
| sodium tripolyphosphate | 0.1 - 10% | 0.1 - 10% | 0.1 - 10% |
| Sodium hexametaphosphate | 0.1 - 10% | 0.1 - 10% | 0.1 - 10% |
| | | | |

| F. Anti-plaque agents | | | |
|---|---|---|---|
| chlorhexidine | 0.0025-2% | 0.0025-2% | 0.0025 - 2% |
| triclosan | 0.01 - 2% | 0.01 - 2% | 0.01 - 2% |
| hexetidine | 0.01 - 2% | 0.01 - 2% | 0.01 - 2% |
| zinc citrate | 0.01 - 25% | 0.01 - 25% | 0.1 - 15% |
| essential oils | 0.001 - 10% | 0.001 - 10% | 0.001 - 10% |
| sodium lauryl sulfate | 0.001 - 2% | 0.001 - 2% | 0.001 - 2% |
| Epigallocatechingallate | 0.001-5% | 0.001-3% | 0.001 - 2% |
| | | | |

| G. Fluoride compounds | | | |
|---|---|---|---|
| sodium fluoride | 0.01 - 1% | 0.01 - 1% | 0.01 - 1% |
| sodium monofluorophosphate | 0.01 - 1% | 0.01 - 1% | 0.01 - 1% |
| stannous fluoride | 0.01 - 1% | 0.01 - 1% | 0.01 - 1% |
| | | | |

| H. Quaternary ammonium compounds | | | |
|---|---|---|---|
| Benzalkonium Chloride | 0.01 - 1% | 0.01 - 1% | 0.01 - 1% |
| Benzethonium Chloride | 0.01 - 1% | 0.01 - 1% | 0.01 - 1% |
| Cetalkonium Chloride | 0.01 - 1% | 0.01 - 1% | 0.01 - 1% |
| Cetrimide | 0.01 - 1% | 0.01 - 1% | 0.01 - 1% |
| Cetrimonium Bromide | 0.01 - 1% | 0.01 - 1% | 0.01 - 1% |
| Cetylpyridinium Chloride | 0.01 - 1% | 0.01 - 1% | 0.01 - 1% |
| Glycidyl Trimethyl Ammonium Chloride | 0.01 - 1% | 0.01 - 1% | 0.01 - 1% |
| Stearalkonium Chloride | 0.01 - 1% | 0.01 - 1% | 0.01 - 1% |
| | | | |

| I. Remineralization agents | | | |
|---|---|---|---|
| phosphopeptide-amorphous calcium phosphate | 0.1 - 5% | 0.1 - 5% | 0.1 - 5% |
| casein phosphoprotein-calcium | 0.1 - 5% | 0.1 - 5% | 0.1 - 5% |

| Components | Coating and/or center fill | Saccharide Portion | Elastomeric Portion |
|---|---|---|---|
| phosphate complex | | | |
| casein phosphopeptide-stabilized calcium phosphate | 0.1 - 5% | 0.1 - 5% | 0.1 - 5% |
| | | | |

| J. Pharmaceutical actives | | | |
|---|---|---|---|
| drugs or medicaments | 0.0001 - 10% | 0.0001 -10% | 0.0001 - 10% |
| vitamins and other dietary supplements | 0.0001 - 10% | 0.0001 - 10% | 0.0001 - 10% |
| minerals | 0.0001 - 10% | 0.0001 - 10% | 0.0001 - 10% |
| caffeine | 0.0001 - 10% | 0.0001 - 10% | 0.0001 -10% |
| nicotine | 0.0001 - 10% | 0.0001 - 10% | 0.0001 - 10% |
| fruit juices | 2 - 10% | 2 - 60% | 1 - 15% |
| | | | |

| K. Micronutrients | | | |
|---|---|---|---|
| vitamin A | 0.0001 - 10% | 0.0001 - 10% | 0.0001 - 10% |
| vitamin D | 0.0001 - 10% | 0.0001 - 10% | 0.0001 - 10% |
| vitamin E | 0.0001 - 10% | 0.0001 - 10% | 0.0001 - 10% |
| vitamin K | 0.0001 - 10% | 0.0001 - 10% | 0.0001 - 10% |
| vitamin C (ascorbic acid) | 0.0001 - 10% | 0.0001 - 10% | 0.0001 - 10% |
| B vitamins (thiamine or B1, riboflavoin or B2, niacin or B3, pyridoxine or B6, folic acid or B9, cyanocobalimin or B 12, pantothenic acid, biotin) | 0.0001 - 10% | 0.0001 - 10% | 0.0001 - 10% |
| sodium | 0.0001 - 10% | 0.0001 - 10% | 0.0001 - 10% |
| magnesium | 0.0001 - 10% | 0.0001 -10% | 0.0001 - 10% |
| chromium | 0.0001 - 10% | 0.0001 - 10% | 0.0001 - 10% |
| iodine | 0.0001 - 10% | 0.0001 - 10% | 0.0001 - 10% |
| iron | 0.0001 - 10% | 0.0001 -10% | 0.0001 - 10% |
| manganese | 0.0001 - 10% | 0.0001 - 10% | 0.0001 - 10% |
| calcium | 0.0001 - 10% | 0.0001 - 10% | 0.0001 - 10% |
| copper | 0.0001 - 10% | 0.0001 - 10% | 0.0001 - 10% |
| fluoride | 0.0001 - 10% | 0.0001 - 10% | 0.0001 - 10% |
| potassium | 0.0001 - 10% | 0.0001 - 10% | 0.0001 - 10% |
| phosphorous | 0.0001 - 10% | 0.0001 - 10% | 0.0001 - 10% |
| molybdenum | 0.0001 - 10% | 0.0001 - 10% | 0.0001 - 10% |
| selenium | 0.0001 - 10% | 0.0001 - 10% | 0.0001 - 10% |
| zinc | 0.0001 - 10% | 0.0001 - 10% | 0.0001 - 10% |
| L-carnitine | 0.0001 - 10% | 0.0001 - 10% | 0.0001 - 10% |
| choline | 0.0001 - 10% | 0.0001 - 10% | 0.0001 - 10% |
| coenzyme Q10 | 0.0001 - 10% | 0.0001 - 10% | 0.0001 - 10% |
| alpha-lipoic acid | 0.0001 - 10% | 0.0001 - 10% | 0.0001 - 10% |
| omega-3-fatty acids | 0.0001 - 10% | 0.0001 -10% | 0.0001 - 10% |
| pepsin | 0.0001 - 10% | 0.0001 - 10% | 0.0001 - 10% |
| phytase | 0.0001 - 10% | 0.0001 - 10% | 0.0001 - 10% |
| trypsin | 0.0001 - 10% | 0.0001 - 10% | 0.0001 - 10% |

| Components | Coating and/or center fill | Saccharide Portion | Elastomeric Portion |
|---|---|---|---|
| lipases | 0.0001 - 10% | 0.0001 - 10% | 0.0001 -10% |
| proteases | 0.0001 - 10% | 0.0001 - 10% | 0.0001 -10% |
| cellulases | 0.0001 - 10% | 0.0001 - 10% | 0.0001 - 10% |
| ascorbic acid | 0.0001 - 10% | 0.0001 - 10% | 0.0001 - 10% |
| citric acid | 0.0001 - 0% | 0.0001 - 10% | 0.0001 - 10% |
| rosemary oil | 0.0001 - 10% | 0.0001 - 10% | 0.0001 - 10% |
| vitamin A | 0.0001 - 10% | 0.0001 - 10% | 0.0001 - 10% |
| vitamin E phosphate | 0.0001 - 10% | 0.0001 - 10% | 0.0001 - 10% |
| tocopherols | 0.0001 - 10% | 0.0001 - 10% | 0.0001 - 10% |
| di-alpha-tocopheryl phosphate | 0.0001 - 10% | 0.0001 - 10% | 0.0001 - 10% |
| tocotrienols | 0.0001 - 10% | 0.0001 - 10% | 0.0001 - 10% |
| alpha lipoic acid | 0.0001 - 10% | 0.0001 - 10% | 0.0001 - 10% |
| dihydrolipoic acid | 0.0001 - 10% | 0.0001 - 10% | 0.0001 - 10% |
| xanthophylls | 0.0001 - 10% | 0.0001 - 10% | 0.0001 - 10% |
| beta cryptoxanthin | 0.0001 - 10% | 0.0001 - 10% | 0.0001 - 10% |
| lycopene | 0.0001 - 10% | 0.0001 - 10% | 0.0001 - 10% |
| lutein | 0.0001 - 10% | 0.0001 - 10% | 0.0001 - 10% |
| zeaxanthin | 0.0001 - 10% | 0.0001 - 10% | 0.0001 - 10% |
| beta-carotene | 0.0001 - 10% | 0.0001 - 10% | 0.0001 - 10% |
| carotenes | 0.0001 - 10% | 0.0001 - 10% | 0.0001 - 10% |
| mixed carotenoids | 0.0001 - 10% | 0.0001 - 10% | 0.0001 - 10% |
| polyphenols | 0.0001 - 10% | 0.0001 - 10% | 0.0001 - 10% |
| flavonoids | 0.0001 - 10% | 0.0001 - 10% | 0.0001 - 10% |
| cartotenoids | 0.0001 - 10% | 0.0001 - 10% | 0.0001 - 10% |
| chlorophyll | 0.0001 - 10% | 0.0001 - 10% | 0.0001 - 10% |
| chlorophyllin | 0.0001 - 10% | 0.0001 - 10% | 0.0001 - 10% |
| fiber | 0.0001 - 10% | 0.0001 - 10% | 0.0001 - 10% |
| anthocyanins | 0.0001 - 10% | 0.0001 - 10% | 0.0001 - 10% |
| cyaniding | 0.0001 - 10% | 0.0001 - 10% | 0.0001 - 10% |
| delphinidin | 0.0001 - 10% | 0.0001 - 10% | 0.0001 - 10% |
| malvidin | 0.0001 - 10% | 0.0001 - 10% | 0.0001 - 10% |
| pelargonidin | 0.0001 - 10% | 0.0001 - 10% | 0.0001 - 10% |
| peonidin | 0.0001 - 10% | 0.0001 - 10% | 0.0001 - 10% |
| petunidin | 0.0001 - 10% | 0.0001 - 10% | 0.0001 - 10% |
| flavanols | 0.0001 - 10% | 0.0001 - 10% | 0.0001 - 10% |
| flavonols | 0.0001 - 10% | 0.0001 - 10% | 0.0001 - 10% |
| catechin | 0.0001 - 10% | 0.0001 - 10% | 0.0001 - 10% |
| epicatechin | 0.0001 - 10% | 0.0001 - 10% | 0.0001 - 10% |
| epigallocatechin | 0.0001 - 10% | 0.0001 - 10% | 0.0001 - 10% |
| epigallocatechingallate | 0.0001 - 10% | 0.0001 - 10% | 0.0001 - 10% |
| theaflavins | 0.0001 - 10% | 0.0001 - 10% | 0.0001 - 10% |
| thearubigins | 0.0001 - 10% | 0.0001 - 10% | 0.0001 - 10% |
| proanthocyanins | 0.0001 - 10% | 0.0001 - 10% | 0.0001 - 10% |
| quercetin | 0.0001 - 10% | 0.0001 - 10% | 0.0001 - 10% |
| kaempferol | 0.0001 - 10% | 0.0001 - 10% | 0.0001 - 10% |
| myricetin | 0.0001 - 10% | 0.0001 - 10% | 0.0001 - 10% |
| isorhamnetin | 0.0001 - 10% | 0.0001 - 10% | 0.0001 - 10% |
| flavononeshesperetin | 0.0001 - 10% | 0.0001 - 10% | 0.0001 - 10% |
| naringenin | 0.0001 - 10% | 0.0001 - 10% | 0.0001 - 10% |
| eriodictyol | 0.0001 - 10% | 0.0001 - 10% | 0.0001 - 10% |
| tangeretin | 0.0001 - 10% | 0.0001 - 10% | 0.0001 - 10% |
| flavones | 0.0001 - 10% | 0.0001 - 10% | 0.0001 - 10% |
| apigenin | 0.0001 - 10% | 0.0001 - 10% | 0.0001 - 10% |
| luteolin | 0.0001 - 10% | 0.0001 - 10% | 0.0001 - 10% |
| lignans | 0.0001 - 10% | 0.0001 - 10% | 0.0001 - 10% |
| phytoestrogens | 0.0001 - 10% | 0.0001 - 10% | 0.0001 - 10% |
| resveratrol | 0.0001 - 10% | 0.0001 - 10% | 0.0001 - 10% |
| isoflavones | 0.0001 - 10% | 0.0001 - 10% | 0.0001 - 10% |
| daidzein | 0.0001 - 10% | 0.0001 - 10% | 0.0001 - 10% |
| genistein | 0.0001 - 10% | 0.0001 - 10% | 0.0001 - 10% |
| soy isoflavones | 0.0001 - 10% | 0.0001 - 10% | 0.0001 - 10% |
| | | | |

| L. Throat care actives | | | |
|---|---|---|---|
| (1) analgesics, anesthetics, antipyretic and antiinflammatory agents | | | |
| menthol | 10-500 ppm | 10-500 ppm | 500 - 20,000 ppm |
| phenol | 0.1 - 10% | 0.1 - 50% | 0.1 - 20% |
| hexylresorcinol | 0.1 - 10% | 0.1 - 50% | 0.1 - 20% |
| benzocaine | 0.1 - 10% | 0.1 - 50% | 0.1 - 20% |
| dyclonine hydrochloride | 0.1 - 10% | 0.1 - 50% | 0.1 - 20% |
| benzyl alcohol | 0.1 - 10% | 0.1 - 50% | 0.1 - 20% |
| salicyl alcohol | 0.1 - 10% | 0.1 - 50% | 0.1 - 20% |
| acetaminophen | 0.1 - 10% | 0.1 - 50% | 0.1 - 20% |
| aspirin | 0.1 - 10% | 0.1 - 50% | 0.1 - 20% |
| diclofenac | 0.1 - 10% | 0.1 - 50% | 0.1 - 20% |
| diflunisal | 0.1 - 10% | 0.1 - 50% | 0.1 - 20% |
| etodolac | 0.1 - 10% | 0.1 - 50% | 0.1 - 20% |
| fenoprofen | 0.1 - 10% | 0.1 - 50% | 0.1 - 20% |
| flurbiprofen | 0.1 - 10% | 0.1 - 50% | 0.1 - 20% |
| ibuprofen | 0.1 - 10% | 0.1 - 50% | 0.1 - 20% |
| ketoprofen | 0.1 - 10% | 0.1 - 50% | 0.1 - 20% |
| ketorolac | 0.1 - 10% | 0.1 - 50% | 0.1 - 20% |
| nabumetone | 0.1 - 10% | 0.1 - 50% | 0.1 - 20% |
| naproxen | 0.1 - 10% | 0.1 - 50% | 0.1 - 20% |
| piroxicam | 0.1 - 10% | 0.1 - 50% | 0.1 - 20% |
| caffeine | 0.0001 - 10% | 0.0001 -10% | 0.0001 - 10% |
| lidocaine | 0.1 - 10% | 0.1 - 50% | 0.1 - 20% |
| benzocaine | 0.1 - 10% | 0.1 - 50% | 0.1 - 20% |
| phenol | 0.1 - 10% | 0.1 - 50% | 0.1 - 20% |
| dyclonine | 0.1 - 10% | 0.1 - 50% | 0.1 - 20% |
| benzonotate | 0.1 - 10% | 0.1 - 50% | 0.1 - 20% |

| Components | Coating and/or center fill | Saccharide Portion | Elastomeric Portion |
|---|---|---|---|
| (2) demulcents | | | |
| slippery elm bark | 0.1 - 10% | 0.1 - 10% | 0.1 - 10% |
| pectin | 0.1 - 10% | 0.1 - 10% | 0.1 - 10% |
| gelatin | 0.1 - 10% | 0.1 - 10% | 0.1 - 10% |

| (3) antiseptics | | | |
|---|---|---|---|
| cetylpyridinium chloride | 0.01 - 1% | 0.01 - 1% | 0.01 - 1% |
| domiphen bromide | 0.01 - 1% | 0.01 - 1% | 0.01 - 1% |
| dequalinium chloride | 0.01 - 1% | 0.01 - 1% | 0.01 - 1% |

| (4) antitussives | | | |
|---|---|---|---|
| chlophedianol hydrochloride | 0.0001 - 2% | 0.0001 - 2% | 0.0001 - 2% |
| codeine | 0.0001 - 2% | 0.0001 - 2% | 0.0001 - 2% |
| codeine phosphate | 0.0001 - 2% | 0.0001 - 2% | 0.0001 - 2% |
| codeine sulfate | 0.0001 - 2% | 0.0001 - 2% | 0.0001 - 2% |
| dextromethorphan | 0.0001 - 2% | 0.0001 - 2% | 0.0001 - 2% |
| dextromethorphan hydrobromide | 0.0001 - 2% | 0.0001 - 2% | 0.0001 - 2% |
| diphenhydramine citrate | 0.0001 - 2% | 0.0001 - 2% | 0.0001 - 2% |
| diphenhydramine hydrochloride | 0.0001 - 2% | 0.0001 - 2% | 0.0001 - 2% |
| dextrorphan | 0.0001 - 2% | 0.0001 - 2% | 0.0001 - 2% |
| diphenhydramine | 0.0001 - 2% | 0.0001 - 2% | 0.0001 - 2% |
| hydrocodone | 0.0001 - 2% | 0.0001 - 2% | 0.0001 - 2% |
| noscapine | 0.0001 - 2% | 0.0001 - 2% | 0.0001 - 2% |
| oxycodone | 0.0001 - 2% | 0.0001 - 2% | 0.0001 - 2% |
| pentoxyverine | 0.0001 - 2% | 0.0001 - 2% | 0.0001 - 2% |

| (5) throat soothing agents | | | |
|---|---|---|---|
| honey | 0.5 - 25% | 0.5 - 90% | 0.5 - 15% |
| propolis | 0.1 - 10% | 0.1 - 10% | 0.1 - 10% |
| aloe vera | 0.1 - 10% | 0.1 - 10% | 0.1 - 10% |
| glycerine | 0.1 - 10% | 0.1 - 10% | 0.1 - 10% |
| menthol | 10-500 ppm | 10-500 ppm | 500 - 20,000 ppm |

| (6) cough suppressants | | | |
|---|---|---|---|
| codeine | 0.0001 - 2% | 0.0001 - 2% | 0.0001 - 2% |
| antihistamines | 0.0001 - 2% | 0.0001 - 2% | 0.0001 - 2% |
| dextromethorphan | 0.0001 - 2% | 0.0001 - 2% | 0.0001 - 2% |
| isoproterenol | 0.0001 - 2% | 0.0001 - 2% | 0.0001 - 2% |

| (7) expectorants | | | |
|---|---|---|---|
| ammonium chloride | 0.0001 - 2% | 0.0001 - 2% | 0.0001 - 2% |
| guaifenesin | 0.0001 - 2% | 0.0001 - 2% | 0.0001 - 2% |
| ipecac fluid extract | 0.0001 - 2% | 0.0001 - 2% | 0.0001 - 2% |
| potassium iodide | 0.0001 - 2% | 0.0001 - 2% | 0.0001 - 2% |

| (8) mucolytics | | | |
|---|---|---|---|
| acetylcycsteine | 0.0001 - 2% | 0.0001 - 2% | 0.0001 - 2% |
| ambroxol | 0.0001 - 2% | 0.0001 - 2% | 0.0001 - 2% |
| bromhexine | 0.0001 - 2% | 0.0001 - 2% | 0.0001 - 2% |

| (9) antihistamines | | | |
|---|---|---|---|
| acrivastine | 0.05 - 10% | 0.05 - 10% | 0.05 - 10% |

| Components | Coating and/or center fill | Saccharide Portion | Elastomeric Portion |
|---|---|---|---|
| azatadine | 0.05 - 10% | 0.05 - 10% | 0.05 - 10% |
| brompheniramine | 0.05 - 10% | 0.05 - 10% | 0.05 - 10% |
| chlorpheniramine | 0.05 - 10% | 0.05 - 10% | 0.05 - 10% |
| clemastine | 0.05 - 10% | 0.05 - 10% | 0.05 - 10% |
| cyproheptadine | 0.05 - 10% | 0.05 - 10% | 0.05 - 10% |
| dexbrompheniramine | 0.05 - 10% | 0.05 - 10% | 0.05 - 10% |
| dimenhydrinate | 0.05 - 10% | 0.05 - 10% | 0.05 - 10% |
| diphenhydramine | 0.05 - 10% | 0.05 - 10% | 0.05 - 10% |
| doxylamine | 0.05 - 10% | 0.05 - 10% | 0.05 - 10% |
| hydroxyzine | 0.05 - 10% | 0.05 - 10% | 0.05 - 10% |
| meclizine | 0.05 - 10% | 0.05 - 10% | 0.05 - 10% |
| phenindamine | 0.05 - 10% | 0.05 - 10% | 0.05 - 10% |
| phenyltoloxamine | 0.05 - 10% | 0.05 - 10% | 0.05 - 10% |
| promethazine | 0.05 - 10% | 0.05 - 10% | 0.05 - 10% |
| pyrilamine | 0.05 - 10% | 0.05 - 10% | 0.05 - 10% |
| tripelennamine | 0.05 - 10% | 0.05 - 10% | 0.05 - 10% |
| triprolidine | 0.05 - 10% | 0.05 - 10% | 0.05 - 10% |
| astemizole | 0.05 - 10% | 0.05 - 10% | 0.05 - 10% |
| cetirizine | 0.05 - 10% | 0.05 - 10% | 0.05 - 10% |
| ebastine | 0.05 - 10% | 0.05 - 10% | 0.05 - 10% |
| fexofenadine | 0.05 - 10% | 0.05 - 10% | 0.05 - 10% |
| loratidine | 0.05 - 10% | 0.05 - 10% | 0.05 - 10% |
| terfenadine | 0.05 - 10% | 0.05 - 10% | 0.05 - 10% |

| (10) nasal decongestants | | | |
|---|---|---|---|
| phenylpropanotamine | 0.1 - 10% | 0.1 - 50% | 0.1 - 20% |
| pseudoephedrine | 0.1 - 10% | 0.1 - 50% | 0.1 - 20% |
| ephedrine | 0.1 - 10% | 0.1 - 50% | 0.1 - 20% |
| phenylephrine | 0.1 - 10% | 0.1 - 50% | 0.1 - 20% |
| oxymetazoline | 0.1 - 10% | 0.1 - 50% | 0.1 - 20% |
| menthol | 0.1 - 10% | 0.1 - 50% | 0.1 - 20% |
| camphor | 0.1 - 10% | 0.1 - 50% . | 0.1 - 20% |
| borneol | 0.1 - 10% | 0.1 - 50% | 0.1 - 20% |
| ephedrine | 0.1 - 10% | 0.1 - 50% | 0.1 - 20% |
| eucalyptus oil | 0.001 - 10% | 0.001 - 10% | 0.001 - 10% |
| peppermint oil | 0.001 - 10% | 0.001 - 10% | 0.001 - 10% |
| methyl salicylate | 0.001 - 10% | 0.001 - 10% | 0.001 - 10% |
| bornyl acetate | 0.001 - 10% | 0.001 - 10% | 0.001 - 10% |
| lavender oil | 0.001 - 10% | 0.001 - 10% | 0.001 - 10% |
| wasabi extracts | 0.001 - 10% | 0.001 - 10% | 0.001 - 10% |
| horseradish extracts | 0.001 - 10% | 0.001 - 10% | 0.001 - 10% |
| | | | |

| M. Tooth whitening/ Stain removing agents | | | |
|---|---|---|---|
| surfactants | 0.001 - 5% | 0.001 - 5% | 0.001 - 5% |
| chelators | 0.1 - 10% | 0.1 - 10% | 0.1 - 10% |
| abrasives | 0.1 - 5% | 0.1 - 5% | 0.1 - 20% |

| Components | Coating and/or center fill | Saccharide Portion | Elastomeric Portion |
|---|---|---|---|
| oxidizing agents | 0.1 - 5% | 0.1 - 5% | 0.1 - 5% |
| hydrolytic agents | 0.1 - 5% | 0.1 - 5% | 0.1 - 5% |
| | | | |

| N. Energy boosting agents | | | |
|---|---|---|---|
| caffeine | 0.0001 - 10% | 0.0001 - 10% | 0.0001- 10% |
| vitamins | 0.0001 - 10% | 0.0001 - 10% | 0.0001 - 10% |
| minerals | 0.0001 - 10% | 0.0001 - 10% | 0.0001 - 10% |
| amino acids | 0.0001 - 10% | 0.0001 - 10% | 0.0001 - 10% |
| ginseng extract | 0.0001 - 10% | 0.0001 - 10% | 0.0001 - 10% |
| ginko extract | 0.0001 - 10% | 0.0001 - 10% | 0.0001 - 10% |
| guarana extract | 0.0001 - 10% | 0.0001 - 10% | 0.0001 - 10% |
| green tea extract | 0.0001 - 10% | 0.0001 - 10% | 0.0001 - 10% |
| taurine | 0.0001 - 10% | 0.0001 - 10% | 0.0001 - 10% |
| kola nut extract | 0.0001 - 10% | 0.0001 - 10% | 0.0001 - 10% |
| yerba mate leaf | 0.0001 - 10% | 0.0001 - 10% | 0.0001 - 10% |
| Niacin | 0.0001 - 10% | 0.0001 - 10% | 0.0001 - 10% |
| rhodiola root extract | 0.0001 - 10% | 0.0001 - 10% | 0.0001 - 10% |
| | | | |

| O. Concentration boosting agents | | | |
|---|---|---|---|
| caffeine | 0.0001 - 10% | 0.0001 - 10% | 0.0001 - 10% |
| ginko extract | 0.0001 - 10% | 0.0001 - 10% | 0.0001 - 10% |
| gotu cola (centella asiatica) | 0.0001 - 10% | 0.0001 - 10% | 0.0001 - 10% |
| German chamomile | 0.0001 - 10% | 0.0001 - 10% | 0.0001 - 10% |
| avina sativa | 0.0001 - 10% | 0.0001 - 10% | 0.0001 - 10% |
| phosphatidyl serine | 0.0001 - 10% | 0.0001 - 10% | 0.0001 - 10% |
| aspalathus linearis | 0.0001 - 10% | 0.0001 - 10% | 0.0001 - 10% |
| pregnenolone | 0.0001 - 10% | 0.0001 - 10% | 0.0001 - 10% |
| rhodiola root extract | 0.0001 - 10% | 0.0001 - 10% | 0.0001 - 10% |
| theanine | 0.0001 - 10% | 0.0001 - 10% | 0.0001 - 10% |
| vinpocetine | 0.0001 - 10% | 0.0001 - 10% | 0.0001 - 10% |
| | | | |

| P. Appetite suppressants | | | |
|---|---|---|---|
| caffeine | 0.0001 - 10% | 0.0001 - 10% | 0.0001 - 10% |
| guarana extract | 0.0001 - 10% | 0.0001 - 10% | 0.0001 - 10% |
| hoodia gordonii | 0.0001 - 10% | 0.0001 - 10% | 0.0001 - 10% |
| fllucornannan | 0.0001 - 10% | 0.0001 - 10% | 0.0001 - 10% |
| calcium | 0.0001 - 10% | 0.0001 - 10% | 0.0001 - 10% |
| garcinia cambogia extract | 0.0001 - 10% | 0.0001 - 10% | 0.0001 - 10% |
| n-acetyl-tyrosine | 0.0001 - 10% | 0.0001 - 10% | 0.0001 - 10% |
| soy phospholipids | 0.0001 - 10% | 0.0001 - 10% | 0.0001 - 10% |
| Green tea extract (epigallocatechingallate) | 0.0001 - 10% | 0.0001 - 10% | 0.0001 - 10% |
| | | | |

| V. Colors | | | |
|---|---|---|---|
| Annatto extract | 0.5 - 10% | 0.5 - 20% | 0.5 - 10% |

| Components | Coating and/or center fill | Saccharide Portion | Elastomeric Portion |
|---|---|---|---|
| Beta-carotene | 0.5 - 10% | 0.5 - 20% | 0.5 - 10% |
| Canthaxanthin | 0.5 - 10% | 0.5 - 20% | 0.5 - 10% |
| Grape color extract | 0.5 - 10% | 0.5 - 20% | 0.5 - 10% |
| Turmeric oleoresin | 0.5 - 10% | 0.5 - 20% | 0.5 - 10% |
| B-Apo-8'-carotenal | 0.5 - 10% | 0.5 - 20% | 0.5 - 10% |
| Beet powder | 0.5 - 10% | 0.5 - 20% | 0.5 - 10% |
| Caramel color | 0.5 - 10% | 0.5 - 20% | 0.5 - 10% |
| Carmine | 0.5 - 10% | 0.5 - 20% | 0.5 - 10% |
| Cochineal extract | 0.5 - 10% | 0.5 - 20% | 0.5 - 10% |
| Grape skin extract | 0.5 - 10% | 0.5 - 20% | 0.5 - 10% |
| Saffron | 0.5 - 10% | 0.5 - 20% | 0.5 - 10% |
| Tumeric | 0.5 - 10% | 0.5 - 20% | 0.5 - 10% |
| Titanium dioxide | 0.05 - 2% | 0.05 - 2% | 0.05 - 2% |
| F.D. & C. Blue No.1 | 0.05 - 2% | 0.05 - 2% | 0.05 - 2% |
| F.D.& C. Blue No.2 | 0.05 - 2% | 0.05 - 2% | 0.05 - 2% |
| F.D.& C. Green No. 1 | 0.05 - 2% | 0.05 - 2% | 0.05 - 2% |
| F.D. & C. Red No.40 | 0.05 - 2% | 0.05 - 2% | 0.05 - 2% |
| F.D. & C. Red No.3 | 0.05 - 2% | 0.05 - 2% | 0.05 - 2% |
| F.D. & C. Yellow No.6 | 0.05 - 2% | 0.05 - 2% | 0.05 - 2% |
| F.D. & C. Yellow No.5 | 0.05 - 2% | 0.05 - 2% | 0.05 - 2% |

As mentioned above, some embodiments described herein may include more than one duality in the confectionery composition. Such compositions may be referred to as multi-modality compositions. In some embodiments, more than one duality of the same type may be included, such as, two different flavor dualities. Alternatively, different types of dualities may be combined in a single confectionery composition. For example, a flavor duality and a sensation duality may be used together. Further, three or even four of the different duality types may be included in one confectionery composition in some embodiments.

### Texture Modification

In some embodiments, the texture of confectionery compositions are varied by varying the ratios and/or characteristics of the various portions, by changing processing parameters, or by including a texture modifying component.

In some embodiments, a confectionery composition comprises 5% to 95% of a saccharide component. In some embodiments, a confectionery composition comprises 5% to 45% of an elastomeric component. In some embodiments, a confectionery composition comprises 0.1% to 6% of a fat component. In some embodiments, a confectionery composition comprises 0.1% to 10% of a polymer component such as gelatin, starches and proteins. In some embodiments, a confectionery composition comprises 0.1% to 30% of a soluble powder component such as sugar and polyols. In some embodimenst, a confectionery composition comprises 0.1% to 20% of an insoluble powders component. In some embodiments, a confectionery composition contains 0.1% to 8% of moisture.

When describing the texture profile of a confectionery composition, both analytical/instrumentation-based measures and sensory evaluation measures can be used. Analytical/instrumentation-based measures can include, but are not limited to, penetrometers, textureometers, tenderometers, universal testing machines, and the Texture Analyzer available from Stable MicroSystems of Surrey, United Kingdom. Sensory evaluation measures can include, but are not limited to, texture profiling and quantitative descriptive analysis. In some embodiments, the methods of measuring texture for a confectionery composition include a temporal component that measures the texture over time while the confectionery composition is being consumed. In other embodiments, the methods of measuring texture elucidate a change in the character of the texture over time. This change in the character of the texture over time can be used to define the texture transformation of the confectionery composition.

For example, the compression strengths of confectionery compositions having different initial desired textures provided by selecting different ratio of a saccharide component and elastomeric component are measured using Instron model 4204, e.g., Slab Gum (13 KgF), Coated pellet gum (24 KgF), Candy/Gum base (65 /35 %) mixture (15 KgF), Candy/Gum base (75/25 %) mixture (27 KgF), Candy/Gum base (85/15%) mixture(34 KgF), Hard Candy (45 KgF).

In some embodiments, varying the ratios and/or characteristics of the portions can vary the texture of the finished confectionery composition. For example, a confectionery composition comprising 60% to 80% w/w of a saccharide composition wherein the saccharide composition is a hard boiled candy with less than 3% moisture will provide a harder initial texture similar to hard candy as compared to a confectionery composition comprising only 20% to 30% w/w of the same saccharide composition. Alternatively, a confectionery composition comprising 40% to 50% w/w of a saccharide composition wherein the saccharide composition is a hard boiled candy with 2% moisture will provide a harder initial texture than a confectionery composition with the same amount (40% - 50% w/w) of a hard boiled candy with 5% moisture. Similarly, a confectionery composition comprising 30% to 40% w/w of a saccharide composition wherein the saccharide composition is a hard boiled candy with 5% moisture will provide a harder initial texture than a confectionery composition with the same amount (40 - 50% w/w) of a chewy candy such as taffy wherein the taffy includes approximately 12% fat and about 8% moisture.

In some embodiments, varying the characteristics of the elastomeric portion can vary the texture of the confectionery composition. For example, an elastomeric portion including low melting point fats can provide a softer confectionery composition when combined with a saccharide portion than an elastomeric portion including high melting point fats. Similarly, elastomeric portions containing lower levels of plasticizers and softeners may provide softer confectionery compositions when combined with saccharide portions than elastomeric portions including higher levels of plasticizers and softeners.

The confectionery composition may include a center-fill. The center-fill can be liquid, semi-solid, solid or gaseous. In some embodiments, a confectionery composition with a liquid center fill has a softer initial texture and requires less energy to bite through than a confectionery composition without a liquid center fill.

The solid center can include particulates. Particulates can include, but are not limited to nuts; seeds; cocoa beans; coffee beans; milk powders; fruit-containing particles such as restructured fruit as described in U.S. Patent 6,027,758; freeze dried fruit; freeze dried vegetables; fat particles; cocoa powder; sucrose; starch; polyols such as xylitol, erythritol, sorbitol, mannitol, maltitol, isomalt, hydrogenated starch hydrolysates; waxes; and combinations thereof.

The solid center can include particles onto which other materials have been complexed. In some embodiments, the solid particle can include an absorbent material to which a second material is absorbed. In some embodiments, the solid particle can include an adsorbent material to which a second material is adsorbed. The solid particle can include a complexation material to which a second material is complexed. In some embodiments, silica particles can absorb at least a second material to form a particulate solid interior portion. Cyclodextrin particles can complex with at least a second material to form a particulate solid interior portion.

Where the solid center can change to a liquid, the solid center can include a mixture of invertase and sucrose such invertase operates on sucrose to form liquid invert sugar resulting in a liquid interior portion over time. In some embodiments, the center can be a fat with melting characteristics such that at manufacturing temperatures the fat is solid and then melts to become liquid at storage temperatures. The solid center can include liquid-filled gelatin or sucrose beads that release liquid when ruptured or disrupted.

The solid center can include a unitary or particulate solid confectionery composition. Such confectionery compositions can include, but are not limited to, chocolate, compound coating, carob coating, cocoa butter, butter fat, hydrogenated vegetable fat, illipe butter, fondant including fondant-based cremes, fudge, frappe, caramel, nougat, compressed tablet, candy floss (also known as cotton candy), marzipan, hard boiled candy, gummy candy, jelly beans, toffees, jellies including pectin-based gels, jams, preserves, butterscotch, nut brittles or croquant, candied fruit, marshmallow, pastilles, pralines or nougats, flour or starch confectionery, truffles, nonpareils, bon bons, after-dinner mints, fourres, nut pastes, peanut butter, chewing gum, kisses, angel kisses, montelimart, nougatine, fruit chews, Turkish delight, hard gums, soft gums, starch jellies, gelatin jellies, agar jellies, persipan, coconut paste, coconut ice, lozenges, cachous, crème paste, dragees, sugared nuts, sugared almonds, comfits, aniseed balls, licorice, licorice paste, chocolate spreads, chocolate crumb, and combinations thereof.

The liquid center can be aqueous while in other embodiments the liquid center can be non-aqueous. In some embodiments, the liquid center can be a solution while in other embodiments, the center can be a suspension while in still other embodiments, the center can be an emulsion.

In some embodiments, the viscosity of the liquid center can be manipulated for a variety of reasons including, but not limited to, processing efficiency or creation of a desired perception. In some embodiments, the viscosity of the liquid center can be 3,000 to 10,000 pascal seconds. In some embodiments, the viscosity of the liquid center can be 4,000 to 6,5000 pascal seconds.

In some embodiments, the water activity of the liquid center can be manipulated for a variety of reasons including, but not limited to, microbial stability or maintenance of a desired texture. In some embodiments, the water activity of the liquid center can be 0.1 to 0.7. In some embodiments, the water activity of the liquid center can be 0.25 to 0.35.

Liquids that can be included in the liquid center can include, but are not limited to, fruit juice; vegetable juice; fruit puree; fruit pulp; vegetable pulp; vegetable puree; fruit sauce; vegetable sauce; honey; maple syrup; molasses; corn syrup; sugar syrup; polyol syrup; hydrogenated starch hydrolysates syrup; emulsions; vegetable oil; glycerin; propylene glycol; ethanol; liqueurs; chocolate syrup, dairy-based liquids such as milk, cream, etc.; and combinations thereof.

A gaseous center can be formed by creating a hollow center. The gas can include a mixed composition gas such as air or it can include a single gas such as nitrogen, carbon dioxide, or oxygen. In some embodiments, a gaseous center will include gas trapped in a matrix such as a candy gum, glassy candy matrix or foam. In some embodiments where gas can be trapped in a candy gum, the candy gum can be a confectionery composition including a saccharide component and a chewing gum base. In some embodiments where gas can be trapped in a glassy candy matrix, the glass matrix can be sucrose and the gas can be carbon dioxide. In some embodiments where gas can be introduced into the center in a foam, the foam can include milk proteins and the gas can include a mixed composition gas such as air.

In some embodiments, varying processing parameters can result in confectionery products with different textures.

A texture modifying component can added to the confectionery composition. Inclusion of the texture modifying component can result in finished confectionery products with a variety of texture characteristics ranging from hard and friable to soft and pliable.

The texture modifying component includes a particulate material. Suitable particulate materials are, sucrose, polyols such as sorbitol, xylitol, mannitol, galactitol, lactitol, maltitol, erythritol, isomalt, hydrogenated starch hydrolysates and mixtures thereof, starches, proteins, and combinations thereof. In some embodiments, the particulate material serving as a texture modifying component is selected based on its ability or lack of ability to crystallize the saccharides in the saccharide portion. For example, when isomalt is included in the saccharide portion, sorbitol powder can be added to the confectionery composition because it will not cause the isomalt to crystallize. Alternatively, when erythritol is included in the saccharide portion, erythritol powder can be added to the confectionery composition because it will cause the erythritol to crystallize. Such particulates can be included in amounts from 5% to 35% w/w of the confectionery composition.

In some embodiments, a particulate texture modifying component can also include a flavoring component. For example, in embodiments where sorbitol is used as a texture modifying component, peppermint flavoring can be added to the sorbitol powder.

In some embodiments, a texture modifying component can include fats, oils, or other hydrophobic materials. Suitable fats can include, but are not limited to, partially hydrogenated vegetable or animal fats, such as coconut oil, corn oil, palm kernel oil, peanut oil, soy bean oil, sesame oil, cottonseed oil, cocoa butter, milk fat, beef tallow, and lard, among others. Suitable hydrophobic materials include chocolate, chocolate crumb, carob coatings, and compound coatings. Such fats, oils, and/or hydrophobic materials can be included in amounts of 1% to 10% w/w of the confectionery composition.

In some embodiments, the sensory perception of the texture modifying component is similar to that of fat, oil, or other hydrophobic materials even though the texture modifying component is present in the confectionery composition at a lower level. For example, a confectionery composition including 2.5% hydrogenated cottonseed oil can provide the same mouthfeel perception as a confection including 10% - 50% fat as measured by sensory evaluation techniques.

In some embodiments, the first, second and/or third initial hardness intensity are similar to, greater than or less than a hard boiled candy with a moisture content of less than 2% w/w..

In some embodiments, the first, second and/or third initial hardness intensity are similar to, greater than or less than a nougat having a fat content of 2% to 10% w/w.

In some embodiments, the first, second and/or third initial hardness intensity are similar to, greater than or less than an uncoated chewing gum having a gum base content of 20% to 30% w/w.

In some embodiments, the first, second and/or third initial hardness intensity are similar to, greater than or less than a gummy candy having a hydrocolloid level of 0.5% to 15% w/w.

In some embodiments, the first, second and/or third initial hardness intensity are similar to, greater than or less than a marshmallow having a moisture content of less than 15% w/w.

In some embodiments, a texture modifying component is incorporated into the confectionery composition when the saccharide composition is being mixed with the elastomeric composition.

In some embodiments the texture of the saccharide region can be designed to be similar to the texture of the elastomeric region so as to provide a desired texture when the regions are combined. In these embodiments, the texture of the confectionery composition can be maintained across a wide range of ratios of saccharide region to elastomeric region because the respective textures of the regions are similar. In other embodiments, the texture of the saccharide region can be designed to be different than the texture of the elastomeric region. In these embodiments, changing the ratio may change the texture and the texture of the predominant region will predominate.

When measuring the various textures, an initial hardness intensity can be determined by having trained judges compare the initial hardness of a sample to the initial hardness of a reference material. Initial hardness intensity can be defined as the force required to bite through a product and cause it to shatter or break apart when it is first placed in the mouth. Suitable reference materials for determining relative initial hardness intensities can include, but are not limited to, hard boiled candy; intermediate texture candies such as nougat (Starburst™ fruit chews for example), uncoated chewing gum, gummy candy (such as gummy bears); and soft texture candies such as marshmallow.

The candy gum confection described herein provides a unique, multi-sensorial experience in a chewing gum product with benefits and attributes added in from candy. The distinctiveness of this product is achieved by way of separately identifiable, quality sensory experiences (or phases) that are perceived through changing sensory cues including changes in texture, flavor, appearance and feeling factors as the product morphs from a hard candy product to a quality piece of chewing gum. These sensory cues can be identified by descriptive analysis and can be reliably and reproducibly measured. Descriptive analysis is a sensory method by which the sensory attributes of a food or product are identified and quantified, using human subjects who have been specifically chosen and trained for this purpose. Once trained, a panel of evaluators will function as a human analytical instrument, generating reproducible values, rather than degrees of liking or dislike as in consumer panels. Though sensory professionals may choose any number of standard methodologies, or variations thereof, to accomplish descriptive evaluations, each method has standard parameters associated with it to ensure testing is sound. These evaluation methods are described in Morten Meilgaard, D.Sc. et al., Sensory Evaluation Techniques (3rd ed. 1999), and in American Society for Testing and Materials (Committee E-18) documents as well.

A variation of the Spectrum™ methodology can be used for descriptive studies and is a customized approach to evaluating the unique characteristics of each product. Highly-trained panelists experience the products, noting all of the sensory attributes perceived as well as how those attributes change. Panelists then develop the intensity scales, verbiage and references that provide the structure within which to best describe and differentiate how the products taste, react and feel in the mouth, as well as any feeling factors experienced. The use of well-chosen references and intensity scales provide the framework within which the sensory work can quantify any differences that may exist among products and also provide the basis for reproducibility of test results. While instruments in the analytical lab provide reproducibility as well, they lack the ability to capture the *integrated* sensory experience as perceived by humans, therefore making descriptive data more valuable.

The integrated sensory experience means that tastes (salt, sweet, sour and bitter), aromatics (e.g. peppermint, butterscotch, etc.), feeling factors (trigeminal stimulations such as cooling, heat, etc.), and texture (how a product feels in the mouth and how it changes with chewing and manipulation) can all be perceived by the trained panelist. These perceptions are registered by way of sensors in the mouth, with the information going to the brain for processing. Moment by moment changes in all of the separate sensory attributes are perceived, but in humans, the sensory perception as a whole can be perceived as well. Analytical instrumentation cannot duplicate this ability. The ability to perceive and integrate all of the sensory input is particularly important in a study where lots of changes are taking place at one time, as with the candy gum confections described herein.

In some embodiments, the candy gum confections described herein can have the added benefit of induce excitement, relieve boredom, facilitates concentration, keep consumer entertained and other positive psychotropic measures. Used relative to consumer research, the term psychometric measures describe specific types of questioning designed to elicit how the consumer feels about the product from an emotional perspective as opposed to eliciting answers that require more of a cognitive based approach. "Emotional responses are multidimensional and measurable quantitatively." Frijda et al (1992). Typically, aspects of the following areas/techniques: positive and negative emotions, energy levels, imagery, memories, projection techniques, and personality types can be employed

To illustrate how the composition of each region and the ratio between the regions can be manipulated to provide desired textures, the charts shown in Tables 3 and 4 illustrate some of the ways the region compositions and ratios of the saccharide and elastomeric regions can be varied to achieve desired textures.

For the saccharide region, some variables that determine the texture of the saccharide region can include moisture amount, hydrocolloid amount, and fat amount. For the elastomeric region, some variables that can determine the texture of the elastomeric region can include filler amount and plasticizer amount. Changing these variables can change the texture of the region.

In a candy gum composition, the amount of each region and its texture help determine the texture of the candy gum. For example, when a saccharide composition with an initial hardness intensity similar to hard candy added to a candy gum product in an amount sufficient to determine the texture of the candy gum, in a candy gum product with an initial hardness intensity similar to hard candy will result.

In some embodiments, the gum base or elastomeric portion and the saccharide portion include fats and/or other texture modifying components in amounts higher than or less than the other component. In still further embodiments, fats and/or other texture modifying components also included in the center-fill material, the coating or both, with varying levels when compared to the gum base and/or saccharide components, e.g., more or less than.

Table 3 provides a conceptual map of how some of the texture influencing variables is varied within each region and how the amounts of the regions are manipulated to influence the texture of the candy gum product.

Table 4 builds on the conceptual map of Table 3 by providing a range of values that illustrate the concepts.

**Table 3**

| | **Initial Hardness Intensity of Candy Gum** | | | | | | | | |
|---|---|---|---|---|---|---|---|---|---|
| | **Hard Candy** | | | **Intermediate Texture Candy ( such as nougat, uncoated chewing gum, or gummy candy)** | | | **Soft Candy (such as marshmallow)** | | |
| **Saccharide Region** | | | | | | | | | |
| Moisture | Low | Low | High | Med | Med | Low | High | High | Low |
| Hydrocolloid | None | None | High | Low | Low | None | High | High | None |
| Fat | None | None | High | Low | Low | None | High. | High | None |
| Amount of Saccharide Region in Candy Gum | N/A* | Amount capable of determining finished product texture | Amount not capable of determining finished product texture | N/A* | Amount capable of determining finished product texture | Amount not capable of determining finishes product texture | N/A* | Amount capable of determining finished product texture | Amount not capable of determining finished product texture |
| | | | | | | | | | |

| **Elastomeric Region** | | | | | | | | | |
|---|---|---|---|---|---|---|---|---|---|
| Filler | Low | High | Low | Med | Low | Med | High | Low | High |
| Plasticizer | Low | High | Low | Med | Low | Med | High | Low | High |
| Amount of Elastomeric Region in Candy Gum | N/A* | Amount not capable of determining finished product texture | Amount capable of determining finished product texture | N/A* | Amount not capable of determining finished product texture | Amount capable of determining finished product texture | N/A* | Amount not capable of determining finished product texture | Amount capable of determining finished product texture |

| | | | | | | | | | |
|---|---|---|---|---|---|---|---|---|---|
| *N/A stands for "Not Applicable" because in these scenarios the saccharide region and elastomeric regions have similar textures and thus changing the amount of each region does not determine the texture of the candy gum product. | | | | | | | | | |

**Table 4**

| | **Initial Hardness Intensity of Candy Gum** | | | | | | | | |
|---|---|---|---|---|---|---|---|---|---|
| | **Hard Candy** | | | **Intermediate Texture Candy (such as nougat, uncoated chewing gum, or gummy candy)** | | | **Soft Candy (such as marshmallow)** | | |
| **Saccharide Region** | % w/w | | | % w/w | | | % w/w | | |
| Moisture | 0.2-2.0 | 0.2-2.0 | 1 - 15 | 0.5-5 | 0.5-5 | 0.2-2.0 | 2 - 15 | 2 - 15 | 0.2-2.0 |
| Hydrocolloid | None | None | 0.5-25 | 0.5-15 | 0.5-15 | None | 0.5-25 | 0.5-25 | None |
| Fat | None | None | 2-15 | 2-10 | 2-10 | None | 2-15 | 2 - 15 | None |
| Amount of Saccharide Region in Candy Gum | 5-95 | 40-95 | 5-30 | 5 - 95 | 40-95 | 5-30 | 5 - 95 | 40-95 | 5-30 |
| | | | | | | | | | |

| **Elastomeric Region** | | | | | | | | | |
|---|---|---|---|---|---|---|---|---|---|
| Filler | 0 - 10 | 30-40 | 0 - 10 | 5 - 15 | 0 - 10 | 5 - 15 | 30-40 | 0 - 10 | 30-40 |
| Plasticizer | 15-50 | 25-65 | 15 - 50 | 20-60 | 15 - 50 | 20-60 | 25-65 | 15-50 | 25-65 |
| Amount of Elastomeric Region in Candy Gum | 5-95 | 5-25 | 30 - 60 | 5-95 | 5-25 | 30 - 60 | 5-95 | 5-25 | 30-60 |

The confectionery composition can be provided with a desired level of shine or gloss. Appearance aspects of shine and gloss can be measured by a variety of methods such as optometric methods including, but not limited to, reflectance meters, spectophotometers, and consumer testing. The confectionery composition can be configured to include portions that have been adjusted to be visually different, e.g. based on color, texture etc.

In some embodiment, a candy gum with high gloss is made by high pressure extrusion. In a preferred embodiment, a candy gum is made by high pressure extrusion at high die pressure. In another preferred embodiment, a candy gum is made by high pressure extrusion at the temperature close to a glass transition point of a saccharide component.

### Additional Components

Additional additives, such as physiological cooling agents, throat-soothing agents, spices, warming agents, tooth-whitening agents, breath-freshening agents, vitamins, nutraceuticals, phytochemicals, polyphenols, antioxidants, minerals, caffeine, drugs and other actives may also be included in any or all portions of the confectionery composition. Such components may be used in amounts sufficient to achieve their intended effects.

Any of the additional components discussed herein may be added to any portion of the confectionery composition in their modified release form and/or without modified release (sometimes referred to as "free" components). In some embodiments, for example, a single component may be added to the confectionery composition in its modified release form and free form. The modified release component and free component may be included together in the same portion of the confectionery composition or, in some embodiments, the two components may be included in different portions of the confectionery composition.

In some other embodiments, for example, two different components that provide the same functionality, e.g., two different flavors, sweeteners, tastes, sensations, or the like, may be included in a confectionery composition. In some embodiments, both components may have modified release properties. Alternatively, in some embodiments, one of the components may be modified release, whereas the other component may be free. The two components may be included in the same or different portions of the confectionery composition.

Types of individual ingredients for which optional managed release from a confectionery composition may be desired, include, but are not limited to sweeteners, flavors, actives, effervescing ingredients, appetite suppressors, breath fresheners, dental care ingredients, emulsifiers, flavor potentiators, bitterness masking or blocking ingredients, food acids, micronutrients, sensates, mouth moistening ingredients, throat care ingredients, colors, and combinations thereof. Ingredients may be available in different forms such as, for example, liquid form, spray-dried form, or crystalline form. In some embodiments, a delivery system or confectionery composition may include the same type of ingredient in different forms. For example, a confectionery composition may include a liquid flavor and a spray-dried version of the same flavor. In some embodiments, the ingredient may be in its free or encapsulated form and may be present in any portion of the confectionery composition such as in the saccharide portion, the elastomeric portion, or the coating or center-fill.

In some embodiments, an ingredient's release is modified such that when a consumer chews the confectionery composition, they may experience an increase in the duration of flavor or sweetness perception and/or the ingredient is released or otherwise made available over a longer period of time. Modified release may be accomplished by any method known in the art, such as by encapsulation. Where modified release is due to encapsulation, this may be accomplished by a variety of means such as by spray coating or extrusion.

Additionally, if early and extended release of the ingredient is desired, the confectionery composition may include ingredients without modified release (sometimes referred to as "free" ingredients), as well as ingredients with modified release. In some embodiments, a free ingredient may be used to deliver an initial amount or "hit" of an ingredient (e.g., flavor, cooling agent) or an initial sensation or benefit caused by the ingredient (e.g., flavor, nasal action, cooling, warming, tingling, saliva generation, breath freshening, teeth whitening, throat soothing, mouth moistening, etc.). In some embodiments, the same ingredient can be provided with modified release characteristics to provide an additional or delayed amount of the same sensation or benefit. By using both the free ingredient and the ingredient with modified release characteristics, the sensation or benefit due to the ingredient may be provided over a longer period of time and/or perception of the sensation or benefit by a consumer may be improved. Also, in some embodiments the initial amount or "hit" of the ingredient may predispose or precondition the consumers' mouth or perception of the confectionery composition.

In some embodiments, modified release can also be affected by where (what portion of the confectionery composition) the ingredient is included. For example, an ingredient that has an affinity for elastomeric materials, can be included in the saccharide portion where it does not have an affinity and thus it will be released faster and more completely. Similarly, in some embodiments, it may be desirable to release an ingredient over time or less completely. In that case, including the ingredient with an affinity for elastomeric materials in the elastomeric portion will provide the desired release.

As another example, in some embodiments it may be desirable to provide a sustained release of an ingredient in a confectionery composition over time. To accomplish sustained release, the ingredient may be modified to allow for a lower concentration of the ingredient to be released over a longer period of time versus the release of a higher concentration of the ingredient over a shorter period of time. A sustained release of an ingredient may be advantageous in situations when the ingredient has a bitter or other bad taste at the higher concentrations. A sustained release of an ingredient also may be advantageous when release of the ingredient in higher concentrations over a shorter period of time may result in a lesser amount of the ingredient being optimally delivered to the consumer. For example, for a tooth whitening or breath freshening ingredient, providing too much of the ingredient too fast may result in a consumer swallowing a significant portion of the ingredient before the ingredient has had a chance to interact with the consumer's teeth, mucous membranes, and/or dental work, thereby wasting the ingredient or at least reducing the benefit of having the ingredient in the confectionery composition.

In some embodiments described herein, the elastomeric portion of the confectionery composition may include at least one modified release component. At least one modified release component optionally may be added to the saccharide portion, the center-fill and/or coating, as well. The additional modified release component that may be included in the saccharide portion, center-fill and/or coating may be the same as or different from the modified release component contained in the elastomeric portion.

### Ingredient Release Management

In different embodiments, different techniques, ingredients, and/or delivery systems, may be used to manage release of one or more ingredients in a confectionery composition. In some embodiments, more than one of the techniques, ingredients, and/or delivery systems may be used.

In some embodiments, the delay in availability or other release of an ingredient in a confectionery composition caused by encapsulation of the ingredient may be based, in whole or in part, by one or more of the following: the type of encapsulating material, the molecular weight of the encapsulating material, the tensile strength of the delivery system containing the ingredient, the hydrophobicity of the encapsulating material, the presence of other materials in the saccharide portion or elastomeric portion (e.g., tensile strength modifying agents, emulsifiers), presence and/or composition of the texture modifying component, the ratio of the amounts of one or more ingredients in the delivery system to the amount of the encapsulating material in the delivery system, the number of layers of encapsulating material, the desired texture, flavor, shelf life, or other characteristic of a confectionery composition, the ratio of the encapsulating material to the ingredient being encapsulated, etc. Thus, by changing or managing one or more of these characteristics of a delivery system or the confectionery composition, release of one or more ingredients in a confectionery composition during consumption of the confectionery composition can be managed more effectively and/or a more desirable release profile for one or more ingredients in the delivery system or the confectionery composition may be obtained. This may lead to a more positive sensory or consumer experience during consumption of the confectionery composition, more effective release of such one or more ingredients during consumption of the confectionery composition, less need for the ingredient (e.g., more effective release of the ingredient may allow the amount of the ingredient in the confectionery composition to be reduced), increased delivery of a therapeutic or other functional benefit to the consumer, etc. Additionally, in some embodiments, managing the release rate or profile can be tailored to specific consumer segments.

### Encapsulation

In some embodiments, one or more ingredients may be encapsulated with an encapsulating material to modify the release profile of the ingredient. In general, partially or completely encapsulating an ingredient used in a confectionery composition with an encapsulating material may delay release of the ingredient during consumption of the confectionery composition, thereby delaying when the ingredient becomes available inside the consumer's mouth, throat, and/or stomach, available to react or mix with another ingredient, and/or available to provide some sensory experience and/or functional or therapeutic benefit. This can be particularly true when the ingredient is water soluble or at least partially water soluble.

In some embodiments, encapsulation may be employed to provide barrier protection to or from a component rather than to modify the release of the component. For example, it often is desirable to limit the exposure of acids to other components in a confectionery composition. Such acids may be encapsulated to limit their exposure to other components, or alternatively, the other components in the confectionery composition may be encapsulated to limit their exposure to the acid.

In some embodiments, a material used to encapsulate an ingredient may include water insoluble polymers, co-polymers, or other materials capable of forming a strong matrix, solid coating, or film as a protective barrier with or for the ingredient. In some embodiments, the encapsulating material may completely surround, coat, cover, or enclose an ingredient. In other embodiments, the encapsulating material may only partially surround, coat, cover, or enclose an ingredient. Different encapsulating materials may provide different release rates or release profiles for the encapsulated ingredient. In some embodiments, encapsulating material used in a delivery system may include one or more of the following: polyvinyl acetate, polyethylene, crosslinked polyvinyl pyrrolidone, polymethylmethacrylate, polylactidacid, polyhydroxyalkanoates, ethylcellulose, polyvinyl acetatephthalate, polyethylene glycol esters, methacrylicacid-co-methylmethacrylate, ethylene-vinylacetate (EVA) copolymer, and the like, and combinations thereof.

In some embodiments, an ingredient may be pre-treated prior to encapsulation with an encapsulating material. For example, an ingredient may be coated with a "coating material" that is not miscible with the ingredient or is at least less miscible with the ingredient relative to the ingredient's miscibility with the encapsulating material.

In some embodiments, an ingredient may be encapsulated with multiple encapsulating materials. For example, an ingredient may be coated with an encapsulating ingredient that contains polyvinyl acetate and may then be coated with an encapsulating ingredient that contains wax. In some embodiments, such multiple encapsulation systems can provide thermal stability protection for ingredients that would be adversely affected by the heat used in confectionery making processes.

In some embodiments, an encapsulation material may be used to individually encapsulate different ingredients in the same confectionery composition. For example, a delivery system may include aspartame encapsulated by polyvinyl acetate. Another delivery system may include acesulfame-K encapsulated by polyvinyl acetate. Both delivery systems may be used as ingredients in the same confectionery composition or in other confectionery compositions. For additional examples, see U.S. Patent Application Serial No. 60/683,634 entitled "Methods and Delivery Systems for Managing Release of One or More Ingredients in an Edible Composition" and filed May 23, 2005.

In some embodiments, different encapsulation materials may be used to individually encapsulate different ingredients used in the same confectionery composition. For example, a delivery system may include aspartame encapsulated by polyvinyl acetate. Another delivery system may include acesulfame-K encapsulated by EVA. Both delivery systems may be used as ingredients in the same confectionery composition or other confectionery compositions. Examples of encapsulated ingredients using different encapsulating materials can be found in U.S. Patent Application Serial No. 60/655,894 filed February 25, 2005, and entitled "Process for Manufacturing a Delivery System for Active Components as Part of an Edible Composition.".

### Methods of Encapsulation

There are many ways to encapsulate one or more ingredients with an encapsulating material. For example, in some embodiments, a sigma blade or Banbury™ type mixer may be used. In other embodiments, an extruder or other type of continuous mixer may be used. In some embodiments, spray coating, spray chilling, absorption, adsorption, inclusion complexing (e.g., creating a flavor/cyclodextrin complex), coacervation, fluidized bed coating, or other process may be used to encapsulate an ingredient with an encapsulating material.

In some embodiment, the temperature of mixing is 60 °C -120 °C. In a preferred embodiment, the temperature of mixing is 60 °C-80 °C. In some embodiment, the mixing time is 10 second to 30 minutes.

Examples of encapsulation of ingredients can be found in U.S. Patent Application Serial Number 60/655,894, filed February 25, 2005, and entitled "Process for Manufacturing a Delivery System for Active Components as Part of an Edible Composition." Other examples of encapsulation of ingredients can be found in U.S. Patent Application Serial Number 10/955,255 filed September 30, 2004, and entitled "Encapsulated Compositions and Methods of Preparation," the entire contents of which are incorporated herein by reference for all purposes. Further examples of encapsulation of ingredients can be found in U.S. Patent Application Serial Number 10/955,149 filed September 30, 2004, and entitled "Thermally Stable High Tensile Strength Encapsulation Compositions for Actives," the entire contents of which are incorporated herein by reference for all purposes. Still further examples of encapsulation of ingredients can be found in U.S. Patent Application Serial Number 11/052,672 filed February 7, 2005, and entitled "Stable Tooth Whitening Confectionery with Reactive Components," the entire contents of which are incorporated herein by reference for all purposes. Further encapsulation techniques and resulting delivery systems may be found in U.S. Patent Nos. 6,770,308, 6,759,066, 6,692,778, 6,592,912, 6,586,023, 6,555,145, 6,479,071, 6,472,000, 6,444,241, 6,365,209, 6,174,514, 5,693,334, 4,711,784, 4,816,265, and 4,384,004, the contents of all of which are incorporated herein by reference for all purposes.

In some embodiments, a delivery system may be ground to a powdered material with a particular size for use as an ingredient in a confectionery composition. For example, in some embodiments, an ingredient may be ground to approximately the same particle size of the other confectionery composition ingredients so as to create a homogeneous mixture. In some embodiments, the delivery system may be ground to a powdered material with an average particle size such as, for example, about 4 to about 100 mesh or about 8 to about 25 mesh or about 12 to about 20 mesh.

### Tensile Strength

In some embodiments, selection of an encapsulating material for one or more ingredients may be based on tensile strength desired for the resulting delivery system For example, in some embodiments, a delivery system produces delayed or otherwise controlled release of an ingredient through the use of a pre-selected or otherwise desired tensile strength.

In some embodiments, increasing the tensile strength of a delivery system may increase the delayed or extended release of an ingredient in the delivery system. The tensile strength for a delivery system may be matched with a desirable release rate selected according to the type of the ingredient(s) to be encapsulated for the delivery system, the encapsulating material used, any other additives incorporated in the delivery system and/or a confectionery composition using the delivery system as an ingredient, the desired rate of release of the ingredient, and the like. In some embodiments, the tensile strength of a delivery system which can be at least 6,500 psi, including 7500, 10,000, 20,000, 30,000, 40,000, 50,000, 60,000, 70,000, 80,000, 90,000, 100,000, 125,000, 135,000, 150,000, 165,000, 175,000, 180,000, 195,000, 200,000 and all ranges and subranges there between, for example, a tensile strength range of 6,500 to 200,000 psi.

In some embodiments, a delivery system for one or more ingredients can be provided based on the tensile strength of the delivery system having a specific tensile strength when compared to a standard. Thus, the design of the delivery system is not focused on one characteristic (e.g., molecular weight) of one of the materials (e.g., encapsulating material) used to produce the delivery system. In this manner, a delivery system can be formulated to express a desired release profile by adjusting and modifying the tensile strength through the specific selection of the ingredient(s), encapsulating material, additives, amount of the ingredient(s), amount of encapsulating material, relative amounts of ingredient(s) to encapsulating material, etc. If a desired tensile strength is chosen for a delivery system, any delivery system that has the desired tensile strength may be used without being limited to a particular encapsulating material and its molecular weight. The formulation process can be extended to encapsulating materials that exhibit similar physical and chemical properties as the encapsulating material forming part of the standard delivery system.

In some embodiments, a delivery system for delivering an ingredient may be formulated to ensure an effective sustained release of the ingredient based on the type and amount of the ingredient and the desired release rate for the ingredient. For example, it may be desirable to affect the controlled release of a high intensity sweetener from a confectionery composition over a period of twenty-five to thirty minutes to ensure against a rapid burst of sweetness that may be offensive to some consumers. A shorter controlled release time may be desirable for other type of ingredients such as pharmaceuticals or therapeutic agents, which may be incorporated into the same confectionery composition by using separate delivery systems for each of these ingredients. Delivery systems may be formulated with a particular tensile strength associated with a range of release rates based on a standard. The standard may comprise a series of known delivery systems having tensile strengths over a range extending, for example, from low to high tensile strength values. Each of the delivery systems of the standard will be associated with a particular release rate or ranges of release rates. Thus, for example, a delivery system can be formulated with a relatively slow release rate by a fabricating a delivering system having a relatively high tensile strength. Conversely, lower tensile strength compositions tend to exhibit relatively faster release rates.

In some embodiments, encapsulating material in a delivery system may be present in amounts of from about 0.2% to 10% by weight based on the total weight of the chewing confectionery composition, including 0.3, 0.5, 0.7, 0.9, 1.0, 1.25, 1.4, 1.7, 1.9, 2.2, 2.45, 2.75, 3.0, 3.5, 4.0, 4.25, 4.8, 5.0, 5.5, 6.0, 6.5, 7.0, 7.25, 7.75, 8.0, 8.3, 8.7, 9.0, 9.25, 9.5, 9.8 and all values and ranges there between, for example, from 1% to 5% by weight. The amount of the encapsulating material can depend in part on the amount of the ingredient(s) component that is encapsulated. The amount of the encapsulating material with respect to the weight of the delivery system, is from about 30% to 99%, including 35, 40, 45, 50, 55, 60, 65, 70, 75, 80, 85, 95, 97 and all values and ranges there between, for example, from about 60% to 90% by weight.

In some embodiments, the tensile strength of a delivery system may be selected from relatively high tensile strengths when a relatively slow rate of release for an ingredient in the delivery system is desired and relatively lower tensile strengths when a faster rate of release for an ingredient in the delivery system is desired. Thus, when employing a tensile strength of 50,000 psi for a delivery system, the release rate of the ingredient, will generally be lower than the release rate of the ingredient in a delivery system having a tensile strength of 10,000 psi regardless of the type of encapsulating material (e.g., polyvinyl acetate) chosen.

In some embodiments, the encapsulating material for a delivery system is polyvinyl acetate. A representative example of a polyvinyl acetate product suitable for use as an encapsulating material in the present invention is Vinnapas® B100 sold by Wacker Polymer Systems of Adrian, Michigan. A delivery system utilizing polyvinyl acetate may be prepared by melting a sufficient amount of polyvinyl acetate at a temperature of about 65°C to 120°C for a short period of time, e.g., five minutes. The melt temperature will depend on the type and tensile strength of the polyvinyl acetate encapsulating material where higher tensile strength materials will generally melt at higher temperatures. Once the encapsulating material is melted, a suitable amount of an ingredient (e.g., high intensity sweetener such as aspartame) is added and blended into the molten mass thoroughly for an additional short period of mixing. The resulting mixture is a semi-solid mass, which is then cooled (e.g., at 0°C) to obtain a solid, and then ground to a U.S. Standard sieve size of from about 30 to 200 (600 to 75 microns). The tensile strength of the resulting delivery system can readily be tested according to ASTM-D638.

For additional information regarding how tensile strength of a delivery system may be used to create managed release of one or more ingredients, see U.S. Patent Application Serial No. 11/083,968 entitled "A Delivery System for Active Components as Part of an Edible Composition Having Preselected Tensile Strength" and filed on March 21, 2005, and U.S. Patent Application Serial No. 10/719,298 entitled "A Delivery System for Active Components as Part of an Edible Composition" and filed November 21, 2003, the complete contents of both of which are incorporated herein by reference for all purposes.

### Hydrophobicity

In some embodiments, the release of one or more ingredients from a delivery system may depend on more than tensile strength. For example, the release of the ingredients may be directly related to the tensile strength of the delivery system and the hydrophobicity (i.e., water resistance) of the encapsulating polymer or other material.

As a more specific example, when a delivery system is used in a confectionery composition, moisture may be absorbed in the encapsulated ingredient(s) during mastication and chewing of the confectionery composition. This may result in softening of the encapsulating material and releasing of the ingredient(s) during the mastication and chewing of the confectionery composition. The softening of the encapsulation material depends on the hydrophobicity of the polymer used as the encapsulation material. In general, the higher the hydrophobicity of the polymer, the longer mastication time is needed for softening the polymer.

As one example, higher hydrophobic polymers such as ethylene-vinylacetate (EVA) copolymer can be used to increase or otherwise manage ingredient (e.g., sweetener) release times from encapsulations. The degree of hydrophobicity can be controlled by adjusting the ratio of ethylene and vinylacetate in the copolymer. In general, the higher the ethylene to vinylacetate ratio, the longer time it will take during consumption to soften the encapsulation particles, and the slower or more delayed will be the release rate of the ingredient. The lower the ethylene to vinylacetate ratio, the shorter time it will take during consumption to soften the encapsulation particles, and the faster or earlier will be the release rate of the ingredient.

As illustrated by the discussion above, in some embodiments, release of an ingredient from a delivery system can be managed or otherwise controlled by formulating the delivery system based on the hydrophobicity of the encapsulating material, e.g., the polymer, for the ingredient. Using highly hydrophobic polymers, the release times of the ingredient can be increased or delayed. In a similar manner, using encapsulating material that is less hydrophobic, the ingredient can be released more rapidly or earlier.

The hydrophobicity of a polymer can be quantified by the relative water-absorption measured according to ASTM D570-98. Thus, by selecting encapsulating material(s) for a delivery system with relatively lower water-absorption properties and adding that to a mixer, the release of the ingredient contained in the produced delivery system can be delayed compared to those encapsulating materials having higher water-absorption properties.

In some embodiments, polymers with water absorption of from about 50 to 100% (as measured according to ASTM D570-98) can be used. Moreover, to decrease the relative delivery rate, the encapsulating material can be selected such that the water absorption would be from about 15% to about 50% (as measured according to ASTM D570-98). Still further, in other embodiments, the water absorption properties of the encapsulating material can be selected to be from 0.0% to about 5% or up to about 15% (as measured according to ASTM D570-98). In other embodiments, mixtures of two or more delivery systems formulated with encapsulating material having different water-absorption properties can also be used in subsequent incorporation into a confectionery composition.

Polymers with suitable hydrophobicity which may be used for delivery systems include homo- and co-polymers of, for example, vinyl acetate, vinyl alcohol, ethylene, acrylic acid, methacrylate, methacrylic acid and others. Suitable hydrophobic copolymers include the following non-limiting examples, vinyl acetate/vinyl alcohol copolymer, ethylene/vinyl alcohol copolymer, ethylene/acrylic acid copolymer, ethylene/methacrylate copolymer, ethylene/methacrylic acid copolymer.

In some examples, the hydrophobic encapsulating material in a delivery system may be present in amounts of from about 0.2% to 10% by weight based on the total weight of a confectionery composition containing the delivery system, including 0.3, 0.5, 0.7, 0.9, 1.0, 1.25, 1.4, 1.7, 1.9, 2.2, 2.45, 2.75, 3.0, 3.5, 4.0, 4.25, 4.8, 5.0, 5.5, 6.0, 6.5, 7.0, 7.25, 7.75, 8.0, 8.3, 8.7, 9.0, 9.25, 9.5, 9.8 and all values and ranges there between, for example, from1% to 5% by weight. The amount of the encapsulating material will, of course, depend in part on the amount of the ingredient that is encapsulated. The amount of the encapsulating material with respect to the weight of the delivery system, is from about 30% to 99%, including 35, 40, 45, 50, 55, 60, 65, 70, 75, 80, 85, 95, 97 and all values and ranges there between, for example, from about 60% to 90% by weight.

In formulating the delivery system based on the selection criteria of hydrophobicity of the encapsulating material, the encapsulated ingredient can be entirely encapsulated within the encapsulating material or incompletely encapsulated within the encapsulating material provided the resulting delivery system meets the criteria set forth hereinabove. The incomplete encapsulation can be accomplished by modifying and/or adjusting the manufacturing process to create partial coverage of the ingredient.

For example, if ethylene-vinyl acetate is the encapsulating material for an ingredient, the degree of hydrophobicity can be controlled by adjusting the ratio of ethylene and vinyl acetate in the copolymer. The higher the ethylene to vinylacetate ratio, the slower the release of the ingredient. Using vinylacetatelethylene copolymer as an example, the ratio of the vinylacetatelethylene in the copolymer can be from about 1 to about 60%, including ratios of 2.5, 5, 7.5, 9, 12, 18, 23, 25, 28, 30, 35, 42, 47, 52, 55, 58.5 % and all values and ranges there between.

In some embodiments, a method of selecting a target delivery system containing an ingredient for a confectionery composition is based on the hydrophobicity of the encapsulating material for the ingredient in the delivery system. The method generally includes preparing a targeted delivery system containing an ingredient to be encapsulated, an encapsulating material and optional additives, with the encapsulating material having a pre-selected or otherwise desired hydrophobicity. The hydrophobicity of the encapsulating material employed in the targeted delivery system can be selected to provide a desirable release rate of the ingredient. This selection of the encapsulating material is based on the hydrophobicity of sample delivery systems having the same or similar ingredient and known release rates of the ingredient. In another embodiment of the invention, the method comprises (a) obtaining a plurality of sample delivery systems comprising at least one ingredient, at least one encapsulating material, and optional additives, wherein each of the delivery systems is prepared with different encapsulating materials having different hydrophobicities; (b) testing the sample delivery systems to determine the respective release rates of the ingredient(s); and (c) formulating a target delivery system containing the same ingredient(s) with a hydrophobic encapsulating material corresponding to a desired release rate of the ingredient(s) based on the obtained sample delivery systems.

The method of selecting at least one delivery system suitable for incorporation into a confectionery composition preferably can begin by determining a desired release rate for an ingredient (i.e., a first active component). The determination of the desired release rate may be from known literature or technical references or by *in vitro* or *in vivo* testing. Once the desired release rate is determined, the desired hydrophobicity of the encapsulating material can be determined (i.e., a first hydrophobic encapsulating material) for a delivery system (i.e., first delivery system) that can release the first active component at the desired release. Once the delivery system is obtained which can deliver the first active component as required it is then selected for eventual inclusion in a confectionery composition.

The method described above may then be repeated for a second active component and for additional active components as described via the determination and selection of a suitable delivery system.

For additional information regarding the relationship of hydrophobicity of an encapsulating material to the release of an ingredient from a delivery system, see U.S. Patent Application Serial No. 60/683,634 entitled "Methods and Delivery Systems for Managing Release of One or More Ingredients in an Edible Composition" and filed on May 23, 2005, with the U.S. Patent and Trademark Office, the complete contents of which are incorporated herein by reference for all purposes.

### Ratio of Ingredient to Encapsulating Material for Ingredient in Delivery System

In general, the "loading" of an ingredient in a delivery system can impact the release profile of the ingredient when the ingredient is used in a confectionery composition. Loading refers to the amount of one or more ingredients contained in the delivery relative to the amount of encapsulating material. More specifically, the ratio of the amount of one or more ingredients in a delivery system to the amount of encapsulating material in the delivery system can impact the release rate of the one or more ingredients. For example, the lower the ratio or loading of the amount of one or more ingredients in a delivery system to the amount of encapsulating material in the delivery system, the longer or more delayed will be the release of the one or more ingredients from the delivery system. The higher the ratio or loading of the amount of one or more ingredients in a delivery system to the amount of encapsulating material in the delivery system, the faster or earlier will be the release of the one or more ingredients from the delivery system. This principle can be further employed to manage the release profiles of the one or more ingredients by using higher loading of ingredients designed to be released early in combination with lower loading of ingredients designed to be released later. In some embodiments, the one or more ingredients can be the same or different.

For additional information regarding the relationship of the ratio of the amount ingredient in a delivery system to the amount of encapsulating material in the delivery system to the release of an ingredient from a delivery system, see U.S. Patent Application Serial No. 11/134,371 entitled "A Delivery System For Active Components as Part of and Edible Composition Including a Ratio of Encapsulating Material and Active Component" and filed on May 23, 2005, with the U.S. Patent and Trademark Office, the complete contents of which are incorporated herein by reference for all purposes.

There are many types of ingredients for which managed release of the ingredients from a confectionery composition may be desired. In addition, there are many groups of two or more ingredients for which managed release of the group of ingredients from a confectionery composition may be desired.

### Flavorants

In some embodiments, flavorants may include those flavors known to the skilled artisan, such as natural and artificial flavors. These flavorings may be chosen from synthetic flavor oils and flavoring aromatics and/or oils, oleoresins and extracts derived from plants, leaves, flowers, fruits, and so forth, and combinations thereof. Nonlimiting representative flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, yazu, sudachi, and fruit essences including apple, pear, peach, grape, blueberry, strawberry, raspberry, cherry, plum, pineapple, apricot, banana, melon, apricot, ume, cherry, raspberry, blackberry, tropical fruit, mango, mangosteen, pomegranate, papaya and so forth. Other potential flavors whose release profiles can be managed include a milk flavor, a butter flavor, a cheese flavor, a cream flavor, and a yogurt flavor, a vanilla flavor; tea or coffee flavors, such as a green tea flavor, a oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a camomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, and a wasabi (Japanese horseradish) flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture. Flavors may also provide breath freshening properties, particularly the mint flavors when used in combination with the cooling agents, described herein below. In some embodiments, flavorants may chose from geraniol, linalool, nerol, nerolidal, citronellol, heliotropine, methyl cyclopentelone, ethyl vanillin, maltol, ethyl maltol, furaneol, alliaceous compounds, rose type compounds such as phenethanol, phenylacetic acid, nerol, linalyl esters, jasmine, sandlewood, patchouli, and/or cedarwood.

In some embodiments, other flavorings include aldehydes and esters such as cinnamyl acetate, cinnamaldehyde, citral diethylacetal, dihydrocarvyl acetate, eugenyl formate, p-methylamisol, and so forth may be used. Generally any flavoring or food additive such as those described in Chemicals Used in Food Processing, publication 1274, pages 63-258, by the National Academy of Sciences, may be used. This publication is incorporated herein by reference. These may include natural as well as synthetic flavors.

Further examples of aldehyde flavorings include but are not limited to acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6-dimethyl-5-heptenal, .e., melonal (melon), 2,6-dimethyloctanal (green fruit), and 2-dodecenal (citrus, mandarin), cherry, grape, blueberry, blackberry, strawberry shortcake, and mixtures thereof.

In some embodiments, flavoring agents are used at levels that provide a perceptible sensory experience i.e. at or above their threshold levels. In other embodiments, flavoring agents are used at levels below their threshold levels such that they do not provide an independent perceptible sensory experience. At subthreshold levels, the flavoring agents may provide an ancillary benefit such as flavor enhancement or potentiation.

In some embodiments, a flavoring agent may be employed in either liquid form and/or dried form. When employed in the latter form, suitable drying means such as spray drying the liquid may be used. Alternatively, the flavoring agent may be absorbed onto water soluble materials, such as cellulose, starch, sugar, maltodextrin, gum arabic and so forth or may be encapsulated. In still other embodiments, the flavoring agent may be adsorbed onto silicas, zeolites, and the like.

In some embodiments, the flavoring agents may be used in many distinct physical forms. Without being limited thereto, such physical forms include free forms, such as spray dried, powdered, beaded forms, encapsulated forms, and mixtures thereof.

Illustrations of the encapsulation of flavors as well as other additional components can be found in the examples provided herein. Typically, encapsulation of a component will result in a delay in the release of the predominant amount of the component during consumption of a confectionery composition that includes the encapsulated component (e.g., as part of a delivery system added as an ingredient to the chewing confectionery composition). In some embodiments, the release profile of the ingredient (e.g., the flavor, sweetener, etc.) can be managed by managing various characteristics of the ingredient, delivery system containing the ingredient, and/or the confectionery composition containing the delivery system and/or how the delivery system is made. For example, characteristics might include one or more of the following: tensile strength of the delivery system, water solubility of the ingredient, water solubility of the encapsulating material, water solubility of the delivery system, ratio of ingredient to encapsulating material in the delivery system, average or maximum particle size of ingredient, average or maximum particle size of ground delivery system, the amount of the ingredient or the delivery system in the confectionery composition, ratio of different polymers used to encapsulate one or more ingredients, hydrophobicity of one or more polymers used to encapsulate one or more ingredients, hydrophobicity of the delivery system, the type or amount of coating on the delivery system, the type or amount of coating on an ingredient prior to the ingredient being encapsulated, etc.

### Sweetening Ingredients

The sweeteners involved may be selected from a wide range of materials including water-soluble sweeteners, water-soluble artificial sweeteners, water-soluble sweeteners derived from naturally occurring water-soluble sweeteners, dipeptide based sweeteners, and protein based sweeteners, including mixtures thereof. Without being limited to particular sweeteners, representative categories and examples include:
(a) water-soluble sweetening agents such as dihydrochalcones, monellin, steviosides, lo han quo, lo han quo derivatives, glycyrrhizin, dihydroflavenol, and sugar alcohols such as sorbitol, mannitol, maltitol, xylitol, erythritol, and L-aminodicarboxylic acid aminoalkenoic acid ester amides, such as those disclosed in U.S. Pat. No. 4,619,834, which disclosure is incorporated herein by reference, and mixtures thereof;
(b) water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and mixtures thereof;
(c) dipeptide based sweeteners, such as L-aspartic acid derived sweeteners, such as L-aspartyl-L-phenylalanine methyl ester (Aspartame), N-[N-(3,3-dimethylbutyl)-L-α-aspartyl]-L-phenylalanine 1-methyl ester (Neotame), and materials described in U.S. Pat. No. 3,492,131, L-alphaaspartyl-N-(2,2,4,4-tetramethyl-3-thietanyl)-D-alaninamide hydrate (Alitame), methyl esters of L-aspartyl-L-phenylglycerine and L-aspartyl-L-2,5-dihydrophenyl-glycine, L-aspartyl-2,5-dihydro-L-phenylalanine; L-aspartyl-L-(1-cyclohexen)-alanine, and mixtures thereof;
(d) water-soluble sweeteners derived from naturally occurring water-soluble sweeteners, such as chlorinated derivatives of ordinary sugar (sucrose), e.g., chlorodeoxysugar derivatives such as derivatives of chlorodeoxysucrose or chlorodeoxygalactosucrose, known, for example, under the product designation of Sucralose; examples of chlorodeoxysucrose and chlorodeoxygalactosucrose derivatives include but are not limited to: 1-chloro-1'-deoxysucrose; 4-chloro-4-deoxy-alpha-D-galactopyranosyl-alpha-D-fructofuranoside, or 4-chloro-4-deoxygalactosucrose; 4-chloro-4-deoxy-alpha-D-galactopyranosyl-1-chloro-1-deoxy-beta-D-fructo-f uranoside, or 4,1'-dichloro-4,1'-dideoxygalactosucrose; 1',6'-dichlorol',6'-dideoxysucrose; 4-chloro-4-deoxy-alpha-D-galactopyranosyl-1,6-dichloro-1,6-dideoxy-beta-D- fructofuranoside, or 4,1',6'-trichloro-4,1',6'-trideoxygalactosucrose; 4,6-dichloro-4,6-dideoxy-alpha-D-galactopyranosyl-6-chloro-6-deoxy-beta-D- fructofuranoside, or 4,6,6'-trichtoro-4,6,6'-trideoxygalactosucrose; 6,1',6'-trichloro-6,1',6'-trideoxysucrose; 4,6-dichloro-4,6-dideoxy-alpha-D-galacto-pyranosyl-1,6-dichloro-1,6-dideox y-beta-D-fructofuranoside, or 4,6,1',6'-tetrachloro4,6,1,6'-tetradeoxygalacto-sucrose; and 4,6,1',6'-tetradeoxy-sucrose, and mixtures thereof;
(e) protein based sweeteners such as thaumaoccous danielli (Thaumatin I and II) and talin; and
(f) the sweetener monatin (2-hydroxy-2-(indol-3-ylmethyl)-4-aminoglutaric acid) and its derivatives.

The intense sweetening agents may be used in many distinct physical forms well-known in the art to provide an initial burst of sweetness and/or a prolonged sensation of sweetness. Without being limited thereto, such physical forms include free forms, spray dried forms, powdered forms, beaded forms, encapsulated forms, and mixtures thereof. In one embodiment, the sweetener is a high intensity sweetener such as aspartame, sucralose, and acesulfame potassium (e.g., Ace-K or acesulfame-K).

In some embodiments, the sweetener may be a polyol. Polyols can include, but are not limited to glycerol, sorbitol, maltitol, maltitol syrup, mannitol, isomalt, erythritol, xylitol, hydrogenated starch hydrolysates, polyglycitol syrups, polyglycitol powders, lactitol, and combinations thereof.

The active component (e.g., sweetener), which is part of the delivery system, may be used in amounts necessary to impart the desired effect associated with use of the active component (e.g., sweetness). In general, an effective amount of intense sweetener may be utilized to provide the level of sweetness desired, and this amount may vary with the sweetener selected. The intense sweetener may be present in amounts from about 0.001% to about 3%, by weight of the composition, depending upon the sweetener or combination of sweeteners used. The exact range of amounts for each type of sweetener may be selected by those skilled in the art.

### Sensate Ingredients

Sensate compounds can include cooling agents, warming agents, tingling agents, effervescent agents, and combinations thereof. A variety of well known cooling agents may be employed. For example, among the useful cooling agents are included xylitol, erythritol, dextrose, sorbitol, menthane, menthone, ketals, menthone ketals, menthone glycerol ketals, substituted p-menthanes, acyclic carboxamides, mono menthyl glutarate, substituted cyclohexanamides, substituted cyclohexane carboxamides, substituted ureas and sulfonamides, substituted menthanols, hydroxymethyl and hydroxymethyl derivatives of p-menthane, 2-mercapto-cyclo-decanone, hydroxycarboxylic acids with 2-6 carbon atoms, cyclohexanamides, menthyl acetate, menthyl salicylate, N,2,3-trimethyl-2-isopropyl butanamide (WS-23), N-ethyl-p-menthane-3-carboxamide (WS-3), isopulegol, 3-(1-menthoxy)propane-1,2-diol, 3-(1-menthoxy)-2-methylpropane-1,2-diol, p-menthane-2,3-diol, p-menthane-3,8-diol, 6-isopropyl-9-methyl-1,4-dioxaspiro[4,5]decane-2-methanol, menthyl succinate and its alkaline earth metal salts, trimethylcyclohexanol, N-ethyl-2-isopropyl-5-methylcyclohexanecarboxamide, Japanese mint oil, peppermint oil, 3-(1-menthoxy)ethan-1-ol, 3-(1-menthoxy)propan-1-ol, 3-(1-menthoxy)butan-1-ol, 1-menthylacetic acid N-ethylamide, 1-menthyl-4-hydroxypentanoate, 1-menthyl-3-hydroxybutyrate, N,2,3-trimethyl-2-(1-methylethyl)-butanamide, n-ethyl-t-2-c-6 nonadienamide, N,N-dimethyl menthyl succinamide, substituted p-menthanes, substituted p-menthane-carboxamides, 2-isopropanyl-5-methylcyclohexanol (from Hisamitsu Pharmaceuticals, hereinafter "isopregol"); menthone glycerol ketals (FEMA 3807, tradename FRESCOLAT® type MGA); 3-1-menthoxypropane-1,2-diol (from Takasago, FEMA 3784); and menthyl lactate; (from Haarman & Reimer, FEMA 3748, tradename FRESCOLAT® type ML), WS-30, WS-14, Eucalyptus extract (p-Mehtha-3,8-Diol), Menthol (its natural or synthetic derivatives), Menthol PG carbonate, Menthol EG carbonate, Menthol glyceryl ether, N-tertbutyl-p-menthane-3-carboxamide, P-menthane-3-carboxylic acid glycerol ester, Methyl-2-isopryl-bicyclo (2.2.1), Heptane-2-carboxamide; and Menthol methyl ether, and menthyl pyrrolidone carboxylate among others. These and other suitable cooling agents are further described in the following U.S. patents, all of which are incorporated in their entirety by reference hereto: U.S. 4,230,688; 4,032,661; 4,459,425; 4,136,163; 5,266,592; 6,627,233.

In some embodiments, warming components may be selected from a wide variety of compounds known to provide the sensory signal of warming to the user. These compounds offer the perceived sensation of warmth, particularly in the oral cavity, and often enhance the perception of flavors, sweeteners and other organoleptic components. In some embodiments, useful warming compounds can include vanillyl alcohol n-butylether (TK-1000) supplied by Takasago Perfumary Company Limited, Tokyo, Japan, vanillyl alcohol n-propylether, vanillyl alcohol isopropylether, vanillyl alcohol isobutylether, vanillyl alcohol n-aminoether, vanillyl alcohol isoamyleather, vanillyl alcohol n-hexyleather, vanillyl alcohol methylether, vanillyl alcohol ethylether, gingerol, shogaol, paradol, zingerone, capsaicin, dihydrocapsaicin, nordihydrocapsaicin, homocapsaicin, homodihydrocapsaicin, ethanol, isopropyl alcohol, iso-amylalcohol, benzyl alcohol, glycerine, and combinations thereof.

In some embodiments, a tingling sensation can be provided. One such tingling sensation is provided by adding jambu, oleoresin, or spilanthol to some examples. In some embodiments, alkylamides extracted from materials such as jambu or sanshool can be included. Additionally, in some embodiments, a sensation is created due to effervescence. Such effervescence is created by combining an alkaline material with an acidic material. In some embodiments, an alkaline material can include alkali metal carbonates, alkali metal bicarbonates, alkaline earth metal carbonates, alkaline earth metal bicarbonates and mixtures thereof. In some embodiments, an acidic material can include acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof. Examples of "tingling" type sensates can be found in U.S. Patent No. 6,780,443, the entire contents of which are incorporated herein by reference for all purposes.

Sensate components may also be referred to as "trigeminal stimulants" such as those disclosed in U.S. Patent Application No. 205/0202118, which is incorporated herein by reference. Trigeminal stimulants are defined as an orally consumed product or agent that stimulates the trigeminal nerve. Examples of cooling agents which are trigeminal stimulants include menthol, WS-3, N-substituted p-menthane carboxamide, acyclic carboxamides including WS-23, methyl succinate, Menthone glycerol ketals, bulk sweeteners such as xylitol, erythritol, dextrose, and sorbitol, and combinations thereof. Trigeminal stimulants can also include flavors, tingling agents, Jambu extract, vanillyl alkyl ethers, such as vanillyl n-butyl ether, spilanthol, Echinacea extract, Northern Prickly Ash extract, capsaicin, capsicum oleoresin, red pepper oleoresin, black pepper oleoresin, piperine, ginger oleoresin, gingerol, shoagol, cinnamon oleoresin, cassia oleoresin, cinnamic aldehyde, eugenol, cyclic acetal of vanillin and menthol glycerin ether, unsaturated amides, and combinations thereof.

In some embodiments, sensate components are used at levels that provide a perceptible sensory experience i.e. at or above their threshold levels. In other embodiments, sensate components are used at levels below their threshold levels such that they do not provide an independent perceptible sensory experience. At subthreshold levels, the sensates may provide an ancillary benefit such as flavor or sweetness enhancement or potentiation.

### Breath Freshening Ingredients

Breath fresheners can include essential oils as well as various aldehydes, alcohols, and similar materials. In some embodiments, essential oils can include oils of spearmint, peppermint, wintergreen, sassafras, chlorophyll, citral, geraniol, cardamom, clove, sage, carvacrol, eucalyptus, cardamom, magnolia bark extract, marjoram, cinnamon, lemon, lime, grapefruit, and orange. In some embodiments, aldehydes such as cinnamic aldehyde and salicylaldehyde can be used. Additionally, chemicals such as menthol, carvone, iso-garrigol, and anethole can function as breath fresheners. Of these, the most commonly employed are oils of peppermint, spearmint and chlorophyll.

In addition to essential oils and chemicals derived from them, in some embodiments breath fresheners can include but are not limited to zinc citrate, zinc acetate, zinc fluoride, zinc ammonium sulfate, zinc bromide, zinc iodide, zinc chloride, zinc nitrate, zinc flurosilicate, zinc gluconate, zinc tartarate, zinc succinate, zinc formate, zinc chromate, zinc phenol sulfonate, zinc dithionate, zinc sulfate, silver nitrate, zinc salicylate, zinc glycerophosphate, copper nitrate, chlorophyll, copper chlorophyll, chlorophyllin, hydrogenated cottonseed oil, chlorine dioxide, beta cyclodextrin, zeolite, silica-based materials, carbon-based materials, enzymes such as laccase, and combinations thereof.

In some embodiments, the release profiles of probiotics can be managed for a confectionery including, but not limited to lactic acid producing microorganisms such as *Bacillus coagulans, Bacillus subtilis, Bacillus laterosporus, Bacillus laevolacticus, Sporolactobacillus inulinus, Lactobacillus acidophilus, Lactobacillus curvatus, Lactobacillus plantarurn, Lactobacillus jenseni, Lactobacillus casei, Lactobacillus fermentum. Lactococcus lactis, Pedioccocus acidilacti, Pedioccocus pentosaceus, Pedioccocus urinae, Leuconostoc mesenteroides, Bacillus coagulans, Bacillus subtilis, Bacillus laterosporus, Bacillus laevolacticus, Sporolactobacillus inulinus* and mixtures thereof. Breath fresheners are also known by the following trade names: Retsyn,™ Actizol,™ and Nutrazin.™ Examples of malodor-controlling compositions are also included in U.S. Patent No. 5,300,305 to Stapler et al. and in U.S. Patent Application Publication Nos. 2003/0215417 and 2004/0081713 which are incorporated in their entirety herein by reference for all purposes.

### Dental Care Ingredients

Dental care ingredients (also known as oral care ingredients) may include but are not limited to tooth whiteners, stain removers, oral cleaning, bleaching agents, desensitizing agents, dental remineralization agents, antibacterial agents, anticaries agents, plaque acid buffering agents, surfactants and anticalculus agents. Non-limiting examples of such ingredients can include, hydrolytic agents including proteolytic enzymes, abrasives such as hydrated silica, calcium carbonate, sodium bicarbonate and alumina, other active stain-removing components such as surface-active agents, including, but not limited to anionic surfactants such as sodium stearate, sodium palminate, sulfated butyl oleate, sodium oleate, salts of fumaric acid, glycerol, hydroxylated lecithin, sodium lauryl sulfate and chelators such as polyphosphates, which are typically employed as tartar control ingredients. In some embodiments, dental care ingredients can also include tetrasodium pyrophosphate and sodium tri-polyphosphate, sodium bicarbonate, sodium acid pyrophosphate, sodium tripolyphosphate, xylitol, sodium hexametaphosphate.

In some embodiments, peroxides such as carbamide peroxide, calcium peroxide, magnesium peroxide, sodium peroxide, hydrogen peroxide, and peroxydiphospate are included. In some embodiments, potassium nitrate and potassium citrate are included. Other examples can include casein glycomacropeptide, calcium casein peptone-calcium phosphate, casein phosphopeptides, casein phosphopeptide-amorphous calcium phosphate (CPP-ACP), and amorphous calcium phosphate. Still other examples can include papaine, krillase, pepsin, trypsin, lysozyme, dextranase, mutanase, glycoamylase, amylase, glucose oxidase, and combinations thereof.

Further examples can include surfactants such as sodium stearate, sodium ricinoleate, and sodium lauryl sulfate surfactants for use in some embodiments to achieve increased prophylactic action and to render the dental care ingredients more cosmetically acceptable. Surfactants can preferably be detersive materials which impart to the composition detersive and foaming properties. Suitable examples of surfactants are water-soluble salts of higher fatty acid monoglyceride monosulfates, such as the sodium salt of the monosulfated monoglyceride of hydgrogenated coconut oil fatty acids, higher alkyl sulfates such as sodium lauryl sulfate, alkyl aryl sulfonates such as sodium dodecyl benzene sulfonate, higher alkyl sulfoacetates, sodium lauryl sulfoacetate, higher fatty acid esters of 1,2-dihydroxy propane sulfonate, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium, and ethanolamine salts of N-lauroyl, N-myristoyl, or N-palmitoyl sarcosine.

In addition to surfactants, dental care ingredients can include antibacterial agents such as, but not limited to, triclosan, chlorhexidine, zinc citrate, silver nitrate, copper, limonene, and cetyl pyridinium chloride. In some embodiments, additional anticaries agents can include fluoride ions or fluorine-providing components such as inorganic fluoride salts. In some embodiments, soluble alkali metal salts, for example, sodium fluoride, potassium fluoride, sodium fluorosilicate, ammonium fluorosilicate, sodium monofluorophosphate, as well as tin fluorides, such as stannous fluoride and stannous chloride can be included. In some embodiments, a fluorine-containing compound having a beneficial effect on the care and hygiene of the oral cavity, e.g., diminution of enamel solubility in acid and protection of the teeth against decay may also be included as an ingredient. Examples thereof include sodium fluoride, stannous fluoride, potassium fluoride, potassium stannous fluoride (SnF.sub.2 -KF), sodium hexafluorostannate, stannous chlorofluoride, sodium fluorozirconate, and sodium monofluorophosphate. In some embodiments, urea is included.

Further examples are included in the following U.S. patents and U.S. published patent applications, the contents of all of which are incorporated in their entirety herein by reference for all purposes: U.S. Patent Nos. 5,227,154 to Reynolds, 5,378,131 to Greenberg, 6,846,500 to Luo et al., 6,733,818 to Luo et al., 6,696,044 to Luo et al., 6,685,916 to Holme et al., 6,485,739 to Luo et al., 6,479,071 to Holme et al., 6,471,945 to Luo et al., U.S. Patent Publication Nos. 20050025721 to Holme et al., 2005008732 to Gebreselassie et al., and 20040136928 to Holme et al.

### Active Ingredients

Actives generally refer to those ingredients that are included in a delivery system and/or confectionery composition for the desired end benefit they provide to the user. In some embodiments, actives can include medicaments, nutrients, nutraceuticals, herbals, nutritional.supplements, pharmaceuticals, drugs, and the like and combinations thereof.

Examples of useful drugs include ace-inhibitors, antianginal drugs, anti-arrhythmias, anti-asthmatics, anti-cholesterolemics, analgesics, anesthetics, anti-convulsants, anti-depressants, anti-diabetic agents, anti-diarrhea preparations, antidotes, anti-histamines, anti-hypertensive drugs, anti-inflammatory agents, anti-lipid agents, anti-manics, anti-nauseants, anti-stroke agents, anti-thyroid preparations, anti-tumor drugs, anti-viral agents, acne drugs, alkaloids, amino acid preparations, anti-tussives, anti-uricemic drugs, anti-viral drugs, anabolic preparations, systemic and non-systemic anti-infective agents, anti-neoplastics, anti-parkinsonian agents, anti-rheumatic agents, appetite stimulants, biological response modifiers, blood modifiers, bone metabolism regulators, cardiovascular agents, central nervous system stimulates, cholinesterase inhibitors, contraceptives, decongestants, dietary supplements, dopamine receptor agonists, endometriosis management agents, enzymes, erectile dysfunction therapies such as sildenafil citrate, which is currently marketed as Viagra™, fertility agents, gastrointestinal agents, homeopathic remedies, hormones, hypercalcemia and hypocalcemia management agents, immunomodulators, immunosuppressives, migraine preparations, motion sickness treatments, muscle relaxants, obesity management agents, osteoporosis preparations, oxytocics, parasympatholytics, parasympathomimetics, prostaglandins, psychotherapeutic agents, respiratory agents, sedatives, smoking cessation aids such as bromocryptine or nicotine, sympatholytics, tremor preparations, urinary tract agents, vasodilators, laxatives, antacids, ion exchange resins, antipyretics, appetite suppressants, expectorants, anti-anxiety agents, anti-ulcer agents, anti-inflammatory substances, coronary dilators, cerebral dilators, peripheral vasodilators, psycho-tropics, stimulants, anti-hypertensive drugs, vasoconstrictors, migraine treatments, antibiotics, tranquilizers, anti-psychotics, anti-tumor drugs, anti-coagulants, anti-thrombotic drugs, hypnotics, anti-emetics, anti-nauseants, anti-convulsants, neuromuscular drugs, hyper-and hypo-glycemic agents, thyroid and anti-thyroid preparations, diuretics, anti-spasmodics, terine relaxants, anti-obesity drugs, erythropoietic drugs, anti-asthmatics, cough suppressants, mucolytics, DNA and genetic modifying drugs, and combinations thereof.

Examples of active ingredients contemplated for use in some embodiments can include antacids, H2-antagonists, and analgesics. For example, antacid dosages can be prepared using the ingredients calcium carbonate alone or in combination with magnesium hydroxide, and/or aluminum hydroxide. Moreover, antacids can be used in combination with H2-antagonists.

Analgesics include opiates and opiate derivatives, such as Oxycontin™, ibuprofen, aspirin, acetaminophen, and combinations thereof that may optionally include caffeine.

Other drug active ingredients for use in embodiments can include anti-diarrheals such as Immodium™ AD, anti-histamines, anti-tussives, decongestants, vitamins, and breath fresheners. Also contemplated for use herein are anxiolytics such as Xanax™; anti-psychotics such as Clozaril™ and Haidol™; non-steroidal anti-inflammatories (NSAID's) such as ibuprofen, naproxen sodium, Voltaren™ and Lodine™, anti-histamines such as Claritin™, Hismanal™, Relafen™, and Tavist™; anti-emetics such as Kytril™ and Cesamet™; bronchodilators such as Bentolin™, Proventil™; anti-depressants such as Prozac™, Zoloft™, and Paxil™; anti-migraines such as Imigra™, ACE-inhibitors such as Vasotec™, Capoten™ and Zestril™; anti-Alzheimer's agents, such as Nicergoline™; and CaH-antagonists such as Procardia™, Adalat™, and Calan™.

The popular H2-antagonists which are contemplated for use in the present invention include cimetidine, ranitidine hydrochloride, famotidine, nizatidien, ebrotidine, mifentidine, roxatidine, pisatidine and aceroxatidine.

Active antacid ingredients can include, but are not limited to, the following: aluminum hydroxide, dihydroxyaluminum aminoacetate, aminoacetic acid, aluminum phosphate, dihydroxyaluminum sodium carbonate, bicarbonate, bismuth aluminate, bismuth carbonate, bismuth subcarbonate, bismuth subgallate, bismuth subnitrate, bismuth subsilysilate, calcium carbonate, calcium phosphate, citrate ion (acid or salt), amino acetic acid, hydrate magnesium aluminate sulfate, magaldrate, magnesium aluminosilicate, magnesium carbonate, magnesium glycinate, magnesium hydroxide, magnesium oxide, magnesium trisilicate, milk solids, aluminum mono-ordibasic calcium phosphate, tricalcium phosphate, potassium bicarbonate, sodium tartrate, sodium bicarbonate, magnesium aluminosilicates, tartaric acids and salts.

A variety of nutritional supplements may also be used as active ingredients including virtually any vitamin or mineral. For example, vitamin A, vitamin C, vitamin D, vitamin E, vitamin K, vitamin B₆, vitamin B₁₂, thiamine, riboflavin, biotin, folic acid, niacin, pantothenic acid, sodium, potassium, calcium, magnesium, phosphorus, sulfur, chlorine, iron, copper, iodine, zinc, selenium, manganese, choline, chromium, molybdenum, fluorine, cobalt and combinations thereof, may be used.

Examples of nutritional supplements that can be used as active ingredients are set forth in U.S. Patent Application Publication Nos. 2003/0157213 A1, 2003/0206993 and 2003/0099741 A1 which are incorporated in their entirety herein by reference for all purposes.

Various herbals may also be used as active ingredients such as those with various medicinal or dietary supplement properties. Herbals are generally aromatic plants or plant parts and or extracts thereof that can be used medicinally or for flavoring. Suitable herbals can be used singly or in various mixtures. Commonly used herbs include Echinacea, Goldenseal, Calendula, Rosemary, Thyme, Kava Kava, Aloe, Blood Root, Grapefruit Seed Extract, Black Cohosh, Ginseng, Guarana, Cranberry, Gingko Biloba, St. John's Wort, Evening Primrose Oil, Yohimbe Bark, Green Tea, Ma Huang, Maca, Bilberry, Lutein, and combinations thereof.

### Effervescing System Ingredients

An effervescent system may include one or more edible acids and one or more edible alkaline materials. The edible acid(s) and the edible alkaline material(s) may react together to generate effervescence.

In some embodiments, the alkaline material(s) may be selected from, but is not limited to, alkali metal carbonates, alkali metal bicarbonates, alkaline earth metal carbonates, alkaline earth metal bicarbonates, and combinations thereof. The edible acid(s) may be selected from, but is not limited to, citric acid, phosphoric acid, tartaric acid, malic acid, ascorbic acid, and combinations thereof. In some embodiments, an effervescing system may include one or more other ingredients such as, for example, carbon dioxide, oral care ingredients, flavorants, etc.

For examples of use of an effervescing system in a chewing confectionery, refer to U.S. Provisional Patent No. 60/618,222 filed October 13, 2004, and entitled "Effervescent Pressed Confectionery Tablet Compositions," the contents of which are incorporated herein by reference for all purposes. Other examples can be found in U.S. Patent No. 6,235,318, the contents of which are incorporated herein by reference for all purposes.

### Appetite Suppressor Ingredients

Appetite suppressors can be ingredients such as fiber and protein that function to depress the desire to consume food. Appetite suppressors can also include benzphetamine, diethylpropion, mazindol, phendimetrazine, phentermine, hoodia (P57), Olibra,™ ephedra, caffeine and combinations thereof. Appetite suppressors are also known by the following trade names: Adipex,™ Adipost,™ Bontril™ PDM, Bontril™ Slow Release, Didrex,™ Fastin,™ Ionamin,™ Mazanor,™ Melfiat,™ Obenix,™ Phendiet,™ Phendiet-105,™ Phentercot,™ Phentride,™ Plegine,™ Prelu-2,™ Pro-Fast,™ PT 105,™ Sanorex,™ Tenuate,™ Sanorex,™ Tenuate,™ Tenuate Dospan,™ Tepanil Ten-Tab,™ Teramine,™ and Zantryl.™ These and other suitable appetite suppressors are further described in the following U.S. patents, all of which are incorporated in their entirety by reference hereto: U.S. 6,838,431 to Portman, U.S. 6,716,815 to Portman, U.S. 6,558,690 to Portman, U.S. 6,468,962 to Portman, U.S. 6,436,899 to Portman.

### Potentiator Ingredients

Potentiators can include of materials that may intensify, supplement, modify or enhance the taste and/or aroma perception of an original material without introducing a characteristic taste and/or aroma perception of their own. In some embodiments, potentiators designed to intensify, supplement, modify, or enhance the perception of flavor, sweetness, tartness, umami, kokumi, saltiness and combinations thereof can be included.

In some embodiments, examples of suitable potentiators, also known as taste potentiators include, but are not limited to, neohesperidin dihydrochalcone, chlorogenic acid, alapyridaine, cynarin, miraculin, glupyridaine, pyridinium-betain compounds, glutamates, such as monosodium glutamate and monopotassium glutamate, neotame, thaumatin, tagatose, trehalose, salts, such as sodium chloride, monoammonium glycyrrhizinate, vanilla extract (in ethyl alcohol), sugar acids, potassium chloride, sodium acid sulfate, hydrolyzed vegetable proteins, hydrolyzed animal proteins, yeast extracts, adenosine monophosphate (AMP), glutathione, nucleotides, such as inosine monophosphate, disodium inosinate, xanthosine monophosphate, guanylate monophosphate, alapyridaine (N-(1-carboxyethyl)-6-(hydroxymethyl)pyridinium-3-ol inner salt, sugar beet extract (alcoholic extract), sugarcane leaf essence (alcoholic extract), curculin, strogin, mabinlin, gymnemic acid, hydroxybenzoic acids, 3-hydrobenzoic acid, 2,4-dihydrobenzoic acid, citrus aurantium, vanilla oleoresin, sugarcane leaf essence, maltol, ethyl maltol, vanillin, licorice glycyrrhizinates, compounds that respond to G-protein coupled receptors (T2Rs and TIRs) and taste potentiator compositions that impart kokumi, as disclosed in U.S. Patent No. 5,679,397 to Kuroda et al., which is incorporated in its entirety herein by reference. "Kokumi" refers to materials that impart "mouthfulness" and "good body".

Sweetener potentiators, which are a type of taste potentiator, enhance the taste of sweetness. In some embodiments, exemplary sweetener potentiators include, but are not limited to, monoammonium glycyrrhizinate, licorice glycyrrhizinates, citrus aurantium, alapyridaine, alapyridaine (N-(1-carboxyethyl)-6-(hydroxymethyl)pyridinium-3-ol) inner salt, miraculin, curculin, strogin, mabinlin, gymnemic acid, cynarin, glupyridaine, pyridinium-betain compounds, sugar beet extract, neotame, thaumatin, neohesperidin dihydrochalcone, hydroxybenzoic acids, tagatose, trehalose, maltol, ethyl maltol, vanilla extract, vanilla oleoresin, vanillin, sugar beet extract (alcoholic extract), sugarcane leaf essence (alcoholic extract), compounds that respond to G-protein coupled receptors (T2Rs and TIRs) and combinations thereof.

Additional examples of potentiators for the enhancement of salt taste include acidic peptides, such as those disclosed in U.S. Patent No. 6,974,597, herein incorporated by reference. Acidic peptides include peptides having a larger number of acidic amino acids, such as aspartic acid and glutamic acid, than basic amino acids, such as lysine, arginine and histidine. The acidic peptides are obtained by peptide synthesis or by subjecting proteins to hydrolysis using endopeptidase, and if necessary, to deamidation. Suitable proteins for use in the production of the acidic peptides or the peptides obtained by subjecting a protein to hydrolysis and deamidation include plant proteins, (e.g. wheat gluten, corn protein (e.g., zein and gluten meal), soybean protein isolate), animal proteins (e.g., milk proteins such as milk casein and milk whey protein, muscle proteins such as meat protein and fish meat protein, egg white protein and collagen), and microbial proteins (e.g., microbial cell protein and polypeptides produced by microorganisms).

The sensation of warming or cooling effects may also be prolonged with the use of a hydrophobic sweetener as described in U.S. Patent Application Publication 2003/0072842 A1 which is incorporated in its entirety herein by reference. For example, such hydrophobic sweeteners include those of the formulae I-XI as set forth below: 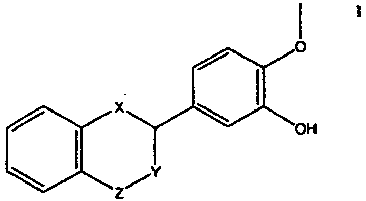 wherein X, Y and Z are selected from the group consisting of CH₂, O and S; 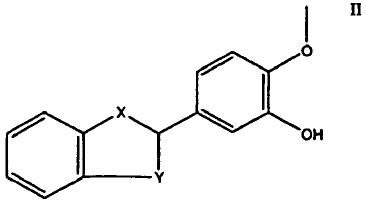 wherein X and Y are selected from the group consisting of S and O; 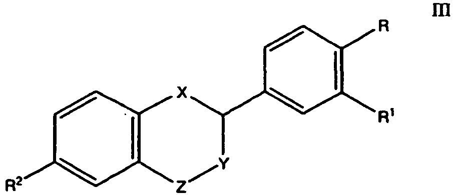 wherein X is S or O; Y is O or CH₂; Z is CH₂, SO₂ or S; R is OCH₃, OH or H; R¹ is SH or OH and R² is H or OH; 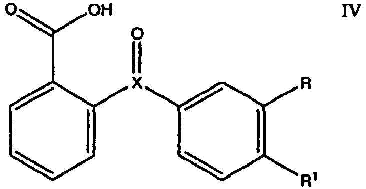 wherein X is C or S; R is OH or H and R¹ is OCH₃ or OH; 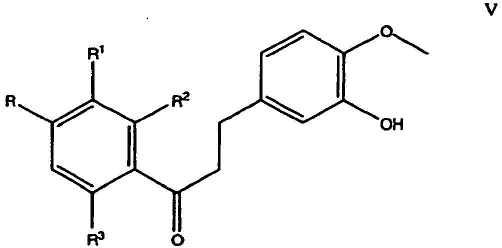 wherein R, R² and R³ are OH or H and R¹ is H or COOH; 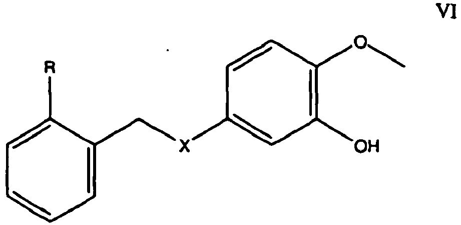 wherein X is O or CH₂ and R is COOH or H; 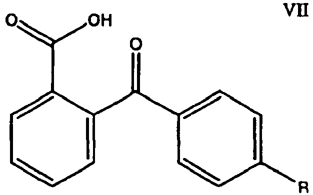 wherein R is CH₃CH₂, OH, N (CH₃)₂ or Cl; 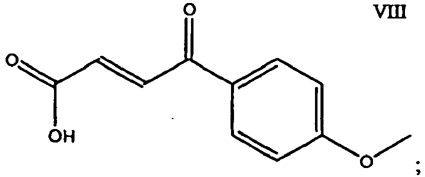 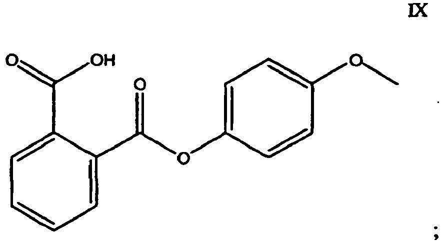 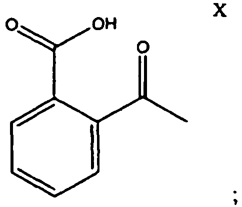 and

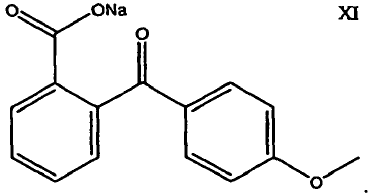

Perillartine may also be added as described in U.S. Patent No. 6,159,509 also incorporated in its entirety herein by reference.

### Food Acid Ingredients

Acids can include, but are not limited to acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof.

### Micronutrient Ingredients

Micronutrients can include materials that have an impact on the nutritional well being of an organism even though the quantity required by the organism to have the desired effect is small relative to macronutrients such as protein, carbohydrate, and fat. Micronutrients can include, but are not limited to vitamins, minerals, enzymes, phytochemicals, antioxidants, and combinations thereof.

In some embodiments, vitamins can include fat soluble vitamins such as vitamin A, vitamin D, vitamin E, and vitamin K and combinations thereof. In some embodiments, vitamins can include water soluble vitamins such as vitamin C (ascorbic acid), the B vitamins (thiamine or B₁, riboflavoin or B₂, niacin or B₃, pyridoxine or B₆, folic acid or B₉, cyanocobalimin or B₁₂, pantothenic acid, biotin), and combinations thereof.

In some embodiments minerals can include but are not limited to sodium, magnesium, chromium, iodine, iron, manganese, calcium, copper, fluoride, potassium, phosphorous, molybdenum, selenium, zinc, and combinations thereof.

In some embodiments micronutrients can include but are not limited to L-carnitine, choline, coenzyme Q10, alpha-lipoic acid, omega-3-fatty acids, pepsin, phytase, trypsin, lipases, proteases, cellulases, and combinations thereof.

Antioxidants can include materials that scavenge free radicals. In some embodiments, antioxidants can include but are not limited to ascorbic acid, citric acid, rosemary oil, vitamin A, vitamin E, vitamin E phosphate, tocopherols, di-alpha-tocopheryl phosphate, tocotrienols, alpha lipoic acid, dihydrolipoic acid, xanthophylls, beta cryptoxanthin, lycopene, lutein, zeaxanthin, astaxanthin, beta-carotene, carotenes, mixed carotenoids, polyphenols, flavonoids, and combinations thereof.

In some embodiments phytochemicals can include but are not limited to cartotenoids, chlorophyll, chlorophyllin, fiber, flavanoids, anthocyanins, cyaniding, delphinidin, malvidin, pelargonidin, peonidin, petunidin, flavanols, catechin, epicatechin, epigallocatechin, epigallocatechingallate (EGCG), theaflavins, thearubigins, proanthocyanins, flavonols, quercetin, kaempferol, myricetin, isorhamnetin, flavononeshesperetin, naringenin, eriodictyol, tangeretin, flavones, apigenin, luteolin, lignans, phytoestrogens, resveratrol, isoflavones, daidzein, genistein, glycitein, soy isoflavones, and combinations thereof.

### Mouth Moistening Ingredients

Mouth moisteners can include, but are not limited to, saliva stimulators such as acids and salts and combinations thereof. In some embodiments, acids can include acetic acid, adipic acid, ascorbic acid, butyric acid, citric acid, formic acid, fumaric acid, glyconic acid, lactic acid, phosphoric acid, malic acid, oxalic acid, succinic acid, tartaric acid and combinations thereof. In some embodiments, salts can include sodium chloride, calcium chloride, potassium chloride, magnesium chloride, sea salt, sodium citrate, and combinations thereof.

Mouth moisteners can also include hydrocolloid materials that hydrate and may adhere to oral surface to provide a sensation of mouth moistening. Hydrocolloid materials can include naturally occurring materials such as plant exudates, seed confectionerys, and seaweed extracts or they can be chemically modified materials such as cellulose, starch, or natural confectionery derivatives. In some embodiments, hydrocolloid materials can include pectin, gum arabic, acacia gum, alginates, agar, carageenans, guar gum, xanthan gum, locust bean gum, gelatin, gellan gum, galactomannans, tragacanth gum, karaya gum, curdlan, konjac, chitosan, xyloglucan, beta glucan, furcellaran, gum ghatti, tamarin, bacterial gums, and combinations thereof. Additionally, in some embodiments, modified natural gums such as propylene glycol alginate, carboxymethyl locust bean gum, low methoxyl pectin, and their combinations can be included. In some embodiments, modified celluloses can be included such as microcrystalline cellulose, carboxymethlcellulose (CMC), methylcellulose (MC), hydroxypropylmethylcellulose (HPCM), and hydroxypropylcellulose (MPC), and combinations thereof.

Similarly, humectants which can provide a perception of mouth hydration can be included. Such humectants can include, but are not limited to glycerol, sorbitol, polyethylene glycol, erythritol, and xylitol. Additionally, in some embodiments, fats can provide a perception of mouth moistening. Such fats can include medium chain triglycerides, vegetable oils, fish oils, mineral oils, and combinations thereof.

### Throat Care Ingredients

Throat soothing ingredients can include analgesics, anesthetics, demulcents, antiseptic, and combinations thereof. In some embodiments, analgesics/anesthetics can include menthol, phenol, hexylresorcinol, benzocaine, dyclonine hydrochloride, benzyl alcohol, salicyl alcohol, and combinations thereof. In some embodiments, demulcents can include but are not limited to slippery elm bark, pectin, gelatin, and combinations thereof. In some embodiments, antiseptic ingredients can include cetylpyridinium chloride, domiphen bromide, dequalinium chloride, and combinations thereof.

In some embodiments, antitussive ingredients such as chlophedianol hydrochloride, codeine, codeine phosphate, codeine sulfate, dextromethorphan, dextromethorphan hydrobromide, diphenhydramine citrate, and diphenhydramine hydrochloride, and combinations thereof can be included.

In some embodiments, throat soothing agents such as honey, propolis, aloe vera, glycerine, menthol and combinations thereof can be included. In still other embodiments, cough suppressants can be included. Such cough suppressants can fall into two groups: those that alter the consistency or production of phlegm such as mucolytics and expectorants; and those that suppress the coughing reflex such as codeine (narcotic cough suppressants), antihistamines, dextromethorphan and isoproterenol (non-narcotic cough suppressants). In some embodiments, ingredients from either or both groups can be included.

In still other embodiments, antitussives can include, but are not limited to, the group consisting of codeine, dextromethorphan, dextrorphan, diphenhydramine, hydrocodone, noscapine, oxycodone, pentoxyverine and combinations thereof. In some embodiments, antihistamines can include, but are not limited to, acrivastine, azatadine, brompheniramine, chlorpheniramine, clemastine, cyproheptadine, dexbrompheniramine, dimenhydrinate, diphenhydramine, doxylamine, hydroxyzine, meclizine, phenindamine, phenyltoloxamine, promethazine, pyrilamine, tripelennamine, triprolidine and combinations thereof. In some embodiments, non-sedating antihistamines can include, but are not limited to, astemizole, cetirizine, ebastine, fexofenadine, loratidine, terfenadine, and combinations thereof.

In some embodiments, expectorants can include, but are not limited to, ammonium chloride, guaifenesin, ipecac fluid extract, potassium iodide and combinations thereof. In some embodiments, mucolytics can include, but are not limited to, acetylcycsteine, ambroxol, bromhexine and combinations thereof. In some embodiments, analgesic, antipyretic and anti-inflammatory agents can include, but are not limited to, acetaminophen, aspirin, diclofenac, diflunisal, etodolac, fenoprofen, flurbiprofen, ibuprofen, ketoprofen, ketorolac, nabumetone, naproxen, piroxicam, caffeine and mixtures thereof. In some embodiments, local anesthetics can include, but are not limited to, lidocaine, benzocaine, phenol, dyclonine, benzonotate and mixtures thereof.

In some embodiments nasal decongestants and ingredients that provide the perception of nasal clearing can be included. In some embodiments, nasal decongestants can include but are not limited to phenylpropanolamine, pseudoephedrine, ephedrine, phenylephrine, oxymetazoline, and combinations thereof. In some embodiments ingredients that provide a perception of nasal clearing can include but are not limited to menthol, camphor, borneol, ephedrine, eucalyptus oil, peppermint oil, methyl salicylate, bornyl acetate, lavender oil, wasabi extracts, horseradish extracts, and combinations thereof. In some embodiments, a perception of nasal clearing can be provided by odoriferous essential oils, extracts from woods, confectionerys, flowers and other botanicals, resins, animal secretions, and synthetic aromatic materials.

### Coloring Ingredients

In some embodiments, one or more colors can be included. As classified by the United States Food, Drug, and Cosmetic Act (21 C.F.R. 73), colors can include exempt from certification colors (sometimes referred to as natural even though they can be synthetically manufactured) and certified colors (sometimes referred to as artificial), or combinations thereof. In some embodiments, exempt from certification or natural colors can include, but are not limited to annatto extract, (E160b), bixin, norbixin, astaxanthin, dehydrated beets (beet powder), beetroot red/betanin (E162), ultramarine blue, canthaxanthin (E161g), cryptoxanthin (E161c), rubixanthin (E161d), violanxanthin (E161e), rhodoxanthin (E161f), caramel (E150(a-d)), β-apo-8'-carotenal (E160e), β-carotene (E160a), alpha carotene, gamma carotene, ethyl ester of beta-apo-8 carotenal (E160f), flavoxanthin (E161a), lutein (E161b), cochineal extract (E120); carmine (E132), carmoisine/azorubine (E122), sodium copper chlorophyllin (E141), chlorophyll (E140), toasted partially defatted cooked cottonseed flour, ferrous gluconate, ferrous lactate, grape color extract, grape skin extract (enocianina), anthocyanins (E163), haematococcus algae meal, synthetic iron oxide, iron oxides and hydroxides (E172), fruit juice, vegetable juice, dried algae meal, tagetes (Aztec marigold) meal and extract, carrot oil, corn endosperm oil, paprika, paprika oleoresin, phaffia yeast, riboflavin (E101), saffron, titanium dioxide, turmeric (E100), turmeric oleoresin, amaranth (E123), capsanthin/capsorbin (E160c), lycopene (E160d), and combinations thereof.

In some embodiments, certified colors can include, but are not limited to, FD&C blue #1, FD&C blue #2, FD&C green #3, FD&C red #3, FD&C red #40, FD&C yellow #5 and FD&C yellow #6, tartrazine (E102), quinoline yellow (E104), sunset yellow (E110), ponceau (E124), erythrosine (E127), patent blue V (E131), titanium dioxide (E171), aluminium (E173), silver (E174), gold (E175), pigment rubine/lithol rubine BK (E180), calcium carbonate (E170), carbon black (E153), black PN/brilliant black BN (E151), green S/acid brilliant green BS (E142), and combinations thereof. In some embodiments, certified colors can include FD&C aluminum lakes. These include of the aluminum salts of FD&C dyes extended on an insoluble substrate of alumina hydrate. Additionally, in some embodiments, certified colors can be included as calcium salts.

### Multiple Ingredients

In some embodiments, a delivery system or confectionery composition may include two or more ingredients for which managed release from the confectionery composition during consumption of the confectionery composition is desired. In some embodiments, the ingredients may be encapsulated or otherwise included separately in different delivery systems. Alternatively, in some embodiments the ingredients may be encapsulated or otherwise included in the same delivery system. As another possibility, one or more of the ingredients may be free (e.g., unencapsulated) while one or more other ingredients may be encapsulated. Additionally, the multiple ingredients can be included in different portions of a confectionery composition.

A confectionery composition may include a group of ingredients for which managed release of the group during consumption of the confectionery composition is desired. Groups of two or more ingredients for which managed release from a confectionery composition during consumption of the confectionery composition may be desired include, but are not limited to: color and flavor, multiple flavors, multiple colors, cooling agent and flavor, warming agent and flavor, cooling agent and warning agent, cooling agent and high intensity sweetener, warming agent and high intensity sweetener, multiple cooling agents (e.g., WS-3 and WS-23, WS-3 and menthyl succinate), menthol and one or more cooling agents, menthol and one or more warming agents, multiple warming agents, high intensity sweetener(s) and tooth whitening active(s), high intensity sweetener(s) and breath freshening active(s), an ingredient with some bitterness and a bitterness suppressor for the ingredient, multiple high intensity sweeteners (e.g., ace-k and aspartame), multiple tooth whitening actives (e.g., an abrasive ingredient and an antimicrobial ingredient, a peroxide and a nitrate, a warming agent and a polyol, a cooling agent and a polyol, multiple polyols, a warming agent and micronutrient, a cooling agent and a micronutrient, a warming agent and a mouth moistening agent, a cooling agent and a mouth moistening agent, a warming agent and a throat care agent, a cooling agent and a throat care agent, a warming agent and a food acid, a cooling agent and food acid, a warming agent and an emulsifier/surfactant, a cooling agent and an emulsifier/surfactant, a warming agent and a color, a cooling agent and a color, a warming agent and a flavor potentiator, a cooling agent and a flavor potentiator, a warming agent with sweetness potentiator, a cooling agent with a sweetness potentiator, a warming agent and an appetite suppressant, a cooling agent and an appetite suppressant, a high intensity sweetener and a flavor, a cooling agent and a teeth whitening agent, a warming agent and a teeth whitening agent, a warming agent and breath freshening agent, a cooling agent and a breath freshening agent, a cooling agent and an effervescing system, a warming agent and an effervescing system, a warming agent and an antimicrobial agent, a cooling agent and an antimicrobial agent, multiple anticalculus ingredients, multiple remineralization ingredients, multiple surfactants, remineralization ingredients with demineralization ingredients, acidic ingredients with acid buffering ingredients, anticalculus ingredients with antibacterial ingredients, remineralization ingredients with anticalculus ingredients, anticalculus ingredients with remineralization ingredients with antibacterial ingredients, surfactant ingredients with anticalculus ingredients, surfactant ingredients with antibacterial ingredients, surfactant ingredients with remineralization ingredients, surfactants with anticalculus ingredients with antibacterial ingredients, multiple types of vitamins or minerals, multiple micronutrients, multiple acids, multiple antimicrobial ingredients, multiple breath freshening ingredients, breath freshening ingredients and antimicrobial ingredients, multiple appetite suppressors, acids and bases that react to effervesce, a bitter compound with a high intensity sweetener, a cooling agent and an appetite suppressant, a warming agent and an appetite suppressant, a high intensity sweetener and an appetite suppressant, a high intensity sweetener with an acid, a probiotic ingredient and a prebiotic ingredient, a vitamin and a mineral, a metabolic enhancement ingredient with a macronutrient, a metabolic enhancement ingredient with a micronutrient, an enzyme with a substrate, a high intensity sweetener with a sweetness potentiator, a cooling compound with a cooling potentiator, a flavor with a flavor potentiator, a warming compound with a warming potentiator, a flavor with salt, a high intensity sweetener with salt, an acid with salt, a cooling compound with salt, a warming compound with salt, a flavor with a surfactant, an astringent compound with an ingredient to provide a sensation of hydration, etc. In some embodiments, the multiple ingredients may be part of the same delivery system or may be part of different delivery systems. Different delivery systems may use the same or different encapsulating materials.

In some embodiments, encapsulation of the multiple ingredients will result in a delay in the release of the predominant amount of the multiple ingredients during consumption of a confectionery composition that includes the encapsulated multiple ingredients (e.g., as part of a delivery system added as an ingredient to the confectionery composition). This may be particularly helpful in situations wherein separate encapsulation of the ingredients may cause them to release with different release profiles. For example, different high intensity sweeteners may have different release profiles because they have different water solubilities or differences in other characteristics. Encapsulating them together may cause them to release more simultaneously.

In some embodiments, the release profile of the multiple ingredients can be managed for a confectionery composition by managing various characteristics of the multiple ingredients, the delivery system containing the multiple ingredients, and/or the portion of the confectionery composition containing the delivery system and/or how the delivery system is made in a manner as previously discussed above.

The additional components, as described above, may be used in any portion of the confectionery composition such as in the saccharide portion, the elastomeric portion, the coating, or the center-fill as desired. Suitable amounts for the additional components are set forth in Table 2, above. The amounts in Table 2 generally apply to each of the additional components as they may be added to a confectionery composition in a free form, i.e., unencapsulated. In some embodiments, where the additional component is provided in an encapsulated form, an amount greater than those amounts as set forth in Table 2 may be used due to the modified release profile of the additional component. Also, because many of the additional components shown in Table 2 are optional, the amounts represent amounts used when the component is selected for inclusion in the composition. In other words, the lower limit of 0% is not included even though the additional component is an optional component.

The components listed in Table 2, above, may be added to any portion of the confectionery composition in their encapsulated and/or unencapsulated forms, as well as in combination with any of the other optional components. For example, a single component may be added to a confectionery composition in its encapsulated and unencapsulated forms. The two different forms of the component may be added to the same or different portions of the confectionery composition the same or different amounts.

In some embodiments, a single component may be added in two or more different encapsulated forms. In particular, two or more different encapsulating materials, such as different polymers, may be used to encapsulate two or more separate portions of the component. The different encapsulated forms of the same component may be added to the same or different portions of the confectionery composition in the same or different amounts. Further, in some embodiments, an unencapsulated form of the same component may be added in combination with the two or more different encapsulated forms. The unencapsulated form of the component may be added to any portion of the confectionery composition in the same or different amount from the encapsulated forms. Moreover, some embodiments may add an unencapsulated form of a similar component in combination with the two or more different encapsulated forms. For example, two encapsulated forms of a single sweetener may be used in combination with an unencapsulated form of a different sweetener.

In some embodiments, combinations of two or more different components from Table 2, above, may be employed. In some embodiments, at least one of the components may be encapsulated, while at least one of the components may be unencapsulated. The multiple components may be the same type of component, e.g., two different sweeteners, or components from distinctly different categories, e.g., a sweetener and a warming agent. The different components may be added to the same or different portions of the confectionery composition in the same or different amounts.

Some embodiments may include multiple components from Table 2, above, each of which is encapsulated. The multiple encapsulated components may be included in the same or different portions of the confectionery composition in the same or different amounts. The multiple encapsulated components may be the same type of component or from distinctly different categories.

In some embodiments in which multiple encapsulated components are added to the confectionery composition, the multiple components may be encapsulated together or separately. In embodiments in which the multiple components are encapsulated together, the components may be mixed together and encapsulated by a single encapsulating material. In embodiments in which the multiple components are encapsulated separately, the material used to encapsulate the components may be the same or different. The amounts provided for the components are based on the specified portion in which the component is contained.

As described above, Table 2 provides a list of components which may optionally be present in one or more portions of the confectionery product. Suitable amounts which may be present in the coating, center-fill, saccharide portion, or elastomeric portion are provided in the table. The amounts in Table 2 are provided as ppm or weight % in a portion or layer of the confectionery product. Table 2 is only representative and is not be construed to limit the ingredients that can be included in the confectionery composition portions in any way.

### Processing

Confectionery compositions can be created by mixing the saccharide portion as described with compositions creating the elastomeric portion using any technique known in the art. For example, mixers including, but not limited to, static mixers, sigma blade mixers, Hobart mixers, Z-blade gum mixers, kneaders, single screw extruder, twin screw extruder, blade-and-pin mixers, etc. can be used to blend specified proportions of the compositions. In some embodiments, a mixer can be steam or hot fluid jacketed, or otherwise heated to maintain the mixing temperature at a minimum or otherwise consistent level.

As used herein, "a continuous mixer" is processing equipment in which the various ingredients used to prepare a composition are fed substantially continuously into the device whilst those ingredients are being mixed and removed or ejected from the mixing apparatus. For example, in a continuous mixing extruder, some ingredients are substantially continuously introduced through various feed ports while others are introduced downstream, all the while the screws, blades or other mixing elements continuing to convey the mixture through the apparatus, all the while mixing the same. At a downstream portion of the extruder, the wholly or partly combined mass is ejected from the extruder by the force of the mass continually being conveyed and/or facilitated by an external pump.

In some embodiments, a confectionery composition is formed by blending 5% - 95% w/w of a saccharide composition together with 5% - 95% w/w of an elastomeric composition. In some embodiments, the composition representing the larger proportion of the confectionery composition is metered or loaded into the mixer first. Then, the composition representing the smaller proportion of the confectionery composition is added to the mixer, with mixing and the final confectionery composition is removed from the mixer once a homogeneous mass is achieved. In some embodiments, the composition representing the small proportion of the confectionery composition is metered or loaded into the mixer first, then, the composition representing the larger proportion of the confectionery composition is added to the mixer. Depending on the nature of the saccharide and elastomeric compositions, the mixer may involve different mixing actions. In some embodiments, a highly distributive mixer supplying vigorous mixing action can be used while in other embodiments, a less intense mixer supplying gentle mixing action can be used.

The saccharide component can be created by applying a heat process that increases the solids content of the saccharide component by removing moisture from an aqueous saccharide solution. In other embodiments, the saccharide component can be created by increasing the solids content of a saccharide without a heat process such as by incorporating solid saccharides into an aqueous syrup.

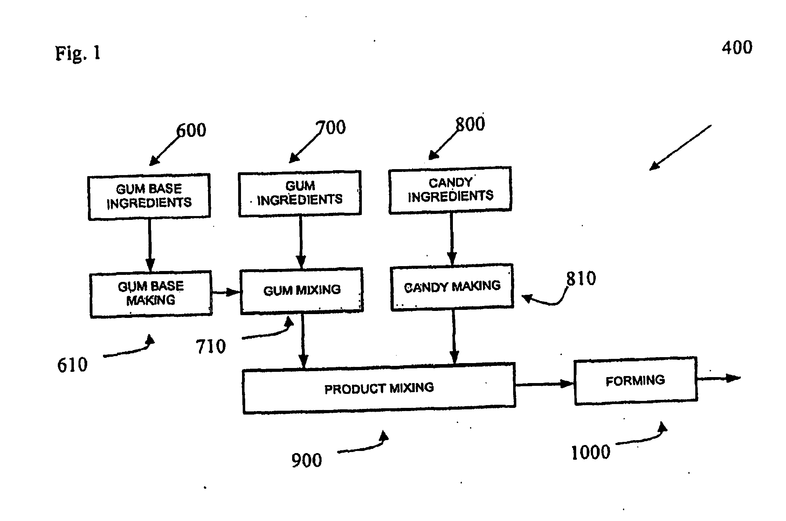

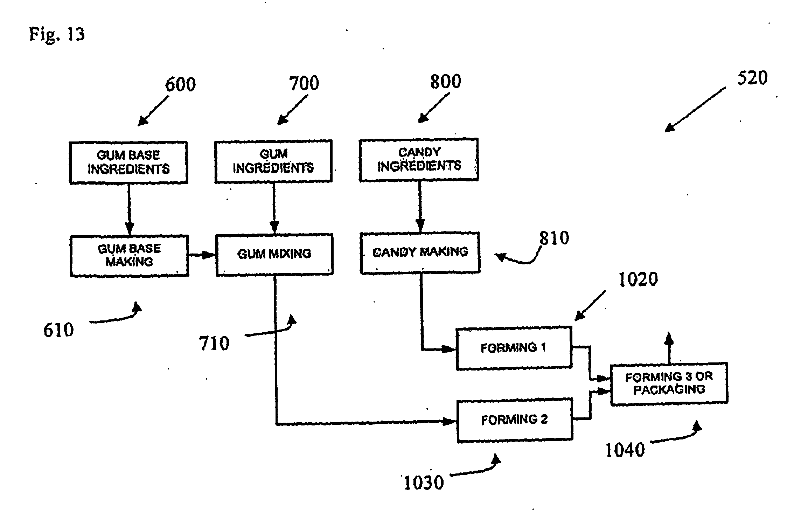

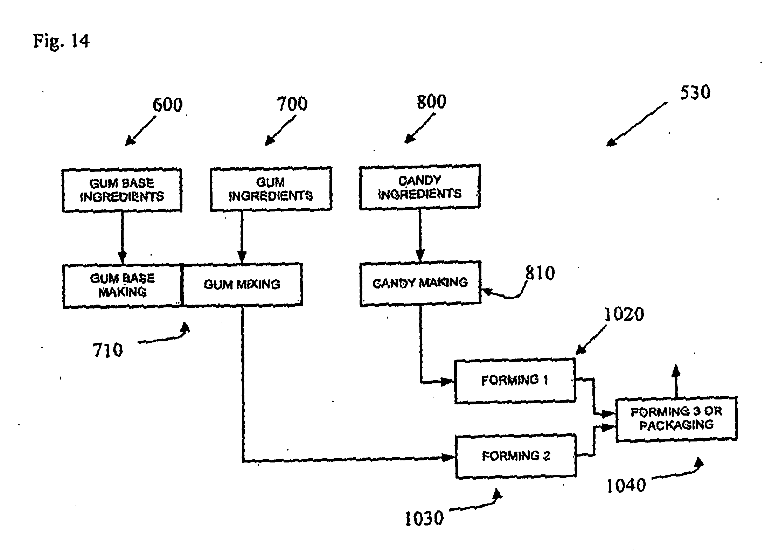

The confectionery composition can be created using a continuous process, a batch process or combinations of these. For example, in one embodiment, the saccharide portion is processed in a batch mixer while the chewing gum base portion is processed in a continuous manner. While combining them is performed in a batch mixing apparatus. In another embodiment, both the saccharide and chewing gum base portions are processed in batch equipment as well as the mixing of the two components together. In another embodiment, both are prepared using continuous processing apprati as well as the mixing of the two components together. Variations of batch and continous mixing to process the confectionery as described herein are also envisioned. For example, table 5 below outlines some of these variations of mixing, stage 1 forming and final forming. For example in the processing embodiments depicted in Figs. 13 and 14, the mixing is where the gum base portion and the candy portion are mixed separately (and, if used, chewing gum ingredients are combined) and then formed and combined in a final forming stage. In other embodiments, e.g., in reference to Fig. 1, after the portions have been combined the product is subjected to forming processes which can include two stages, e.g., a first forming and final forming, e.g., when the product is formed into a first form and then combined with a second product in the final forming stage. In Table 5, (A) is manual mixing, (B) is a bowl mixer, (C) is a vertical cone mixer, (D) is a Ruffinatti kneader, (E) is a double arm Z-blade, sigma blade mixer, dough kneader, dough mixer or mix-truder, (F) is a static mixer, (G) is a pin mixer, (H) is a mixing or kneading extruder, (I) is an Executive or Ruffinatti puller, (AA) is a batch former with a rope sizer, (BB) is an extruder with or without a rope sizer, (AAA) is a chain die, (BBB) is a rotary die, (CCC) is cut and wrap, and (DDD) is drop roll.

| **Mixing** | **Stage 1 forming** | **Final Forming** | | **Mixing** | **Stage 1 forming** | **Final Forming** | | **Mixing** | **Stage 1 forming** | **Final Forming** |
|---|---|---|---|---|---|---|---|---|---|---|
| A | AA | AAA | | D | AA | AAA | | G | AA | AAA |
| A | AA | BBB | | D | AA | BBB | | G | AA | BBB |
| A | AA | CCC | | D | AA | CCC | | G | AA | CCC |
| A | AA | DDD | | D | AA | DDD | | G | AA | DDD |
| A | BB | AAA | | D | BB | AAA | | G | BB | AAA |
| A | BB | BBB | | D | BB | BBB | | G | BB | BBB |
| A | BB | CCC | | D | BB | CCC | | G | BB | CCC |
| A | BB | DDD | | D | BB | DDD | | G | BB | DDD |
| B | AA | AAA | | E | AA | AAA | | H | AA | AAA |
| B | AA | BBB | | E | AA | BBB | | H | AA | BBB |
| B | AA | CCC | | E | AA | CCC | | H | AA | CCC |
| B | AA | DDD | | E | AA | DDD | | H | AA | DDD |
| B | BB | AAA | | E | BB | AAA | | H | BB | AAA |
| B | BB | BBB | | E | BB | BBB | | H | BB | BBB |
| B | BB | CCC | | E | BB | CCC | | H | BB | CCC |
| B | BB | DDD | | E | BB | DDD | | H | BB | DDD |
| C | AA | AAA | | F | AA | AAA | | I | AA | AAA |
| C | AA | BBB | | F | AA | BBB | | I | AA | BBB |
| C | AA | CCC | | F | AA | CCC | | I | AA | CCC |
| C | AA | DDD | | F | AA | DDD | | I | AA | DDD |
| C | BB | AAA | | F | BB | AAA | | I | BB | AAA |
| C | BB | BBB | | F | BB | BBB | | I | BB | BBB |
| C | BB | CCC | | F | BB | CCC | | I | BB | CCC |
| C | BB | DDD | | F | BB | DDD | | I | BB | DDD |

In some embodiments, a continuous process employing a mixing or kneading extruder is used to blend the saccharide composition and the elastomeric portion. As with batch processes, the nature of the saccharide and elastomeric compositions dictates the type of mixing elements used in the extruder. In some embodiments, highly distributive mixing elements can be used while in other embodiments; less intensive mixing can be used. In one particularly advantageous embodiment of using an extruder to process the saccharide and gum base portions together is that the confectionery that comes out of the extruder, after cooling exhibits a shine or gloss that is about the same or, in some cases, greater than the shine or gloss on a typically coated and polished gum product as known in the field. Preferably, to achieve a glossy confection, high pressure-shear is used with a die pressure and temperature close to the glass transition temperature of the polymeric matrix making up the gum base. In one embodiment, such shiny or glossy confectioneries have a lower porosity when compared to a conventional chewing gum. Without being limited to a particular theory, when the gum matrix is uniform and there is no or very little crystalline bulking agents (sugar/sugar alcohol), high pressure/shear extrusion results in a smooth and glossy surface.

In some embodiments, to achieve a glossy confection from an extruder, the processing temperature is from 60-100 °C, including 60-80 °C with a mixing time of 10 seconds to 30 minutes. In one embodiment, to achieve a glossy confection from the extruder, the processing temperature is around 60°C for a sugar free composition. In another embodiment, to achieve a glossy confection from the extruder, the processing temperature is around 100°C for a composition containing a sugar.

In addition, the amount of certain ingredients can affect the texture of such a product, with examples of amounts of ingredients including the saccharide in an amount of 5 to 95% by weight, elastomeric material in an amount of 5-45% by weight, fats in an amount of 0.1 to 6% by weight, moisture in an amount of 0.01-8% by weight, polymers such as gelatin, starch, and proteins in an amount of 0.1-10% by weight, soluble powders such as sugar and polyols in an amount of 0.1-30% by weight, and insoluble powder such as talc, dicalcium phosphate, and silica in an amount of 0.1-20% by weight. In addition, non-limiting examples of ingredients that can be used to enhance the gloss when added to the saccharide portion and/or the gum base portion include, titanium oxide, mica, polyethylene glycol (e.g., PEG 6000), wax (polishing), and/or pearlescent pigments. In certain embodiments, it is preferably to add these gloss enhancing components to the saccharide portion.

In some embodiments, the ingredients being added, e.g., to a batch mixer, can be added all at once or at spatially distinct times. In a similar manner, the addition of ingredients in a continuous mixing apparatus, e.g., an extruder can be at different positions so as to partially or wholly mix the ingredients added to a certain point prior to the addition of further ingredients.

In some embodiments, some or all of one or more of the ingredients added to the gum portion, the candy or saccharide portion, the coating, the shell, the center-fill, etc. may be encapsulated.

In some embodiments, the same ingredient may be added in both encapsulated and free forms in the same portion of the edible composition and/or in different portions of the candy gum.

In some embodiments, an edible composition may include multiples of the same types of ingredient (e.g., sensates, functional ingredients, flavor, color, food acid, sweetener, potentiator) which may be located in the same portion of the edible composition or in different portions, free and/or encapsulated.

As with the mixing operation, confectionery product forming operations can include any technique known in the art. In some embodiments, the confectionery product can be formed using rolling and scoring operations, cut and wrap operations, chain die operations, or any other confectionery or chewing gum forming operation. Additionally, in some embodiments, the viscosity of the confectionery composition can be low enough to employ confectionery depositing operations.

One processing challenge to combining two portions of a confectionery that are traditionally used to make saccharide compositions (e.g., candies) and chewing gum is that the processing temperatures vary. For example, typically chewing gums are processed at a lower temperature than candies. In addition, sugars tend to be more temperature sensitive because they can contain reducing sugars that brown at elevated temperatures whereas sugar free products made from polyols do not typically brown.

In one embodiment, the gum portion and the saccharide portion are mixed at a temperature of at least 100°C.

When amorphous isomalt is the main or substantially only component in the saccharide portion, the saccharide portion and the gum base portion can be mixed at a temperature of at least 85-90°C.

In one embodiment of the invention, prior to mixing the gum base portion with the saccharide portion the saccharide is tempered to about 70-100°C, for example, 70-80°C. The saccharide if it includes substantially or completely polyols such as isomalt, it can be tempered to a temperature from about 50-70°C, for example 50-55°C. For example, in one embodiment, the saccharide component is cooked in a batch cooker at 140°C and the gum base is melted at 110°C and mixed at a temperature of 130°C. The components are combined and then tempered to about 80°C to form a confectionery. In another example, the saccharide is cooked at a temperature of 140°C for a time to reduce the water content to about 2% or less (by weight) whilst melting the gum base, the two portions are combined and mixed at 110°C whilst also adding colorants, flavors and/or other ingredients. This mixture can then be tempered to about 65-80°C cooled, formed, shaped and/or packaged.

Cost savings may arise because the confectionery compositions can be processed using equipment designed for confectionery compositions such as hard and soft candies. Additionally, in some embodiments, confectionery compositions may be processed without the need for some chewing gum unit operations such as rolling and scoring and conditioning. The confectionery compositions can demonstrate shelf life stability that negates the need for moisture resistant packaging.

Cost savings may arise because a saccharide portion contains an amount of water that substitutes for more expensive components such as those in an elastomeric portion.

Cost savings may arise because a saccharide composition can include a higher amount of a cheaper material. For example, in some embodiments, a starch can be used to replace sucrose and/or corn syrup.

In some embodiments, release (non-stick) agents can be used during the processing of the confectionery described herein to facilitate processability of the mass and/or to reduce sticking of the mass to the processing equipment. The release agent can be powder and/or oil based and be applied and/or sprayed onto the confectionery mass (before and/or during processing), the processing equipment or both. The adherence of the release agent on the surface of the confectionery can also provide the advantage of a pleasant initial sensory experience upon consumption of the confectionery. Additionally or alternatively, the apparatus can be cooled and/or heated to minimize reduce sticking of the mass to the processing equipment.

Non-limiting examples of release agents include, calcium hydroxide, talc, D-mannitol, silicon dioxide, sucrose ester, calcium stearate, zinc stearate, magnesium stearate, polyoxyethylene monostearate, silicates, polyethylene glycols, silicate dioxide, fumed silica, stearic acid, calcium carbonate, oily or fatty substances such as vegetable and/or animal oils and/or mineral oils. Mixtures of these can also be used. In one preferred embodiment, powder based release agents are used.

Although powdered polyols, such as sorbitol are mentioned above, in one particularly advantageous embodiment of the present invention is that by adding such powdered polyols to the processing steps, e.g., during the stage of combining the saccharide portion and the gum base portion, the powdered polyol can facilitate disruption of the matrix. In certain embodiments, such as when polyol syrups are used to prepare the saccharide portion, the powdered polyols can reduce friability of the product because the powdered polyol, e.g., sorbitol, does not seed the polyols. Although in some embodiments, seeding of the polyol is acceptable, in certain embodiments where certain textures are being generated, the addition of the powdered polyol permits controlling the texture of the polyol during its cooking process, while combining with the gum base, or both. In this regard, the product will be less glassy and more friable or softer with a higher quantity of powdered polyol and conversely, the opposite would be occur if lower amounts of powdered polyol is employed.

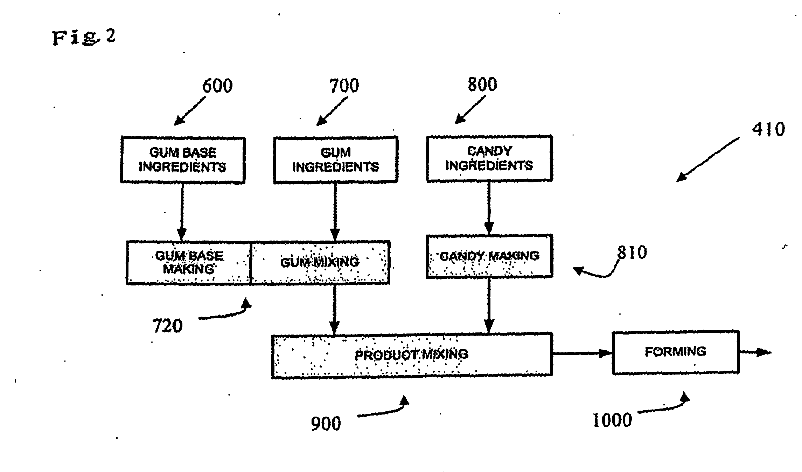

Referring to Figs. 1 and 2, processing (400 or 410) of the confectionery composition can be performed whereby gum base ingredients (600) are compounded in a mixing apparatus (610), e.g., a dispersive mixing apparatus or a mixing apparatus having at least a portion therewith including dispersive mixing elements. The completed gum base is then optinally mixed with chewing gum ingredients (700). This can be performed in separate mixers (610, 710, Fig. 1) or in the same mixer (720 Fig. 2), e.g., in a downstream portion of the mixer used to compound the chewing gum base.

Separately, a candy composition is prepared (810) whereby sugar and/or other saccharides, e.g., polyols such as isomalt, (800) are heated, tempered and may also be combined with other flavorants, colorants, and/or functional ingredients.

The completed chewing gum composition and the candy composition are then combined (900), preferably at a temperature at which the candy and gum base are partly or wholly in a molten state. In one embodiment, the combination of chewing gum and candy portions of the confectionery composition are mixed to achieve a substantially homogenous distribution of one portion with the other. In other embodiments, however, it is also possible to have a non-homogenous distribution of e.g., the candy portion in the chewing gum portion so as to provide a unique sensory perception. For example, by not homogenously mixing the candy portion with the chewing gum portion, small portions of candy will be contained in the chewing gum to provide different levels of crunch.

After the chewing gum and candy portions have been suitably mixed, the mixture can be passed to one or more downstream forming, polishing, coating and/or packaging steps (1000) as conventionally used in the art.

The gum base, gum mixing if performed, and candy processing can be done in an extruder or batch mixer as well as in the same extruder or batch mixer. In addition, combinations of such devices can also be used, e.g., gum base and chewing gum mixing can be done in the same extruder or batch mixer and if performed in an extruder, at different addition ports or inlets in the extruder. Although described in relation to Figs. 1 and 2, if a continuous mixer, such as an extruder is used, the mixer can be configured to have particular elements for applying the requisite mixing at the different positions of ingredient addition. For example, at the point where gum base is initially added for mixing, high shear or dispersive mixing elements may be used and where the optional chewing gum ingredients and/or candy portion/ingredients are added a lower shear element(s) may be used (e.g., distributive mixing elements).

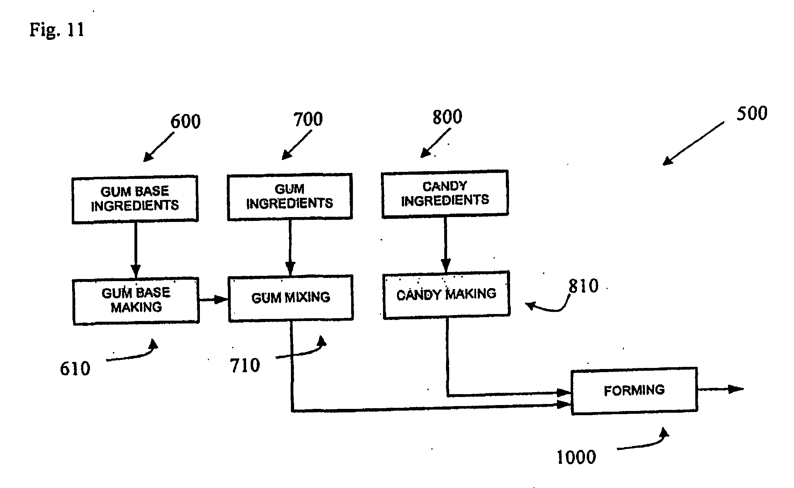

In other embodiments of the processes in Figs. 1 and 2 (see Figs. 11 and 12, 500 and 510) the mixing of the chewing gum portion and the candy portion is accomplished not in a mixing process but incorporated together during subsequent manipulation steps, such as forming processes (1010).

As discussed further below, forming can create pellets, chunks, pillows, laminates or other multi-layered or multi-regioned products, cylindrical canes, multi-layered products with different colors, slabs, center-fill or multi-extruded products, sticks, twists, etc. Laminates or multi-region products may include one or more layers/regions of candy/gum, candy, gum, chocolate, candy particles, nuts, fruit, slurries, gels, fat pastes (e.g., peanut butter, hazelnut cream), powders, etc.

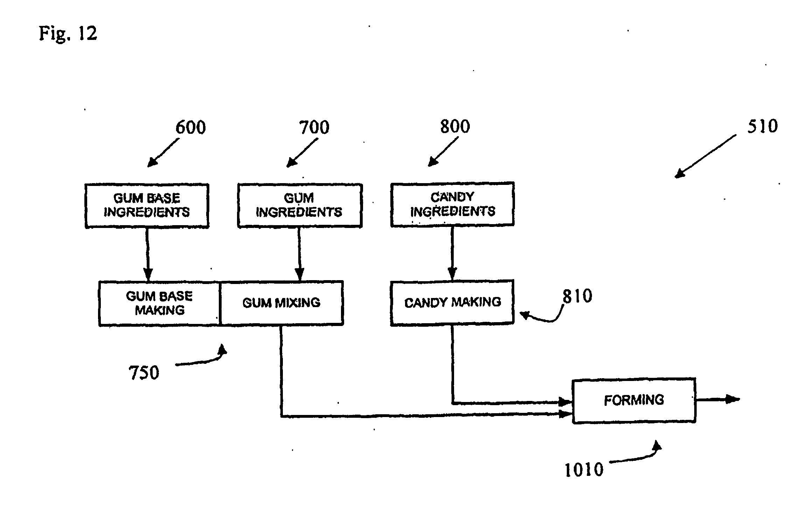

In another embodiment of the processes in Figs. 11 and 12 (500 and 510), each of the gum and candy portions are preformed (1020, 1030) prior to being combined together during a forming process and/or the packaging process, 1040 (Figs. 13 and 14, 520 and 530).

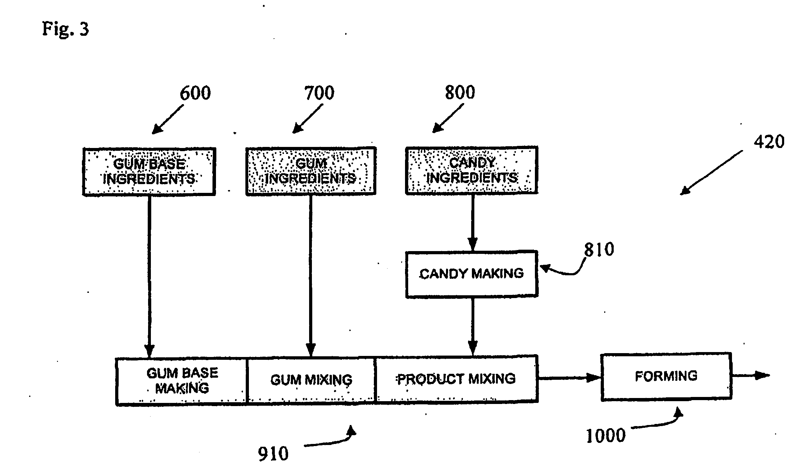

Referring to Fig. 3, the confectionery composition is made in a continuous mixing apparatus (910), which can be either physically the same apparatus or a series of devices set up in series. In the alternative, a single batch mixer can be used (as 910) whereby the different ingredients/components are added during the different times during the manufacturing process. In this embodiment, the gum base ingredients (600) are added and mixed (910), wholly or partly, e.g., by dispersive mixing. Thereafter, if used, the chewing gum ingredients (700) are added and mixed with the gum base portion whereby the chewing gum ingredients are mixed with a finished gum base or the gum base is finally compounded while being mixed with the chewing gum ingredients (910). This mixture, substantially homogenously mixed or partly so, is then mixed (910) with a separately made candy portion

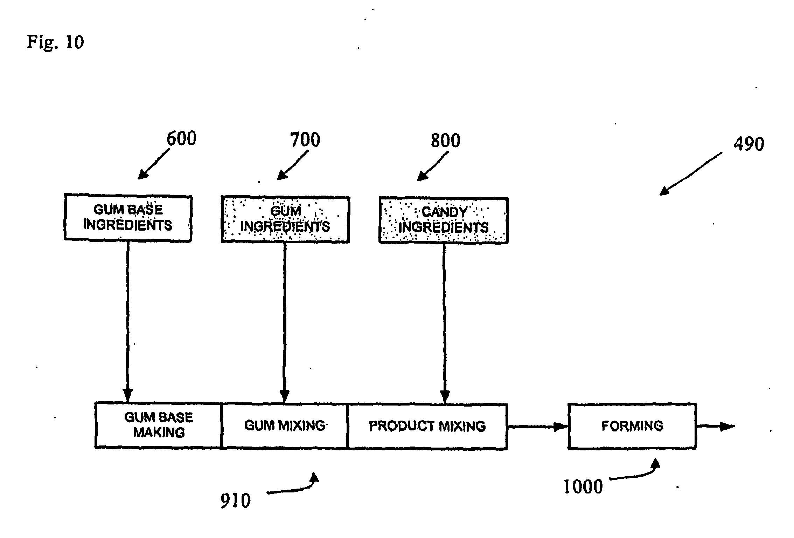

(810) as described above. In another embodiment (Fig. 10), the candy portion is not substantially prepared prior to its addition to the mixing process.

After the chewing gum and candy portions have been suitably mixed, the mixture can be passed to one or more downstream forming, polishing, coating and/or packaging steps (1000) as conventionally used in the art and described herein.

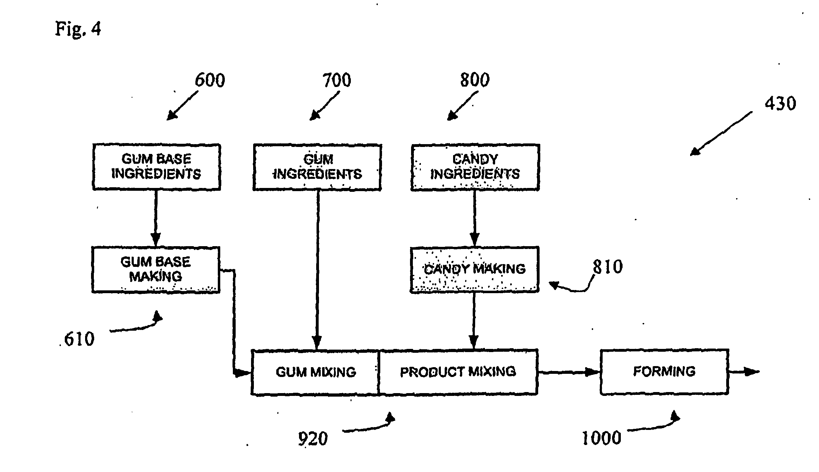

Referring to Fig. 4 (430), the gum base is manufactured (610) before being added to a mixing process (920) where the chewing gum ingredients (700), if used, are added to the gum base mixed to achieve either a substantially homogenous mixture or partly mixed and thereafter, a wholly or partly made candy (810) is added thereto and mixed to form a confectionery composition as described herein. The components can be made in continuous and/or batch mixing processes as described herein.

After the chewing gum and candy portions have been suitably mixed, the mixture can be passed to one or more downstream forming, polishing, coating and/or packaging steps (1000) as conventionally used in the art.

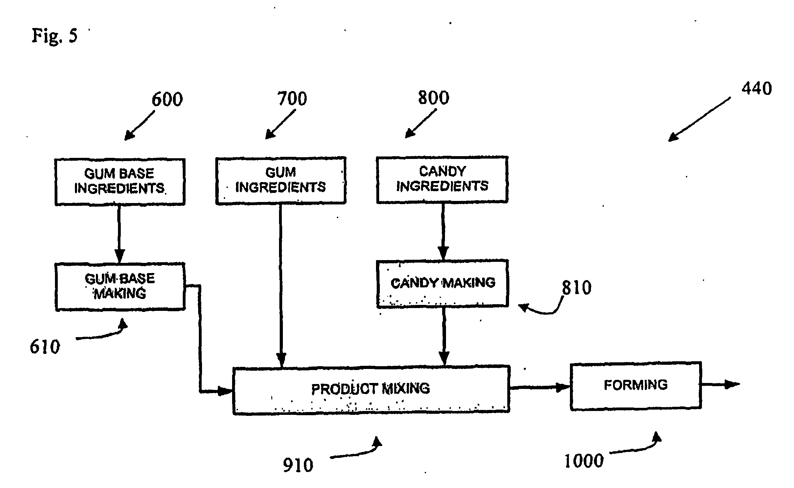

Referring to Fig. 5 (440), the gum base is manufactured (610) before being added to a mixing process (910) where the chewing gum ingredients (700), if used and a wholly or partly premade candy (810) are added to the gum base and mixed (910) until the desired homogeneity is achieved to yield a confectionery composition as described herein. The components can be made in a continuous and/or batch mixing processes as described herein.

After the chewing gum and candy portions have been suitably mixed, the mixture can be passed to one or more downstream forming, polishing, coating and/or packaging steps (1000) as conventionally used in the art.

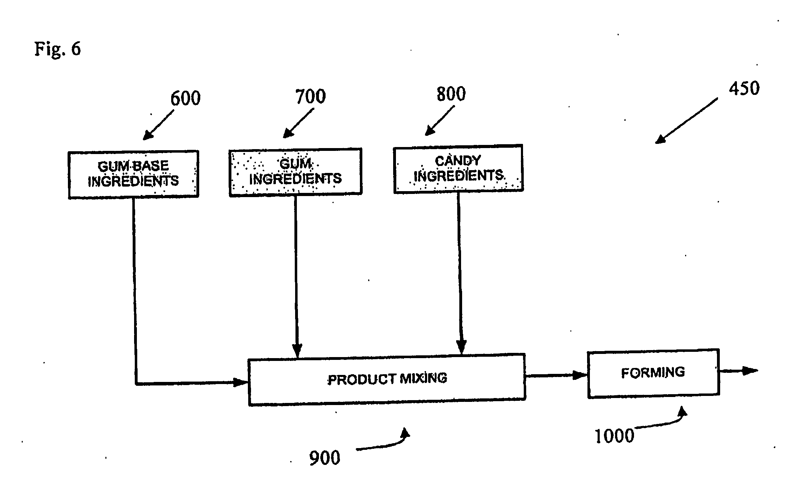

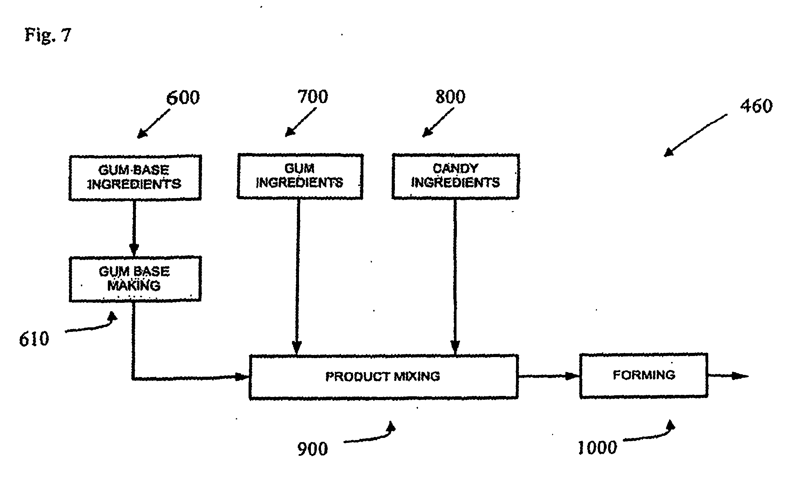

In the embodiment depicted in Fig. 6 (450), all of the ingredients (600, 700, 800) of the confectionery composition are added, simulatenously or sequentially, to a single mixing apparatus or a series of mixers set up in tandem (900) and compounded together prior to subsequent manipulations, such as forming (100). In one preferred aspect of this embodiment, the gum base is at least partly mixed prior to the addition of the chewing gum ingredients (700), if used, and/or the candy ingredients (800). Additionally, or alternatively, the candy ingredients (800) are added prior to the chewing gum ingredients (700) such that the mixing process (900) can be performed at an elevated temperature suitably to achieve at least partial heating of the sugar and/or polyol portions of the candy portion so as to avoid unnecessary volatilization and/or degradation of certain heat labile chewing gum ingredients. Fig. 7 (460) depicts an embodiment where a wholly or partly pre-made gum base (610) is added to a similar processing line (900) as discussed above for Fig. 6. Again, the chewing gum ingredient addition to the gum base is optional and the components can be made in continuous and/or batch mixing processes as described herein.

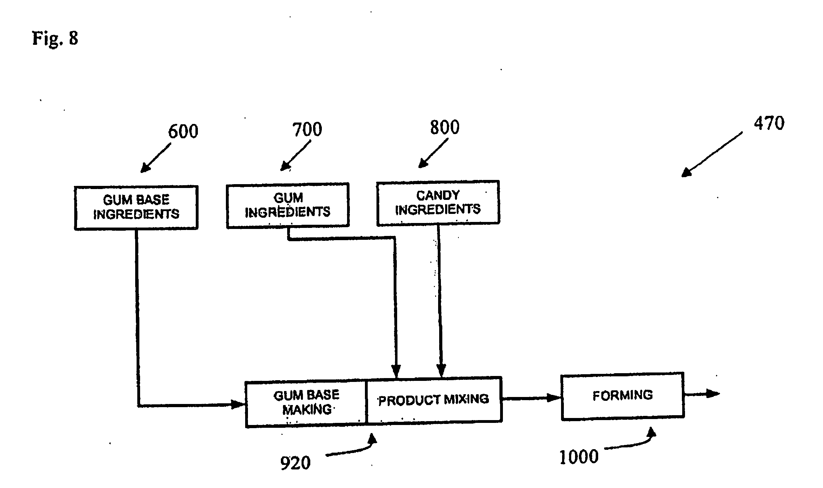

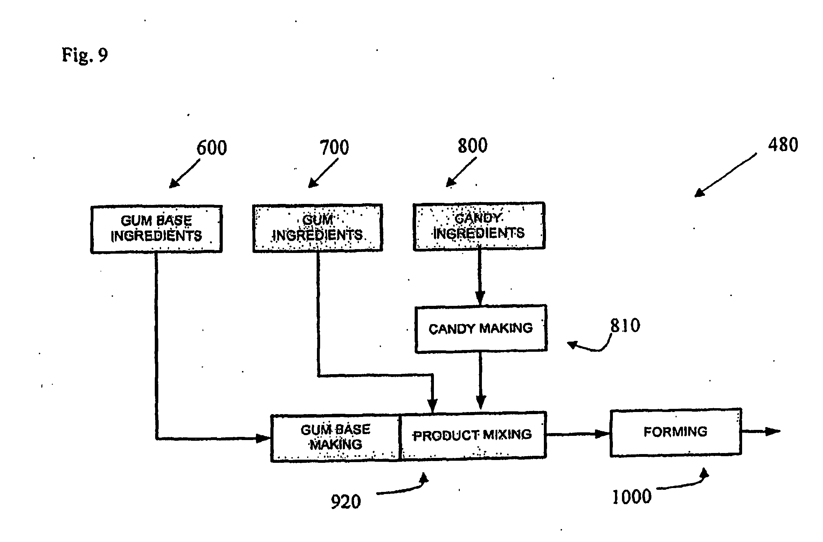

In the embodiment depicted in Fig. 8 (470), the preparation of all portions of the confectionery composition are prepared in a substantially continuous manner, either in a single mixing device or a devices set up in series. In an alternative embodiment, a batch mixer can be used whereby different ingredients are added to the batch mixer over time and distinct mixing operations can be used. In this example, the gum base ingredients (600) are fed to the mixing process (920) and compounded. Thereafter, if used, the chewing gum ingredients (700) and candy ingredients (800) are fed downstream or at a later time point compared to the gum base ingredients. In one embodiment, the candy ingredients are added to the mixing process prior to the chewing gum ingredients. In another embodiment (e.g., Fig. 9, 480), the candy is made (810) prior to its addition to the mixing process (920) for compounding with gum base and chewing gum ingredients (700), if used. Subsequent manipulation steps, such as forming, can then be carried out (1000).

In certain embodiments, the processing equipment can be monitored and/or controlled automatically. For example, the processing equitement can be coupled to a computerized system which allows the user to input certain and/or all of the operational parameters, including, e.g., feeding of the ingredients, mixing or processing the ingredients, conveying the ingredients. In certain embodiments, the system can be coupled to batch processing equipment, continuous processing equipment, or both if both types are used. In some embodiments, changing the input parameters used to control the processing can create changes in the final product as discussed hereinabove, e.g., texture, hardness, crunch, shine, etc. For example, the ingredient and/or processing temperatures and/or feed rates of the ingredients can be monitored and fed back to a central unit so that the operator can adjust as needed and/or in which the system can automatically adjust. After the ingredients have been mixed, the formation, processing (e.g., dusting, coating, and/or packaging of the mixed ingredients into a particular shape and/or form can also be monitored and fed back for the operators input and/or automatic adjustment. An audible and/or visual alarm can also be implemented to signal the operator when the system detects a problem and/or a variation in one or more processing parameters. Each of the mixing apparatus 610, 710, 720, 750, 810, 900, 910, and/or 920 in Fig. 1-14 can be a batch mixer or a continuous mixing apparatus. For example, in some embodiments, some of the mixers 610, 710, 720, 750, 810, 900, 910, and/or 920 in Fig. 1-4 may be continuous mixers and some of the mixers 610, 710, 720, 750, 810, 900, 910, and/or 920 in Fig. 1-4 may be batch mixers. In some embodiments, all continuous mixers might be used while in other embodiments all batch mixers might be used.

In certain embodiments where a batch mixer, mixing temperature of about 140°C and 50 psig can inhibit mixer induced sugar graining.

In some embodiments, candy gum can be prone to deformation "shrinkage" of the finished yet fluid formed pieces. Deformation continues until candy has cooled and solidified or the memory contained within the confection has been adequately relieved via deformation. Therefore, in some embodiments, the level of gum base can be decreased to reduce the degree of deformation. In another embodiment, the product can be grained or slightly grained prior to forming creates a rigid structure less prone to distortion post forming. In another embodiment, a relaxation table can be used after rope sizing and prior to final forming. In another embodiment, under sizing the final rope (making smaller in diameter than required) prior to the final forming and rope sizing permits product to relax slightly and minimizes final compression in forming thereby reducing shrinkage. In yet further embodiments, rope sizers in which the rope is permitted to relax slightly between stages and/or wse of an extruder in which the rope is sized to near ideal proportions coming out of the nozzle in which distortion has already been accounted. In another embodiment, the candy gum can be rapidly set after forming through use of cryogenic cooling and/or cool the outer portion of the candy gum whereby the higher the jacket percentage, the lower the deformation.

In some embodiments, during tempering small thin and fibrous "wisps" fly out of the mass. Over a prolonged period this can lead to a build-up on processing equipment which is difficult to clean off due to the gum content. Therefore, in one embodiment, the resin content of the gum base can be reduced to help minimize "wisping" via reducing the sticky nature of the gum base at temperatures above 50°C thereby reducing the likelihood to adhere to the mechanical or manual devices and pull away from the bulk mass during processing. In another embodiment, the amount of work induced into the saccharide portion during tempering can be reduced (e.g. minimizing turning and kneading) via utilizing a cooling drum or cooling belt to uniformly temper the product will minimize this effect.

In some embodiments, sticking of the mass can cause problems in terms of processing. Therefore, in one embodiment, the level of gum base can be reduced to modify the glass transition point of the gum base to more closely resemble that of the candy thereby decreasing the fluidity of the gum base and hence it propensity to adhere to surfaces during forming. In another embodiment, the glass transition point of the candy base to more closely resemble that of the gum base will decrease the fluidity of the gum base and hence it propensity to adhere to surfaces during forming. In one embodiment, this can be accomplished via altering the sugar / glucose ratio, switching to a high maltose glucose having a lower processing viscosity, adding sugar free components (0 - 100%) in the base candy. In another embodiment, release agents (Ket Lub, Mineral oil etc...) as discussed herein above can be used. In another embodiment, drop rolls can be used as they are typically the most rugged in terms of minimal adhesion due to the inherent nature of water cooled rolls (surface cooling) and release. In another embodiment, coating all or a part of the candy gum with a hard candy shell minimizes and/or removes the potential for the gum content to contact any mechanical surfaces. In another embodiment, the contact surfaces of the processing equipment can be cooled directly or indirectly to minimize sticking.

In some embodiments, the confectionery may become grained and suffer a loss of elasticity (becoming short texture) during forming and loss of crunch in its final state. Therefore, in one embodiment, sugars, such as glucose can be increased to reduce the propensity to grain. In another embodiment, final moisture content can be increased to reduce the propensity to grain. In another embodiment, the sugar-free component can be increased to reduce the propensity to grain. In another embodiment, gum base content can be reduced to decreases the propensity to grain during processing. In another embodiment, mechanical mixing speeds and/or shear can be reduced to reduce propensity to grain. In another embodiment, mix times can be reduced to reduce propensity to grain. In another embodiment, heated side walls and mixer blades can be used to reduce propensity to grain. In another embodiment, mixing saccharide portion and gum base portion at temperatures close to a temperature at which the saccharide portion is cooked, if done, can reduce the degree of sugar super-saturation and hence the propensity to grain. In addition, the longer residence time typical in a static mixer coupled with low shear mixing may reduce graining and/or allow more thorough mixing of some ingredients (e.g., mixing of flavor with elastomers or gum base). In another embodiment" utilizing heated static mixers reduces the propensity to grain. In another embodiment, minimizing or avoiding the pulling of the crunchy gum mass and or over tempering reduces likelihood of graining. In another embodiment, aeration, graining, pulling, rope sizing and forming can be reduced to increase finished product gloss, particular in those embodiments where an extruder is used for processing.

In some embodiments, to controlling the degree of crunch, the gum base levels can be adjusted whereby in some embodiments, the higher the gum-base content, the less hardness and perceived crunch of the final product. In another embodiment, the work imparted into the candy through tempering (kneading, aerating, folding etc) can be reduced to enhance the degree of crunch.

### Center fill

The confectionery compositions with center-fill may be formed by any technique known in the art which includes the method described by U.S. Patent No. 6,280,780 to Degady et al. ("Degady") which is hereby incorporated in its entirety for all purposes. Degady describes an apparatus and method for forming center-filled confectionery pellets. The method includes first extruding a liquid-filled rope of a confectionery layer and passing the rope through a sizing mechanism including a series of pairs of pulley-shaped roller members. The roller members "size" the rope or strand of confectionery material such that it leaves the series of rollers with the desired size and shape for entering a tablet-forming mechanism.

The rope is then led into a tablet-forming mechanism including a pair of rotating chain die members which are endless chain mechanisms and both rotate at the same speed by a motor and gear mechanism. Each of the chain mechanisms include a plurality of open curved die groove members which mate and form die cavities in which the pieces of confectionery composition material (pellets or tablets) are formed. While Degady discusses the formation of pellet or tablet shaped pieces, the confectionery pieces may be of other shapes as described above. The shape of the die groove members may be altered to provide any desired shape and chain or rotary dies may be used.

The confectionery composition may optionally be passed through a cooling tunnel either before entering the tablet-forming mechanism, after exiting the tablet-forming mechanism or both. Cooling of the rope prior to entering the tablet-forming mechanism may be beneficial to prevent rebound of the individual pieces and thus may provide an increase in productivity.

The cooled pieces of confectionery composition material can then be fed into a storage container for conditioning and further processing. At this point, the cooled pieces of confectionery material could also be fed directly into a coating tunnel mechanism, such as a rotating tunnel mechanism.

The center-fill can be liquid, semi-solid, powdery or particulate, solid or gaseous. Combinations of these in a single center-fill pocket as well as two or more separate pockets within the confectionery can also be employed.

As discussed herein, center edible cores in the confectioneries of the present invention can be increased substantially compared to traditional center fill chewing gums. In some embodiments, a confectionery composition with a liquid center fill has a softer initial texture and requires less energy to bite through than a confectionery composition without a liquid center fill. In one aspect of the invention, the center fill is present in an amount of at least 15% by weight of the total confectionery product. In other embodiments, the center-fill is present in an amount up to about 40% by weight. Inclusive are 16, 17, 18, 19, 20, 21, 22, 23, 24, 25, 26, 27, 28, 29, 30, 31, 32, 33, 34, 35, 36, 37, 38, 39 and all values and subranges therebetween.

### Granulation

The granulated confectionery of the present invention has, in addition, to its ease of manufacture, the ability to form a pleasing chewing experience upon consumption of the product. In other words, the confectionery when consumed in granular form and masticated, comes together to form a gum bolus on which the consumer can chew like a piece of chewing gum. This is particularly beneficial to consumers who seek novel experiences when consuming confectioneries.

In another embodiment, the granulated confectionery as described herein can be used as a filling of another similar confectionery that has not been granulated, filling in a chewing gum or chewing gum base, and/or filling in a soft, chewy or hard candy mass. Combinations of these are also envisioned.

In one embodiment, the granulated confectionery can be used to coat a similar confectionery that has not been granulated, filling in a chewing gum or chewing gum base, and/or filling in a soft, chewy or hard candy mass. Combinations of these are also envisioned.

In one embodiment, the granulated confectionery can be layered between layers of similar confectionery that has not been granulated, filling in a chewing gum or chewing gum base, and/or filling in a soft, chewy or hard candy mass. Combinations of these are also envisioned.

In one embodiment, the granulated confectionery can be used to decorate the surface and/or partially incorporate into similar confectionery that has not been granulated, filling in a chewing gum or chewing gum base, and/or filling in a soft, chewy or hard candy mass. Combinations of these are also envisioned.

In one embodiment, the granulated confectionery in addition to providing textural benefits to the consumer by itself and/or when combined with similar confectionery that has not been granulated, filling in a chewing gum or chewing gum base, and/or filling in a soft, chewy or hard candy mass as discussed above, can be used a vehicle for delivering various ingredients to the consumer, e.g., flavors, sweeteners (including high-intensity sweeteners), functional actives, cooling agents, food acids, warming agents, and/or to provide dualities and/or multi-modalities as described herein.

Granulating the confectionery can be performed by any conventional methods used in the field, for example, a grinder can be used. In one embodiment, after mixing the saccharide portion and the gum base portion, the confectionery is formed into strands which are ground in a Fitz mill under ambient conditions. The ground confectionery can then be dry mixed with additional ingredients and/or used as a further component in other confectioneries, e.g., as a coating, center fill, and other as described herein.

In certain embodiments, the confectionery is granulated to a size of from 1 to 3000 micron.

### Introduction of Gas

As discussed herein, one aspect of the invention is that a confectionery in which chewing gum base is mixed with a saccharide can be gasified in a manner that is traditionally used for hard candies and provide the additional advantage of providing a pleasing chewing experience coupled with the sensation of gas release from the matrix of the confectionery.

In one embodiment, candy gum when prepared under controlled condition exhibits properties of candy rather than chewing gum, is plastic at high temperature and can be cooled and quickly harden. This physical change from plastic state to glass transition stage and can be usedto trap gases. In one embodiment, the components of the candy gum is mixed at 120-140 °C is then gasified and cooled to trap gas.

In one embodiment, the gas is carbon dioxide. In another embodiment, the gas is a mixture of gas, including carbon dioxide. In certain aspects of the invention, the amount of gas incorporated into the confection is from 0.5 to 15 ml per gram of saccharide included in the saccharide component. This range includes 1, 2, 3, 4, 5, 6, 7, 8, 9, 10, 11, 12, 13, 14 and all values and subranges there between.

In one embodiment, the confection contains a sugar component, which becomes amorphous after it is cooked to its desired temperature and prior to the introduction of gas.

In one embodiment, the gas is introduced into the confection by gasifying the saccharide portion prior to its mixing with the chewing gum base. In one aspect of this embodiment, the saccharide portion is gasified after it is reached the hard ball stage (e.g., about 250 °F).

In another embodiment, the confection is gasified by introducing gas while the candy portion and the gum base portion are being mixed together. In another embodiment, the confection is gasified after the candy and the gum base portions are mixed.

Combinations of the gasifying embodiments are also envisioned. For example, in some instances it may be advantageous to introduce gas after the candy portion has been made, while the candy portion is being mixed with the gum base portion, and or after the two portions have been mixed.

The introduction of gas or gases into a confection, such as hard candies is known. In this regard, reference is made to U.S. patent nos. 3,012,893; 4,150,161; 3,985,909; and 3,985,910, all of which are incorporated herein by reference. For example, after the saccharide has been melted, and perhaps combined with other ingredients in the confection, the melted mass is contacted with gas, preferably under pressure (e.g., 50 to 1000 psi) and which may also include agitation, mixing caused by a batch mixer and/or in an extruder (continuous or discontinuous). Thereafter the gasified mass is cooled to allow solidification and entrapment of the gas.

### Shaping/Formation

The compositions described herein, that is including a saccharide portion, are formed into one or more shapes regular and or irregular shapes, such as rings, rods, canes, wedges, ropes, twists, rocks, stones, jewelry, caricatures of real or animated animals, people and the like. The confectioneries can be formed into traditional chewing gum formats such as slabs, pellets, balls, and the like. In some embodiments, a surface of the traditional formats can have a smooth or rough surface optionally with a concave, convex, embossing, debossing or dimpling feature.

The confectioneries described herein can be shaped using devices and techniques that are known in the candy art. For example, the confectioneries can be shaped by hand, with molds and/or dies, cutters, pullers, stretchers, extruders and the like. Non-limiting examples of molds that can be used include, flexible (e.g., made of silicone), metal, plastic, glass, impressions made in powder materials such as starch, and the packaging itself (e.g., by placing the unshapened mass into the pack), such as a blister pack. Combinations of these may also be employed.

The shaped confectioneries can be the combination of saccharide portions with gum base portions as well as in embodiments where the shaped confectionery comprises a center fill, the shaped confectionery is a coated, the shaped confectionery itself is incorporated into another confectionery product in whole and/or after it has been granulated, e.g., to a mesh size of 20 to 200. In other embodiments, the shaped confectionery can take the form of a layered confectionery which is composed of two or more layers of different confectioneries of saccharide portions with gum base portions alone (e.g., different flavors, colors and/or other ingredients as well as different forms, e.g., solid, soft, chewy, granulated, etc.) and/or in combination with other confectionery products such as chewing gums, gum bases, soft candies, chewy candies and/or hard candies. Thus, in one embodiment, the layered confectioneries including at least confectioneries of saccharide portions with gum base portions are shaped. In another embodiment, the confectioneries can take the form of honeycombed products where the confectioneries of saccharide portions with gum base portions are part of the center, the jacket or both. In embodiments, where confectioneries of saccharide portions with gum base portions comprise both portions of the honeycomb product, they can be the same or different, such as by the inclusion of different flavors, colorants and/or other ingredients as described herein.

The formation of layered or honey combed confectioneries is known in the art, e.g., U.S. patent nos. 5,017,385 and 4,648,316; Sugar Confectionery Manufacture, 2nd Ed. E.B. Jackson (ed.), section 7.5.1 "Laminated or honey-combed sweets" Blackie Academic & Professional; and 42nd P.M.C.A. Production Conference, 1988 "Updated Technologies in Honey-Combing."

### Coating

Whether the pieces of formed confecterionery material are first stored, transported in a storage container, or fed directly into a coating tunnel or mechanism, the individual pieces of confectionery material may subsequently be subjected to a conventional sugar or sugarless coating process in order to form a hard exterior shell on the confectionery composition material. A variety of coating processes or mechanisms of this type are known. In some embodiments, the coating is applied in numerous thin layers of material in order to form an appropriate uniform coated and finished quality surface on the confectionery products. The hard coating material, which may include sugar, maltitol, erythritol, isomalt, sorbitol or any other polyol, including those described herein, and optionally flavoring, is sprayed onto the pellets of confectionery composition material as they pass through a coating mechanism or a coating tunnel and are tumbled and rotated therein. In addition, conditioned air is circulated or forced into the coating tunnel or mechanism in order to dry each of the successive coating layers on the formed products. In some embodiments, the coating, or outermost portion, can be formed by lamination, dual or multiple extrusion, or any other process that creates an outermost portion.

Enrobing and or Coating can be used to enhance the appearance, provide textural interest, separate components, act as a protective barrier, decorate the product, or contribute to flavor and flavor release of the confectionery compositions containing an elastomeric component and a saccharide component. The process generally depends on the properties of the coating to be used, as well as the geometry and structure of the product over which it will be applied. Rounded pieces that can tumble can be panned or tumble coated in a rotating vessel. Flatter pieces would preferably be processed in an enrobing device, which would also preferably include a device to apply coating on the bottom face of the product, concurrently or separately to a falling curtain of the coating, which can then be subjected to vibration, e.g., shaking, to eliminate an excess of the coating and control the desired weight of coating. In one embodiment of decorating the product ink jet printing technology can be employed

The coatings can be applied only once, as a single layer, or as a succession of layers if so desired. In the particular case of chocolate coatings, tumble coating has the advantage that the chocolate does not need to be pre-tempered, the tumbling itself tempers the chocolate, and also that the equipment needed will be less complex and cost less than an chocolate enrober with an outboard or an inboard tempering unit.

Enrobing applies a thin layer of coating over the confectionery composition, by placing it on a perforated belt or mesh that goes through a bottomer. The bottomer can be a devise that creates a trough of the coating, such that as the mesh moves the core across it, the coating sticks to the bottom of the piece. The piece is then shaken to eliminate the excess coating and, if desired or necessary, can be run through a cooling step to set or solidify the bottom coating.

Following the bottoming and/or concurrently therewith, the core on the conveyor mesh is conveyed through an enrober, which as typically used in this field is a curtain of the fluid coating. As the core crosses the curtain, the coating sticks to the top and sides. The excess coating can be blown off with air, or by applying vibration and shaking the candy pieces. This helps to remove air bubbles and create a uniform coating of the correct weight. The coating can then be set or solidified by running it through the cooling operation.

A decoration can also be applied to the top, bottom, sides, or all of these, preferably prior to the coating being set or cooled. The decoration can also be imparted onto the product during the enrobing process itself, e.g., swirls, peaks, streaks, stripes, crosses, webs, etc.

In an alternate procedure, the coating can be applied sprayed onto the confectionery piece, e.g., using a nozzle sprayer. In this case, setting or cooling may not be required.

The enrobing process can result in a complete coating, i.e. covering all surfaces of the product, or an incomplete coating, e.g., covering the top only.

An alternative process for coating is tumble coating. Tumble coating is suitable for products that are rounder and can tumble. It is also useful for smaller pieces that may not process well with an enrober. The products can be conditioned or prepared to be tumbled. This preparation can include, but not limited to, a cooling step or a priming step to ensure the coating sets when it is applied to the product as it tumbles in a revolving pan or coating drum. The coating is usually applied with the help of a nozzle, to create droplets of the coating that then stick to the cores, spread, and set with or without the aid of cool air. The revolving action with the continuous or semi-continuous application of the coating creates a uniform layer or layers of the coating over the product core, and it is applied until the desired amount is added. The process can be batch or continuous.

One variation of this method can be used when a thin layer of hard candy or another sticky, hard to apply coating is to be applied over the product core. In this case, the coating is placed under the right temperature conditions and desired ratio on a vessel or heated screw conveyor, and the actual product core is conveyed by an agitator element in the vessel or conveyor. This folding action is gentler when compared to a more vigorous tumbling enables the distribution of a thin coating of the material over the product core.

These types of coating process as known in the field are described in, for example, Enrobing: Enrobing Technology, by M.S. Jeffery (Cadbury USA) The Manufacturing Confectioner, June 1988, p87; Enrobing: Cooling Tunnels, by Kurt Muntener (Richard Frisse GmbH) The Manufacturing Confectioner, June 1988, p91; and Enrobing: Coating Machines and Bottomers, by Heinz Schremmer (Sollich GmbH & Co. KG) The Manufacturing Confectioner, June 1988, p99.

The coating can be present in an amount of from 0.5% by weight and higher, e.g., at least 50% by weight, inclusive of 5%, 10%, 20%, 30%, 50%, 100%, 150% by weight of the total product; and in some embodiments even higher owing to the unique properties of the combination of the gum base and the saccharide component.

In addition to forming a confectionery composition product, in some embodiments, the confectionery compositions as described herein can become components of other compositions. For example, in some embodiments, a confectionery composition including a saccharide portion and an elastomeric portion can become one of a plurality of layers in a confectionery product.

The consumer-preferred qualities provided by the candy gum may be marketed to consumers in a variety of manners. Suitable marketing strategies, include, for example, print, radio, satellite radio, television, movie theater and online advertising campaigns, point-of-purchase advertisements, billboard advertisements, public transportation and telephone booth advertisements, indicia on the product packaging (e.g., slogans, trademarks, terms and colors), labels, images, graphics, descriptive wording and colors) instant messaging, ringtones, and the like.

The package assembly of the present invention may be used to market and reinforce the candy gum product to consumers. In one preferred embodiment, the package assembly of the present invention includes indicia e.g., descriptive matter such as words, phrases, slogans, pictures, symbols, contests, marketing campaigns, textures, colors, intensities, or other characteristics or features, that are be used to relate information to consumers concerning the product.

Several advantages may be derived by providing indicia on the package assembly. For example, a package assembly having indicia may provide notice to consumers that a specific product of known quality, aroma and taste, is available. The indicia also may provide consumers with a latent image of the product and reinforce consumer image of the product.

In one preferred embodiment, the indicia extends over a portion of an outer surface of the package assembly. By providing the indicia on the package, the package assembly of the present invention may be utilized as a marketing tool. For example, a consumer viewing the product package may be immediately informed of the multi-modality of the product, such as a first tastant and second tastant, contained in the product.

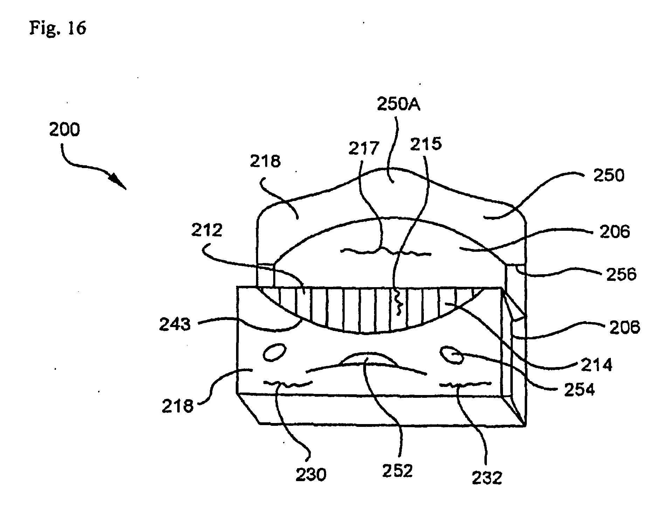

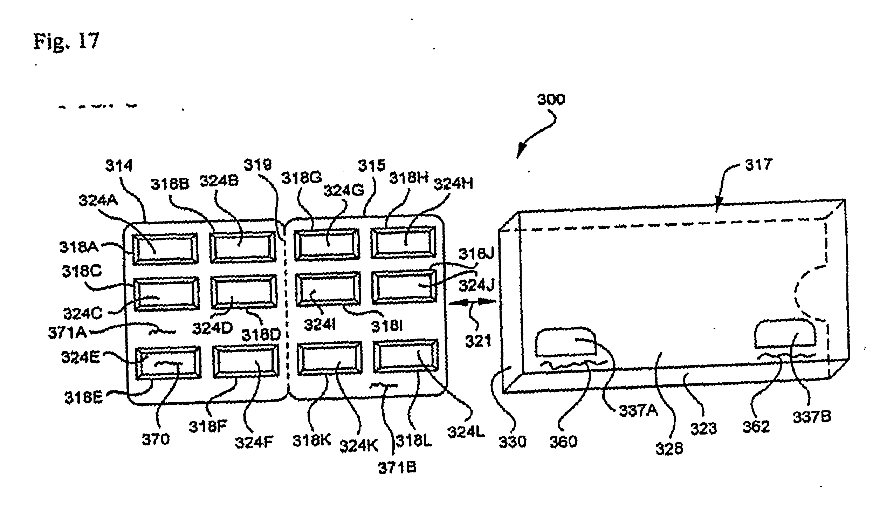

The present invention, however, is not limited to the indicia being included on the outer surface of the assembly. For example, indicia also may be included on an overwrap for a package (as opposed to or in addition to the package assembly itself) as discussed in connection with FIG. 16, as well as printed on the product itself, discussed in connection with FIG. 17. In one preferred embodiment, as shown and discussed in connection with FIG. 16, indicia also may be provided on the surfaces of one or more wrappers used to protect and store the candy gum product in the assembly. Although the wrapper is provided with indicia of which at least some may be substantially the same as that on the outer surface of the assembly, the wrapper indicia also may include further information relating to, say, a contest or promotion of the product contained in the assembly. The contest or promotion may include a sweepstake, prize, token or redeemable voucher.

The communication of the indicia to consumers can be done through a number of media outlets and includes such things as printed indicia on product packages, printed messages in magazines, newspapers, newsletters and the like, audio and visual outlets such as radio and television as well as other outlets, such as the Internet, that are suitable for use in delivering the message to a targeted demographic.

Various marketing techniques also may be developed using the indicia of the present invention. For example, an advertisement for the product may be provided that utilizes at least a portion of the indicia included on the package. In one preferred embodiment, for example, the advertisement takes the form of an audio message that is played upon the user removing the product from the packaging.

In another embodiment, a display device, is configured to support the package assembly and thereby, show at least a portion of the indicia indicating the advantages, e.g., the multi-modality, of the product contained therein. For example, in one preferred embodiment, a display stand is provided for displaying and supporting the package assembly. The display stand may be used in a retail establishment at the point of purchase such as the cash register. The display stand may be formed from transparent plastic to allow clear viewing of the indicia included on the package assembly.

Other marketing techniques also may be developed to generate sales of the product. For example, in some preferred embodiments, audio messages are created that recite indicia (e.g., slogans, jingles) included on the product package. This may include a method of publishing audio files to the Internet, and allowing consumers to subscribe to a feed to receive the audio files containing the indicia. Visual images of the product also may be prepared that include at least a portion of the indicia included on the product package. For example live or delayed sound or video broadcasts of the indicia may be provided to consumers using web technologies over the Internet. Radio and television commercials also may be used to market the product to consumers using at least a portion of the indicia, and thereby increase revenue. Of course, it is to be understood that the invention is not limited to the above referenced techniques and that various changes and modifications to the techniques may be affected herein by one skilled in the art without departing from the scope or spirit of the invention. It is also to be understood and that it is intended to claim all such changes and modifications that fall within the scope of the invention.

Various packaging assemblies known in the art may be adapted to incorporate features of the present invention. For example, as disclosed in U.S. Patent Number 2,279,471 to Wilson, which is incorporated herein in its entirety, a box is disclosed that may support chocolate and other confectionery products that may be adapted to support the gum compositions and provide the indicia of the present invention. U.S. Design Patent Numbers D398,520 and D392,885, which are incorporated herein in their entirety, also disclose a box-type packaging that can be adapted to support and provide the gum compositions and the indicia of the present invention, respectively.

Gum stick packaging known in the art also may be adapted to incorporate the above described indicia and support the multi-modality candy gum product of the present invention. For example, in U.S. Patent 6,926,951 to Huffer et. al., which is incorporated herein in its entirety, a laminate for gum packaging is disclosed that may include the indicia of the present invention. In addition, U.S. Patent 5,029,712 to O'Brien et. al., which is incorporated herein in its entirety, discloses a reclosable package for holding items having an adhesive front label applied to the front portion of the container. The reclosable package may be used to support the gum compositions of the present invention. Indicia of the present invention also may be provided by the reclosable package. Chewing gum packages, such as those disclosed in U.S. Patent No. 2,192,472 and U.S. Patent No. 2,192,473, which are incorporated herein in their entirety, also may be adapted to provide the indicia and support the candy gum product of the present invention.

The present invention may be applied to packaging tins used for supporting confectionery products. For example, in U.S. Design Patent No. D480,561 to Simon et. al., which is incorporated herein in its entirety, a case for a chewing gum packet is disclosed that can be adapted to support the gum compositions and provide the indicia of the present invention. U.S. Design Patent No. D471, 804 to Staples, which is incorporated herein in its entirety, discloses a chewing gum tin that also may be used to support the gum compositions and provide the indicia of the present invention. In addition, U.S. Design Patent No. D457,427 to Diaz, which is incorporated herein in its entirety, discloses a combined chewing gum box and clip that may be used to support the gum compositions and provide the indicia of the present invention. Other tin packaging assemblies that may be adapted to support the multi-modality candy gum product and include indicia of the present invention include U.S. Design Patent D412,279, U.S. Design Patent No. D406,496, U.S. Design Patent No. D35 1,789 and U.S. Design Patent No. D449,782, all of which are incorporated herein in their entirety.

Dispensors also may be used to support the multi-modality candy gum product and provide the indicia of the present invention. For example, U.S. Patent Number 5,540,353 to Coleman et. al., which is incorporated herein in its entirety, discloses a candy container and dispenser that includes a housing having a top enclosure with an aperture, wherein pieces of candy having a size smaller than the aperture may be dispensed by shaking. The candy container and dispenser may be adapted to house the candy gum product and provide the indicia of the present invention. U.S. Patent Number 5,370,219 to Violett, which is incorporated herein in its entirety, discloses containers for the storage and transportation of sticks of gum that may be adapted to support the chewing gum and provide the indicia of the present invention. U.S. Patent Number 5,056,683 to O'Brien et. al., which is incorporated herein in its entirety, discloses a cardboard stick gum dispenser that may be adapted to support the multi-modality chewing gum and indicia of the present invention. Similarly, other dispensor packages may be used with the present invention. For example, U.S. Patent Number 4,465,208 to Buban et al., which is incorporated herein in its entirety, discloses a chewing gum dispenser having an upper cover whose central region is cut away to afford access to a stick product. The chewing gum dispenser of Buban et. al. may be adapted to provide the indicia and support the candy gum product of the present invention. Similarly, the dispensors disclosed in U.S. Patent Nos. 4.170,914 and 3,591,043, the contents of which are incorporated herein in their entirety, also may be adapted to support the multi-modality chewing gum and to provide the indicia of the present invention.

Of course, the present invention is not limited to the above identified package assemblies and various other types of package assemblies may also be used to support the multi- modality candy gum of the present invention. For example, tablet packs, as disclosed in U.S. Design Patent No. D344,018 to Kelsey et. al., which is incorporated herein in its entirety, may be adapted to provide the indicia and support the product of the present invention. The stacked articles disclosed in U.S. Patent No. 3,591,043 to Murphy also may be adapted to support the candy gum product and provide the indicia of the present invention.

Sealed packets also may be used to support the multi-modality candy gum product and provide the indicia of the present invention. For example, as disclosed in U.S. Patent 4,874,096 to Tessera-Chiesa, which is incorporated herein in its entirety, a sealed packet containing food products in pieces, particularly sweets and the like, is disclosed that can be adapted to provide the candy gum product and indicia of the present invention. Furthermore, combination packages, such as the apparatus for carrying gum and mints disclosed in U.S. Patent No. 6,655,488, which is incorporated herein in its entirety, may be used to support the candy gum product and provide the indicia of the present invention and overwrapping packages, such as those disclosed in U.S. Patent No. 2,571,516 and U.S. Design Patent No. D383,973, which are incorporated herein in their entirety, may be adapted to support the multi-modality candy gum product and provide the indicia of the present invention.

Reference will now be made to the accompanying drawings, which further assist in illustrating the various pertinent features of the packaging assembly of the present invention. Although the invention will now be described primarily in conjunction with gum packaging, it should be expressly understood that the invention may be applicable to other applications where multiple separable compartments, each for one or more removable objects, is required/desired. In this regard, the following description of a gum packaging assembly is presented for purposes of illustration and description. Furthermore, the descriptions are not intended to limit the invention to the forms disclosed herein. Consequently, variations and modifications commensurate with the following teachings, and skill and knowledge of the relevant art, are within the scope of the packaging assemblies.

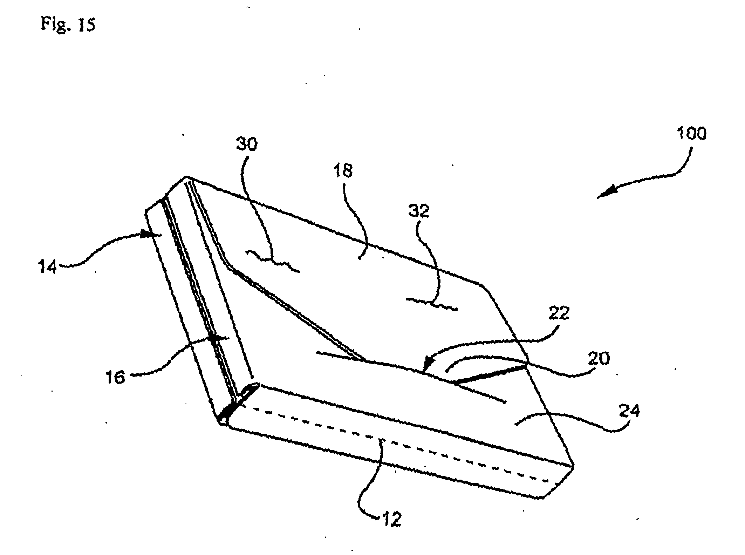

Referring now to FIG. 15, a first embodiment of a package assembly 100 according to the present invention is disclosed. The package assembly 100 includes an upper compartment 14 and a lower compartment 16 that are used to support multi-modality chewing gum compositions. The upper and lower compartments 14, 16 may be easily separated from one another along a perforation 12. As shown in FIG. 15, the compartments 14 and 16 are typically provided in an attached manner and are folded together to place one facing the other. A cover flap 18 is provided from the upper compartment 14 having an end 20 laid over a receiving slot 22 in a back panel 24 of the lower compartment 16 to close the assembly 100. The consumer can tuck the end 20 into the slot 22 to form a compact package 26. Details of forming the package assembly 100 of FIG. 15 are described in U.S. Patent Application Ser. No 10/883,468, filed on July 1, 2004, which is incorporated herein in its entirety.

As shown in FIG. 15, each of the compartments includes indicia 30, 32 that are indicative of the duality provided by the product contained therein. The location of the indicia 30, 32 may vary based on design considerations as well as functional considerations decided upon to effectively market the product. For example, in one preferred embodiment, one of the indicia 30 is included on one of upper compartment 14 and the other indicia 32 is included on the lower compartment 16. The indicia 30, 32 may be indicative of the flavors, sensations, tastes, functionalities, or other characteristics or benefits provided by the gum compositions contained in the compartments that are complementary to each other, opposed to each other, i.e., distinct, or different in intensity from each other. For example, in one preferred embodiment, one indicia may indicate a sweet flavor gum composition accessible from the upper compartment 14 and another indicia 32 indicate a sour flavor gum composition accessible from the lower compartment 16. Of course, as mentioned previously, the indicia may be any words, phrases, slogans, pictures, symbols, contests, marketing campaigns, textures, colors, intensities, or other characteristics or features relating to the candy gum product.

Referring now to FIG. 16, a second embodiment of a package assembly 200 according to the present invention is disclosed. As shown in FIG. 16, when the consumer opens the assembly 200 by pulling up the tab 250a of a flap 250, a foil portion of a packet 206, e.g., an overwrap, supporting the multi-modality chewing gum compositions of the present invention 10 tears along a scoring 243 leaving an array 212 of filled-wrappers of gum 214. The wrappers 214 protect and store the candy gum product in the assembly 200. As shown in FIG. 16, in one preferred embodiment, for example, one or more of the gum wrappers 214 include indicia 215 that may be used to provide further information relating to the product, or a contest or promotion relating to the product. Once the foil portion of the packet 206 tears, the consumer may pull out as many gum slabs 214 as needed. In some preferred embodiments, as shown in FIG. 16, the foil portion of the packet 206 also may contain indicia 217 relating to the product, such as a freshness data, as well as contest or promotional information relating to the candy gum product. Finally, the consumer may simply pull the flap 250 downward and tuck the tab 250a into a slot 252 to close the package assembly 200. Details of forming the package assembly 200 of FIG. 16 are described in U.S. Patent Application Ser. No. 10/001,352, filed on October 31, 2001, which is incorporated herein in its entirety.

As shown in FIG. 16, a front wall 218 of the packet also may include indicia 230, 232 that are indicative of the duality provided by the gum composition included therein. Similar to the indicia 30, 32 described previously in connection with FIG. 15, the indicia 230, 232 may be indicative of the flavors, sensations, tastes, functionalities, or other characteristics or benefits provided by the gum compositions. The location and number of indicia 230, 232 included in the package assembly 200 also may vary depending upon the marketing strategy chosen. For example, in some preferred embodiments, indicia are also included on the flap 250 of the assembly and are visible when the assembly 200 is opened.

A third embodiment of a product package assembly 300 of the present invention is shown in FIG. 17. The package assembly includes a sleeve 317 having a uniform shape. The assembly 300 also includes one or more package or blister trays 314, 315 that are removable from and reinsertable into the sleeve 317. For example, as shown by the arrow 321 in FIG. 17, a consumer may slidably remove blister trays 314, 315 from the sleeve 317 and slidably reinsert the blister trays 314,315 into the sleeve 317 as desired. In a preferred embodiment, for example, each blister tray 314,315 includes indicia 371a, 371b that provides information to consumers relating to the packaging date of the assembly. Of course, as mentioned previously, other indicia relating to the product may be provided thereto. Preferably, the blister trays 314, 315 of the present invention are made primarily from plastic and/or plastic or metal foils. As shown in FIG. 17, each tray 314, 315 may be attached to each other via a perforated line 319 that allows the trays 314,315 to be separated from each other. For example, the blister trays 314, 315 may be easily separated from each other by tearing along the perforated line 319.

As shown in FIG. 17, each of the trays 314, 315 includes a plurality of compartments 31 8a-f, 31 8e-1, respectively, that extend outwardly from each tray 314, 315. The compartments 3 18a-1 support the multi-modality chewing gum compositions of the present invention. For example, one blister tray 314 may include a sweet flavor gum composition 324a- f, and one blister tray 315 may include a sour flavor gum composition 324g-1. The front wall 328 of the sleeve 317 includes indicia 360, 362 that are indicative of the multi-modality of the candy gum product supported therein. The indicia 360, 362 may indicate the flavors, sensations, tastes, functionalities, or other characteristics or benefits provided by the gum compositions included in the blister trays 314, 315. As shown in FIG. 17, in one preferred embodiment, the top wall 328 of the sleeve 317 includes openings 337a, 337b that allow for displaying the multi-modality chewing gum compositions when one or more trays 314, 315 are inserted into the sleeve 317. For example, in one preferred embodiment, a sweet flavor candy gum composition 324e is displayed through opening 337a and a sour flavor gum composition 324L is displayed through opening 337b. Indicia 360, 362 indicating "Sweet" and "Sour" flavors, respectively, are located adjacent to the openings 337a, 337b and thereby, are indicative of the flavors. Of course, the indicia 360, 362 also inform the consumer of the multi-modality of the candy gum compositions.

As shown in FIG. 17, one or more of the candy gum product may have included therein indicia 370 that may be pre-printed, stamped or etched onto the product. The indicia 370 may include a name, such as a brand name, or other images, symbols, or other descriptive matter that is related to the product or marketing of the product.

### EXAMPLES

Individual confectionery pieces of any of these examples may be optionally center filled with liquid, semi-liquid, or solid fillings and they may be optionally coated. Furthermore, the shape of the confectionery pieces may be chosen from any shape such as ball, pellet, chunk, slab, etc.

### Examples 1.3

**Table 6**

| **Component** | **% w/w** | | |
|---|---|---|---|
| | **1** | **2** | **3** |
| Granulated sugar | 43.40 | 44.50 | 45.20 |
| Glucose Syrup | 35.50 | 36.50 | 37.00 |
| Color | 0.20 | 0.20 | 0.20 |
| Flavor | 1.80 | 1.80 | 1.60 |
| High Intensity Sweetener | | 0.30 | 0.44 |
| Gum Base* | 19.10 | 16.70 | 15.56 |
| **Total** | 100.00 | 100.00 | 100.00 |

| | | | |
|---|---|---|---|
| *Gum Base may include, but is not limited to, elastomer, plasticizer, and filler. | | | |

For Examples 1-3, a saccharide solution is prepared by dissolving the granulated sugar and corn syrup in water. The color is then dissolved in water and a color solution is added to the saccharide solution. Next, the saccharide and color solutions are cooked to 145 °C to form a candy mass. The candy mass is then placed on a cooling table where the flavor is mixed in. The high intensity sweeteners can be added to this candy mass at the same time the flavor is added. Alternatively, the high intensity sweeteners can be added to the gum base component that forms the elastomeric portion. Once the flavor (and possibly the high intensity sweetener) is dispersed in the candy mass, the gum base is heated to 70-90°C and kneaded into the flavored candy mass to form a homogeneous confectionery mass. Lastly, the homogeneous confectionery mass is shaped into finished product pieces. One method of shaping is to pass the confectionery mass through a drop roller to form finished product pieces.

### Examples 4-12

**Table 7**

| **Componen t** | **% w/w** | | | | | | | | |
|---|---|---|---|---|---|---|---|---|---|
| | **4** | **5** | **6** | **7** | **8** | **9** | **10** | **11** | **12** |
| Isomalt | 80.00 | 55.00 | 35.00 | 60.00 | 71.67 | 63.33 | 73.33 | 66.67 | 48.33 |
| Flavor | 2.50 | 2.50 | 2.50 | 2.50 | 2.50 | 2.50 | 2.50 | 2.50 | 2.50 |
| Gum Base* | 15.00 | 40.00 | 40.00 | 15.00 | 23.33 | 31.67 | 15.00 | 15.00 | 40.00 |
| Powdered Isomalt | | | | | | | | | |
| Powdered Sorbitol | | | 20.00 | 20.00 | | | 6.67 | 13.33 | 6.67 |
| Aspartame | 2.00 | 2.00 | 2.00 | 2.00 | 2.00 | 2.00 | 2.00 | 2.00 | 2.00 |
| Acesulfam e-K | 0.50 | 0.50 | 0.50 | 0.50 | 0.50 | 0.50 | 0.50 | 0.50 | 0.50 |
| | | | | | | | | | |
| **Total** | | | | | | | | | |

| | | | | | | | | | |
|---|---|---|---|---|---|---|---|---|---|
| *Gum Base may include, but is not limited to, elastomer, plasticizer, and filler. | | | | | | | | | |

### Examples 13-17

**Table 8**

| **Component** | **% w/w** | | | | |
|---|---|---|---|---|---|
| | **13** | **14** | **15** | **16** | **17** |
| Isomalt | 41.67 | 43.33 | 51.67 | 57.50 | 59.73 |
| Flavor | 2.50 | 2.50 | 2.50 | 2.50 | 1.49 |
| Gum Base* | 40.00 | 31.67 | 23.33 | 27.50 | 22.00 |
| Powdered Isomalt | | | | | 15.00 |
| Powdered Sorbitol | 13.33 | 20.00 | 20.00 | 10.00 | |
| Aspartame | 2.00 | 2.00 | 2.00 | 2.00 | 1.43 |
| Acesulfam e-K | 0.50 | 0.50 | 0.50 | 0.50 | 0.35 |
| | | | | | |
| **Total** | 100.00 | 100.00 | 100.00 | 100.00 | 100.00 |

| | | | | | |
|---|---|---|---|---|---|
| *Gum Base may include, but is not limited to, elastomer, plasticizer, and filler. | | | | | |

For these examples, a saccharide solution is prepared by dissolving the isomalt in water and cooking to 172°C to form a candy mass. Alternatively, the isomalt can be melted by heating to 172°C without water to form a candy mass. Next, the candy mass is placed on a cooling table where the flavor and powdered isomalt or powdered sorbitol are mixed in. The high intensity sweeteners can be added to the candy mass at the same time the flavor is added. Alternatively, the high intensity sweeteners can be added to the gum base component that forms an elastomeric portion. Once the flavor (and possibly the high intensity sweetener) is dispersed in the candy mass, the gum base is heated to 70-90°C and kneaded into the flavored candy mass to form a homogeneous confectionery mass. Lastly, the homogeneous confectionery mass is shaped into finished product pieces. One method of shaping is to pass the confectionery mass through a drop roller to form finished product pieces.

### Example 18

**Table 9**

| **Component** | **% w/w** |
|---|---|
| Granular Sugar | 15.00 - 22.00 |
| Glucose Syrup | 20.00 - 25.00 |
| Gelatin Solution | 3.00 - 6.00 |
| Fat Mixture | 8.00 - 12.00 |
| Fondant | 6.00 - 10.00 |
| Food Acid Blend | 0.75 - 2.50 |
| Flavor | 0.80 - 1.80 |
| Color | 0.01 - 0.10 |
| High Intensity Sweetener | 0.75 - 3.00 |
| Gum Base* | 15.00 - 45.00 |

| | |
|---|---|
| *Gum Base may include, but is not limited to, elastomer, plasticizer, and filler. | |

The examples below demonstrate some embodiments where gum base, chewing gum, or both are added to sugar or sugar-free saccharide components. In some examples, the saccharide component includes a hydrocolloid such as gelatin which can provide a textural benefit.

**Table 10 Examples 19 - 23; Gum Bases**

| | **% w/w** | | | | |
|---|---|---|---|---|---|
| **Ingredient** | **19** | **20** | **21** | **22** | **23** |
| | **High Filler** | **Low Filler** | **Without Butyl Rubber** | **Highly Plasticized (Soft)** | **Less Plasticized (Firm)** |
| **Elastomers (High Molecular Weight)** | | | | | |
| Butyl Rubber | 8 - 12 | 4 - 8 | 0 | 4 - 8 | 6 - 10 |
| Styrene-butadiene Rubber | | | 5 - 11 | | |
| | | | | | |

| **Elastomers (Low Molecular Weight**) | | | | | |
|---|---|---|---|---|---|
| Polyisobutylene | 10 - 20 | 8 - 10 | 0 | 5 - 10 | 10 - 20 |
| | | | | | |

| **Softeners** | | | | | |
|---|---|---|---|---|---|
| Rosin esters | 5 - 30 | 5 -10 | 15 -20 | 20 - 35 | 5 - 10 |
| Waxes | 4 - 10 | 8 - 12 | 5 - 15 | 5 - 10 | 5 - 10 |
| Vegetable oils (hydrogenated) | 10 - 30 | 15 - 25 | 5-15 | 2 - 5 | 20 - 30 |
| | | | | | |

| **Texture Modifiers (polymers)** | | | | | |
|---|---|---|---|---|---|
| Polyvinyl Acetate | 5 - 15 | 10 - 30 | 15 - 25 | 10 - 20 | 20 - 30 |
| | | | | | |
| **Emulsifiers** | 3-8 | 5- 10 | 3-8 | 0-2 | 2-5 |
| Triacetin | | | | | |
| Glycerol Monostearate | | | | | |
| Lecithin | | | | | |
| | | | | | |

| **Fillers** | | | | | |
|---|---|---|---|---|---|
| Calcium Carbonate | 30 - 40 | 0 - 10 | 10 - 20 | | |
| Talc | | | | 10 - 20 | 10 - 20 |
| | | | | | |
| **Totals:** | 100 | 100 | 100 | 100 | 100 |

Processing: The elastomers are added to a preheated kettle and mixed for 3 minutes under high shear until the mass reaches a temperature of 190 °F. The fillers are then added and mixed for 3 minutes. Rosin esters are slowly added next and mixing continues for 5 minutes. Then, the following materials are added in sequence while mixing is continued: polyvinyl acetate, waxes, hydrogenated vegetable oils, and emulsifiers. The final mass is blended for about 30 minutes. The final temperature should range from 160 °F to 210 °F. The completed gum base can then be discharged from the kettle stored and/or further processed with additional components.

**Table 11 Examples 24-28; Chewing Gums**

| **Ingredient** | **24** | **25** | **26** | **27** | **28** |
|---|---|---|---|---|---|
| Sucrose | 30%-70% | 0 | 25%-50% | 30%-70% | 0 |
| Corn Syrup | 10%-30% | 0 | 10-30% | 10%-30% | 0 |
| Hydrogenated Starch Hydrolysates | 0 | 0.1-10% | 0.1-10% | | 0.1-10% |
| Sorbitol | 0 | 30%-70% | 10%-30% | | 30%-70% |
| Erythritol | 0 | 0-20% | 1%-10% | | 0-20% |
| Xylitol | 0 | 0-20% | 0-10% | | 0-20% |
| Maltitol | 0 | 0-60% | 0-30% | | 0-60% |
| Isomalt | 0 | 0-20% | 0-10% | | 0-20% |
| Gum Base | 40%-60% | | | | |
| from Example 19 | | | | | |
| Gum Base from Example 20 | | 10%-30% | | | |
| Gum Base from Example 21 | | | 20%-50% | | |
| Gum Base from Example 22 | | | | 30%-60% | |
| Gum Base from Example 23 | | | | | 10%-30% |
| Glycerin | 0.1-1% | 0.1-15% | 0.1-5% | 0.1-1% | 0.1-1% |
| Lecithin | 0.1-10% | 0.1-10% | 0.1%-10% | 0.1-10% | 0.1-10% |
| Vegetable Oil | 5-15% | 0.1-5% | 0.1-10% | 0.1-5%% | 5-15% |
| Flavor | 2-5% | 0.1-3% | 0.1-3% | 0.1-3% | 2-5% |
| Acid(s) | 0.1-10% | 0.1-10% | | 0.1-10% | |
| Color(s) | 0.001-0.2% | 001-0.2% | 0.001-0.2% | 0.001-0.2% | 0.001-0.2% |
| Free Sweetener | | 0.1-0.5 | 0.01%-0.2 | | 0.01%-0.2 |
| Encapsulated Sweetener | | 0.5-7% | 0.1%-2% | | 0.1%-2% |
| Cooling Compounds | 0.01-0.25% | 0.1-0.25% | 0.1-0.25% | 0.01-0.25% | 0.1-0.25% |

Processing: Gum base is placed into a mixer and heated to a temperature of about 65°C. The liquid and powdered bulk sweeteners are added with mixing and mixing proceeds until a homogeneous mass is achieved. Minor ingredients including flavor(s), acid(s), color(s) and sweetener(s) are added during the final 5-10 minutes of mixing. The thoroughly mixed batch is then discharged from the mixer.

**Table 12 Examples 29-33; Candy Gum with Gum Base**

| **Ingredient** | **29** | **30** | **31** | **32** | **33** |
|---|---|---|---|---|---|
| **Saccharide Component:** | | | | | |
| Sucrose | 30%-60% | | 30-50% | | 30%-60% |
| Corn Syrup | 60%-30% | | 30-50% | | 60%-30% |
| Water | 10%-20% | 10-30% | 10-25% | 10-30% | 10%-20% |
| Fat | 0%-8% | | 0%-8% | | 0%-8% |
| Hydrocolloids | | | 0%-8% | | 0%-8% |
| Lecithin | 0%-0.8% | 0%-1% | 0%-1% | | 0%-1% |
| GMS | 0%-0.8% | 0%-1% | 0%-1% | | 0%-1% |
| Hydrogenated Starch Hydrolysates | | 0%-10% | | | |
| Sorbitol | | 0%-60% | | | |
| Erythritol | | | 0%-15% | 0%-25% | |
| Xylitol | | | | 0%-60% | |
| Maltitol | | | | 0-60% | |
| Isomalt | | 0%-80% | 0%-30% | | |
| Mannitol | | 0-25% | | | |
| Flavor | 0.5%-5.0% | 0.3%-2.5% | 0.3%-2.5% | 0.3%-2.5% | 0.3%-3.0% |
| Acid(s) | 0.1-3.0% | | 0.1-3.0% | | 0.1-3.0% |
| Color(s) | 0% -0.2% | 0% -0.2% | 0% -0.2% | 0% -0.2% | 0% -0.2% |
| Free Sweetener | | 0.1%-0.5% | 0.01-0.2% | 0.01-0.2% | |
| Encapsulated Sweetener | | 0.5%-7% | 0.1%-2% | 0.1%-2% | 0.1%-2% |
| Cooling Compounds | 0.01-0.25% | | 0.01-0.25% | | 0.01-0.25% |

| **Elastomeric Component:** | | | | | |
|---|---|---|---|---|---|
| Gum Base from Example 19 | 5%-40% | | | | |
| Gum Base from Example 20 | | 5%-40% | | | |
| Gum Base from Example 21 | | | 5%-40% | | |
| Gum Base from Example 22 | | | | 5%-40% | |
| Gum Base from Example 23 | | | | | 5%-40% |

### Candy Making Processing:

Weigh formula portions of sugar, corn syrup, Sorbitol, Xylitol, Erythritol, maltitol, Mannitol, Isomalt, and water. Start Boiling. While boiling add fat, Lecithin, GMS and mix. Boil to cook temperature. Add Hydrocolloid in pre-dissolved slurry format. Add flavors, acids, and colors.

### Candy Gum Making Processing:

Heat gum base until molten. Mix cooked candy with Gum base at 70°-110 °C. Temper on belt or table. Pull /knead. Form by chain die, rotary die, drop roller, or cut and wrap. The compositions can also be formed using confectionery processes to produce shapes including candy canes, sticks, tubes, slabs, laminates, multilayered products, multi-region products, chunls, center-fill products, molded products, etc.

**Table 13 Examples 34-36; Candy Gum with Chewing Gum**

| | **% w/w** | | | | |
|---|---|---|---|---|---|
| **Ingredient** | **34** | **35** | **36** | **37** | **38** |
| **Saccharide Component:** | | | | | |
| Sucrose | 10%-35% | | 10%-30% | | 30%-60% |
| Corn Syrup | 2%-18% | | 10%-30% | | 60%-30% |
| Water | 3%-12% | 3%-18% | 3%-15% | 10-30% | 10%-20% |
| Fat | 0%-5% | | 0%-5% | | 0%-5% |
| Hydrocolloids | | | 0%-5% | | 0%-5% |
| Lecithin | 0%-0.5% | 0%-0.6% | 0%-0.6% | | 0%-0.6% |
| GMS | 0%-0.5% | 0%-0.6% | 0%-0.6% | | 0%-0.6% |
| Hydrogenated Starch Hydrolysates | | 0%-6% | | | |
| Sorbitol | | 0%-35% | | | |
| Erythritol | | | 0%-9% | 0%-25% | |
| Xylitol | | | | 0%-60% | |
| Maltitol | | | | 0-60% | |
| Isomalt | | 0%-50% | 0%-18% | | |
| Mannitol | | 0-15% | | | |
| Flavor | 0.15%-3.0% | 0.1%-1.5% | 0.1%-1.5% | 0.1%-1.5% | 0.3%-3.0% |
| Acid(s) | 0.1%-1.5% | | 0.1%-1.5% | | 0.1%-1.5% |
| Color(s) | 0%-0.15% | 0%-0.12% | 0%-0.12% | 0%-0.12% | 0%-0.12% |
| Free Sweetener | | 0.1%-0.5% | 0.01-0.2% | 0.01-0.2% | |
| Encapsulated Sweetener | | 0.5%-7% | 0.1%-2% | 0.1%-2% | 0.1%-2% |
| Cooling Compounds | 0.01-0.25% | | 0.01-0.25% | | 0.01-0.25% |

| **Elastomeric Component:** | | | | | |
|---|---|---|---|---|---|
| Gum Base from Example 19 | | | | | |
| Gum Base from Example 20 | | | | | |
| Gum Base from Example 21 | | | | | |
| Gum Base from Example 22 | | | | | 1%-5% |
| Gum Base from Example 23 | | | | 1%-5% | |
| | | | | | |
| Chewing Gum from Example 24 | 50%-80% | | | | |
| Chewing Gum from Example 25 | | 50%-80% | | | |
| Chewing Gum from Example 26 | | | 50%-80% | | |
| Chewing Gum from Example 27 | | | | 48%-75% | |
| Chewing Gum from Example 28 | | | | | 48%-75% |
| | | | | | |
| Totals: | 100 | 100 | 100 | 100 | 100 |

### Candy Making Processing:

Weigh formula portions of sugar, corn syrup, Sorbitol, Xylitol, Erythritol, maltitol, Mannitol, Isomalt, and water. Start Boiling. While boiling add fat, Lecithin, GMS and mix. Boil to cook temperature. Add Hydrocolloid in pre-dissolved slurry format. Add flavors, acids, and colors.

### Candy Gum Making Processing:

Heat gum base until molten. Mix cooked candy with Gum base at 70°-110 °C. Fold in chewing gum. Temper on belt or table. Pull/knead. Form by chain die, rotary die, drop roller, or cut and wrap. The compositions can also be formed using confectionery processes to produce shapes including candy canes, sticks, tubes, slabs, laminates, multilayered products, multi-region products, chunks, center-fill products, molded products, etc.

The next sets of examples are directed to embodiments where the saccharide component comprises a soft candy composition. Examples 400-470 are directed to various soft candy saccharide components and Examples 500-570 shown in Table 5 are directed to candy gum compositions including those soft candy compositions.

### Example 39 - Caramel Soft Candy

| | | |
|---|---|---|
| Water | 9.43% | w/w |
| White granular sugar | 14.12% | |
| Brown sugar | 14.12% | |
| Glucose syrup | 24.16% | |
| Sweet condensed milk | 25.73% | |
| Hydrogenated vegetable fat | 11.29% | |
| Glyceryl monostearate | 0.71% | |
| Salt | 0.44% | |

The ingredients are mixed together and heated slowly until thoroughly dissolved and mixed. Heating is continued with mixing until a final temperature of 118°C for soft caramels, 121°C for medium caramels and 128°C for hard caramels is reached. The mass is then discharged from the cooker and further processed for mixing with the gum portion.

### Example 40 - Fudge Soft Candy

| | | |
|---|---|---|
| Sweet Condensed Milk | 41.36% | w/w |
| Butter | 11.69% | |
| Granulated Sugar | 19.65% | |
| Semisweet Chocolate | 25.85% | |
| Glucose Syrup | 1.03% | |
| Vanilla | 0.42% | |

The sweet condensed milk, sugar and butter are combined in a steam jacketed kettle and heated with stirring until the temperature reaches 150°F. Vanilla is added during this mixing step. Heating continues until the mass reaches a temperature of 238°F at which point the steam is turned off and the chocolate is added with vigorous stirring. Next, the glucose syrup is added with stirring. The mass is cooled with stirring until it reaches a temperature of 180-190°F. The mass can then be further processed for mixing with the gum portion, including a tempering stage whereby the mass is cooled to the appropriate temperature for mixing with the gum portion.

### Example 41 - Truffle Soft Candy

| | | |
|---|---|---|
| Fondant | 73.50% | w/w |
| Cocoa liquor | 8.80% | |
| Sweet condensed milk | 17.70% | |
| Vanilla | to taste | |

The fondant is melted at 60-63C and the cocoa liquor is stirred into the melted fondant. In a separate vessel, the sweet condensed milk is heated to 93°C with stirring to prevent scorching and held for 15 minutes. Next, the heated milk is added to the fondant/cocoa liquor mixture and mixed well. The truffle mass can then be further processed for mixing with the gum portion.

### Example 42 - Marshmallow Soft Candy

| | | |
|---|---|---|
| Gelatin | 2.03% | w/w |
| Water | 9.44% | |
| Egg albumen | 0.67% | |
| Water | 4.06% | |
| Sugar | 37.92% | |
| Glucose syrup | 16.25% | |
| Water | 13.50% | |
| Invert sugar | 16.13% | |
| Flavor | to taste | |

The gelatin is soaked in the first quantity of water and then dissolved by slowly warming the mixture. In a separate vessel, the egg albumen is likewise soaked in the second quantity of water and dissolved by slowly warming the mixture. The gelatin and egg albumen solutions are then mixed together. Separately, the sugar, glucose syrup and third quantity of water are heated together to dissolve and then cooked to 112°C. The invert sugar is then added to the cooked sugar solution and cooled to 71°C. The sugars are then added to the mixed gelatin/egg albumen and aerated to a density of 0.40 to 0.50. The aerated mass is then further processed for mixing with the gum portion.

### Example 43 - Chewy Nougat Soft Candy

| | | |
|---|---|---|
| Egg albumen | 0.37% | w/w |
| Water | 3.13% | |
| Sugar | 6.59% | |
| Water | 2.00% | |
| Sugar | 36.63% | |
| Glucose syrup | 36.63% | |
| Water | 14.65% | |

The egg albumen is dissolved in the first quantity of water while the first quantity of sugar is dissolved in the second quantity of water. The egg albumen and sugar solution are mixed together and aerated. In a separate vessel, the second quantity of sugar is dissolved in the third quantity of water and the glucose syrup is added with mixing. This sugar solution is then boiled to 141°C. The boiled sugar solution is then added to the whipped egg albumen/sugar solution in a thin stream. The mass is then further processed for mixing with the gum portion.

### Example 44 - Starch Jellies Soft Candy

| | | |
|---|---|---|
| Sugar | 18.84% | w/w |
| Glucose syrup | 23.34% | |
| Invert syrup | 4.50% | |
| Water | 23.63% | |
| Thin boiling starch | 6.04% | |
| Water | 23.63% | |
| Citric acid | 0.02% | |
| Flavor | to taste | |
| Color | as needed | |

The sugar is dissolved in the first quantity of water and mixed together with the glucose syrup and invert sugar and brought to a boil. In a separate vessel, a starch slurry is prepared by mixing the starch with the second quantity of water (cold). The starch slurry is added to the boiling sugar solution in a thin stream with mixing. The mixture is cooked until it reaches 76 -78% solids. The mass is then further processed for mixing with the gum portion.

### Example 45 - Gelatin Jellies Soft Candy

| | | |
|---|---|---|
| Sugar | 42.00% | w/w |
| Glucose syrup | 30.25% | |
| Water | 16.80% | |
| Gelatin | 5.37% | |
| Water | 5.37% | |
| Citric acid | 0.84% | |
| Water | 0.84% | |
| Flavor | to taste | |
| Color | as needed | |

The sugar and glucose syrup are dissolved together in the first quantity of water and boiled to 115°C. Separately, the gelatin is soaked in the second quantity of water and then warmed to dissolve the gelatin. The sugar solution is cooled to 80°C and the gelatin is added to the sugar. Lastly, the citric acid (dissolved in the third quantity of water) is added to the mass along with flavor and color. The product can then further processed for mixing with the gum portion.

### Example 46 - Sugar free Chewy Candy

| | |
|---|---|
| Maltitol | 25-35%w/w |
| Polydextrose | 25-35% |
| Soluble Fiber | 5-10% |
| Sucralose | 0.001-0.05% |
| Vegetable Oil | 3-8% |
| Lecithin | 0.5-1.2% |
| Gelatin | 1-5% |
| Food Acid | 5-15% |
| Food Color | as needed |
| Flavor | 0.5-2.3% |
| Maltitol Fondant | 1.5-9% |

The maltitol, polydextrose, and soluble fiber are mixed together with enough water to dissolve the solids. The mixture is then heated to boiling when the vegetable oil and sucralose are added. Heating is continued until a temperature of 130°C is reached. With the heat off, the pre-hydrated gelatin, acid, and color are added and mixed for one minute. The mixture is transferred to a tempering table set for 65°F at which point the flavor and fondant are folded in. The mixture can then be further processed for mixing with the gum portion.

**Table 14 Examples 47 - 54; Candy Gum with Soft Candy Saccharide Components**

| | **% w/w** | | | | | | | |
|---|---|---|---|---|---|---|---|---|
| **Ingredient** | **47** | **48** | **49** | **50** | **51** | **52** | **53** | **54** |
| **Saccharide Component:** | | | | | | | | |
| Sucrose | | | 10-15 | | | | 5-10 | |
| Sorbitol | | | | | | | | 3-8 |
| Isomalt | | 5-10 | | | 5-30 | | | 3-8 |
| Soft Candy from Example 39 | 20-80 | | | | | | | |
| Soft Candy from Example 40 | | 10-20 | | | | | | |
| Soft Candy from Example 41 | | | 25-50 | | | | | |
| Soft Candy from Example 42 | | | | 10-70 | | | | |
| Soft Candy from Example 43 | | | | | 30-90 | | | |
| Soft Candy from Example 44 | | | | | | 30-90 | | |
| Soft Candy from Example 45 | | | | | | | 30-90 | |
| Soft Candy from Example 46 | | | | | | | | 20-80 |

| Elastomeric Component: | | | | | | | | |
|---|---|---|---|---|---|---|---|---|
| Gum Base from Example 19 | | | 30-50 | | | | | |
| Gum Base from Example 20 | | 50-10 | | | | 50-10 | | |
| Gum Base from Example 21 | | | | | | | | 30-70 |
| Gum Base from Example 22 | | | | | 30-70 | | | |
| Gum Base from Example 23 | 15-70 | | | | | | | |
| | | | | | | | | |
| Chewing Gum from Example 24 | 5-8 | | | | | | | |
| Chewing Gum from Example 25 | | | | | | | 25-75 | |
| Chewing Gum from Example 26 | | | | 30-90 | | | | |
| Chewing Gum from Example 27 | | | | | | 10-70 | | |
| Chewing Gum from Example 28 | | 15-70 | | | | | | |
| | | | | | | | | |
| Totals: | 100 | 100 | 100 | 100 | 100 | 100 | 100 | 100 |
| | | | | | | | | |

### Candy Gum Making Processing:

Heat gum base (if any) until molten. Mix Soft Candy with Gum base (if any) at 70°-110 °C. Fold in Chewing Gum (if any). Temper on belt or table. Pull /knead. Form by chain die, rotary die, drop roller, or cut and wrap.

The next set of examples is directed to embodiments where candy gum compositions are used to prepare various confectionery formats such as coated formats, center filled formats, layered formats, laminated formats, and granulated formats.

### Center Fill Examples

Example 55 - Cooling in Candy Gum Portion and Warming in Gelatin Bead Center

| **Candy Gum composition containing Encapsulated Menthol** | |
|---|---|
| | |

| **Ingredient** | **Weight percent** |
|---|---|
| | |
| Candy Gum from Example 31 | 93.00 |
| Encapsulated Menthol | 3.00 |
| | |
| **Total** | **100.00** |
| | |

| Procedure: Candy Gum is prepared as in Example 31 with the encapsulated menthol being added to the candy gum mixture during tempering. The thoroughly mixed candy gum is then introduced into a feeder for a nozzle other than the innermost nozzle of a multiple nozzle extruder. | |
|---|---|
| | |

| **Gelatin bead center fill composition with warming** | |
|---|---|
| | |

| **Ingredient** | **Weight percent** |
|---|---|
| | |

| *Capsule Film Material:* | |
|---|---|
| Gelatin | 15.00 |
| Water | 80.00 |
| Glycerin | 5.00 |
| **Total** | **100.00** |
| | |

| *Capsule Filler Material:* | |
|---|---|
| Flavor | 35.00 |
| Vegetable Oil | 35.00 |
| Sugar | 29.95 |
| Capsaicin | 0.05 |
| **Total** | **100.00** |
| | |
| Procedure: As described in US 4,426,337, gelatin beads can be prepared by mixing the capsule-film solution in one tank and mixing the capsule filler material in a second tank. Using equipment with concentrically aligned coaxial conduits, the capsule-film material is fed through an outer conduit while the capsule filler material is fed through the center conduit and both conduits feed the materials into a cooling liquid where the final capsules are formed. The conduit flow rates are configured to create a finished capsule with 80% filler material and 20% capsule film material. | |
| | |
| The gelatin beads are introduced into the center region of the candy gum by feeding them through the innermost nozzle of a multiple extruder. The beads are metered through the nozzle to provide a finished product with 20% center fill material. | |

### Example 56 - Spice Flavor in Candy Gum Portion and Indulgent Flavor in Milk Chocolate Center

| **Cinnamon candy gum composition containing multiple encapsulated Sucralose/polyvinyl acetate matrix. (Slowest release sucralose encapsulation).** | |
|---|---|
| | |

| **Composition:** | |
|---|---|
| **Ingredient** | **Weight percent** |
| | |
| Candy Gum from Example 54 | 95.25 |
| Cinnamon Flavor | 1.90 |
| Sucralose/polyvinyl acetate matrix | 2.85 |
| | |
| **Total** | **100.00** |
| | |
| Procedure: Candy Gum is prepared as in Example 54 with the cinnamon flavor being added to the sugar free nougat composition and the sucralose encapsulation being added to the candy gum composition during tempering. The thoroughly mixed candy gum is then introduced into a feeder for a nozzle other than the innermost nozzle of a multiple nozzle extruder. | |
| | |

| **Milk Chocolate center** | |
|---|---|
| | |

| **Ingredient percent** | **Weight** |
|---|---|
| | |

| *Milk Crumb:* | |
|---|---|
| Cocoa liquor | 13.50 |
| Sugar | 53.50 |
| Milk solids | 32.00 |
| | |
| The milk solids and sugar are kneaded together with the cocoa liquor such that controlled crystallization can occur. The crumb is then dried to the desired final moisture content. Drying can involve vacuum drying alone or drying can occur in combination with drum driers. | |

| **Ingredient percent** | **Weight** |
|---|---|
| | |

| *Milk Chocolate:* | |
|---|---|
| Milk crumb | 84.40 |
| Cocoa butter | 15.00 |
| Lecithin | 0.50 |
| Caramel Flavor | 0.10 |
| | |
| The ingredients are mixed in either a continuous or batch system until thoroughly blended and then refined until a desired consistency and particle size are reached. Refiners can include a series of rollers that use shear forces to break up the sugar and cocoa particles. The refined mass is then further agitated in a conch. Lastly, the milk chocolate is tempered, molded and cooled. | |
| | |
| The milk chocolate is introduced into the center region of the candy gum by feeding the milk chocolate through the innermost nozzle of a multiple extruder. The milk chocolate is metered through the nozzle to provide a finished product with 5% center fill material. | |

### Example 57 - Fruit Flavor in Chewing Gum Portion and Fruit Flavor Potentiator in Starch Jelly Center

| **Candy gum composition containing Encapsulated Citric Acid - Polyvinyl acetate Matrix** | |
|---|---|
| | |

| **Ingredient** | **Weight percent** |
|---|---|
| | |
| Candy Gum from Example 52 | 96.00 |
| Encapsulated Citric Acid | 4.00 |
| | |
| **Total** | **100.00** |
| | |
| Procedure: Candy Gum is prepared as in Example 52 with the encapsulated citric acid being added to the candy gum composition during tempering. The thoroughly mixed candy gum is then introduced into a feeder for a nozzle other than the innermost nozzle of a multiple nozzle extruder. | |
| | |

| Starch Jelly Center with Inosine Monophosphate (IMP) | |
|---|---|
| **Ingredient** | **Weight percent** |
| Sugar | 18.84 |
| Glucose syrup | 23.34 |
| Invert syrup | 4.50 |
| Water | 23.63 |
| Thin boiling starch | 6.04 |
| Water | 23.17 |
| Citric acid | 0.02 |
| IMP | 0.46 |
| **Total** | **100.00** |
| | |
| The sugar is dissolved in the first quantity of water and mixed together with the glucose syrup and invert sugar and brought to a boil. In a separate vessel, a starch slurry is prepared by mixing the starch with the second quantity of water (cold). The starch slurry is added to the boiling sugar solution in a thin stream with mixing. The mixture is cooked until it reaches 76 - 78% solids. | |
| | |
| The starch jelly is introduced into the center of the candy gum by feeding the mass through the inner most nozzle of a multiple nozzle extruder. Alternatively, the starch jelly mass can be cast into starch and allowed to set up prior to introducing into the chewing gum via the inner most nozzle of a multiple nozzle extruder. The starch jelly is metered through the inner most nozzle to provide a finished product with 8% center. | |

### Example 58 - First Fruit Flavor in Candy Gum Portion and Second Complementary Fruit Flavor in Chewy Nougat Center

| **Candy gum composition containing Encapsulated Citric Acid - Polyvinyl acetate Matrix** | |
|---|---|
| | |

| **Ingredient** | **Weight percent** |
|---|---|
| | |
| Candy Gum from Example 50 | 96.00 |
| Encapsulated Citric Acid | 4.00 |
| | |
| **Total** | **100.00** |
| | |
| Procedure: Candy Gum is prepared as in Example 50 with the encapsulated citric acid being added to the candy gum mixture during tempering. The thoroughly mixed candy gum is then introduced into a feeder for a nozzle other than the innermost nozzle of a multiple nozzle extruder. | |
| | |

| Chewy Nougat with Kiwi Flavor | |
|---|---|
| **Ingredient** | **Weight percent** |
| Egg albumen | 0.37 |
| Water | 3.13 |
| Sugar | 6.59 |
| Water | 2.00 |
| Sugar | 35.73 |
| Glucose syrup | 35.73 |
| Water | 14.65 |
| Kiwi Flavor | 1.80 |
| | |
| **Total** | **100.00** |
| | |
| The egg albumen is dissolved in the first quantity of water while the first quantity of sugar is dissolved in the second quantity of water. The egg albumen and sugar solution are mixed together and aerated. In a separate vessel, the second quantity of sugar is dissolved in the third quantity of water and the glucose syrup is added with mixing. This sugar solution is then boiled to 141°C. The boiled sugar solution is then added to the whipped egg albumen/sugar solution in a thin stream. | |
| | |
| The chewy nougat is introduced into the center of the candy gum by feeding the mass through the inner most nozzle of a multiple nozzle extruder. Alternatively, the chewy nougat mass can be poured onto a cooling table and cut prior to introducing into the chewing gum via the inner most nozzle of a multiple nozzle extruder. The chewy nougat is metered through the inner most nozzle to provide a finished product with 25% center. | |

### Example 59 - First Mint Flavor in Candy Gum Portion and Second Mint Flavor of a Different Variety in Dark Chocolate Center

| **Candy gum composition containing Encapsulated Spray Dried Peppermint Flavor** | |
|---|---|
| | |

| **Ingredient** | **Weight percent** |
|---|---|
| | |
| Candy Gum from Example 33 | 94.00 |
| Encapsulated Spray Dried Peppermint Flavor (from Example 7) | 6.00 |
| | |
| **Total** | **100.00** |
| | |
| Procedure: Candy Gum is prepared as in Example 33 with the encapsulated spray dried peppermint flavor being added to the candy gum composition during tempering. The thoroughly mixed candy gum is then introduced into a feeder for a nozzle other than the innermost nozzle of a multiple nozzle extruder. | |
| | |

| **Dark Chocolate center** | |
|---|---|
| | |

| **Ingredient percent** | **Weight** |
|---|---|
| | |
| Sucrose | 43.25 |
| Cocoa mass | 43.25 |
| Cocoa butter | 12.30 |
| Lecithin | 0.50 |
| Eucalyptus flavor | 0.70 |
| | |
| The ingredients are mixed in either a continuous or batch system until thoroughly blended and then refined until a desired consistency and particle size are reached. Refiners can include a series of rollers that use shear forces to break up the sugar and cocoa particles. The refined mass is then further agitated in a conch. Lastly, the dark chocolate is tempered, molded and cooled. | |
| | |
| The dark chocolate is introduced into the center region of the candy gum by feeding the dark chocolate through the innermost nozzle of a multiple extruder. The dark chocolate is metered through the nozzle to provide a finished product with 5% center fill material. | |

### Example 60 - Sweet Taste in Candy Gum Portion and Sour Taste in Fondant Center

| **Candy gum composition containing Encapsulated Glycyrrhizin** | |
|---|---|
| | |

| **Ingredient** | **Weight percent** |
|---|---|
| | |
| Candy Gum from Example 53 | 98.90 |
| Encapsulated Glycyrrhizin | 1.10 |
| | |
| **Total** | **100.00** |
| | |
| Procedure: Candy Gum is prepared as in Example 53 with the encapsulated glycyrrhizin being added to the candy gum mixture during tempering. The thoroughly mixed candy gum is then introduced into a feeder for a nozzle other than the innermost nozzle of a multiple nozzle extruder. | |
| | |

| **Fondant Center with Sour Taste** | |
|---|---|
| | |

| **Ingredient** | **Weight percent** |
|---|---|
| Sugar | 58.81 |
| Glucose syrup | 14.49 |
| Water | 25.00 |
| Encapsulated acid blend | 1.20 |
| Citric Acid | 0.50 |
| | |
| **Total** | **100.00** |
| | |
| The sugar and glucose syrup are added to water and dissolved. The solution is boiled until it reaches 117°C or about 88% solids. The evaporated syrup is then agitated while cooling to induce rapid crystallization. The encapsulated acid blend and the citric acid are adding near the end of the crystallization process. | |
| | |
| The fondant is introduced into the center region of the candy gum by feeding the fondant through the innermost nozzle of a multiple extruder. The fondant is metered through the nozzle to provide a finished product with 12% center fill material. | |

### Example 61 - Bitter Taste in Candy Gum Portion and Astringent Taste in White Chocolate Center

| **Candy gum composition containing 15% Naringin (Bitter Taste) Grapefruit Flavor** | |
|---|---|
| | |

| **Ingredient** | **Weight percent** |
|---|---|
| | |
| Candy Gum from Example 33 | 96.33 |
| Grapefruit Flavor with 15% naringin | 3.67 |
| | |
| **Total** | **100.00** |
| | |
| Procedure: Candy Gum is prepared as in Example 33 with the grapefruit flavor with 15% naringin being added to the candy gum mixture during tempering. The thoroughly mixed candy gum is then introduced into a feeder for a nozzle other than the innermost nozzle of a multiple nozzle extruder. | |
| | |

| **White Chocolate center with Quinine (Astringent Taste)** | |
|---|---|
| | |

| **Ingredient percent** | **Weight** |
|---|---|
| | |
| Cocoa butter equivalent | 26.45 |
| Whole milk powder | 25.00 |
| Sugar | 48.00 |
| Lecithin | 0.50 |
| Quinine | 0.05 |
| | |
| The ingredients are mixed in either a continuous or batch system until thoroughly blended and then refined until a desired consistency and particle size are reached. Refiners can include a series of rollers that use shear forces to break up the sugar and cocoa particles. The refined mass is then further agitated in a conch. Lastly, the dark chocolate is tempered, molded and cooled. | |
| | |
| The white chocolate is introduced into the center region of the candy gum by feeding the white chocolate through the innermost nozzle of a multiple extruder. The white chocolate is metered through the nozzle to provide a finished product with 5% center fill material. | |

### Example 62 - Breath Freshening in the Candy Gum Portion and Whitening in the Gasified Candy Center

| **Candy gum composition containing Encapsulated Zinc Citrate** | |
|---|---|
| | |

| **Ingredient** | **Weight percent** |
|---|---|
| | |
| Candy Gum from Example 32 | 96.00 |
| Encapsulated Zinc Citrate | 4.00 |
| **Total** | **100.00** |
| | |
| Procedure: Candy Gum is prepared as in Example 32 with the encapsulated zinc citrate being added to the candy gum mixture during tempering. The thoroughly mixed gum is then introduced into a feeder for a nozzle other than the innermost nozzle of a multiple nozzle extruder. | |
| | |

| **Gasified Candy with Sodium Stearate** | |
|---|---|
| | |

| **Ingredient** | **Weight percent** |
|---|---|
| | |
| Isomalt | 57.50 |
| Sorbitol Solution | 37.50 |
| Sodium Stearate | 5.00 |
| Flavor and color | to taste |
| | |
| As described in U.S. Patent Number 4,289,794, the isomalt and sorbitol solution are mixed together and cooked to a temperature of about 280°F. Additives including sodium stearate, flavor, and color are then added. The cooked candy is gasified by introducing carbon dioxide gas at superatmospheric temperature into a closed vessel containing the cooked candy at 500-700 psi of pressure. The mixture is stirred for two to six minutes to incorporate the gas. The gasified candy is then allowed to solidify in a cooling tube. Once solid, the pressure is released causing the candy to fracture. The fractured, gasified candy can then be sized and fed into the inner most nozzle of a multiple nozzle extruder. | |
| | |
| The gasified candy is introduced into the center region of the candy gum by feeding the gasified candy through the innermost nozzle of a multiple extruder. The gasified candy is metered through the nozzle to provide a finished product with 10% center fill material. | |

### Example 63 - Breath Freshening in the Candy Gum Portion and Remineralization in a Powdered Center

| **Candy gum composition containing Chlorophyll** | | |
|---|---|---|
| | | |

| **Ingredient** | | **Weight percent** |
|---|---|---|
| | | |
| Candy Gum from Example 35 | | 99.50 |
| Chlorophyll | | 0.50 |
| | | |
| **Total** | | **100.00** |
| Procedure: Candy Gum is prepared as in Example 35 with the chlorophyll being added to the candy gum mixture during tempering. The thoroughly mixed candy gum is then introduced into a feeder for a nozzle other than the innermost nozzle of a multiple nozzle extruder. | | |
| | | |

| Powdered Center with casein phosphopeptide-amorphous calcium phosphate (CPP-ACP), | | |
|---|---|---|
| | | |

| **Ingredient** | **Weight percent** | |
|---|---|---|
| Erythritol | 70.00 | |
| | | |
| Xylitol | 20.00 | |
| CPP-ACP | 10.00 | |
| | | |
| The powders are dry blended using any suitable means that creates a uniform mixture. | | |
| | | |
| The powder blend is introduced into the center region of the candy gum composition by feeding the powder blend through the innermost nozzle of a multiple extruder. The powder blend is metered through the nozzle to provide a finished product with 10% center fill material. | | |

### Example 64 - Teeth Strengthening in the Candy Gum Portion and Calcium in a Liquid Center

| Candy gum composition containing casein phosphopeptide-amorphous calcium phosphate (CPP-ACP), | | | |
|---|---|---|---|
| | | | |

| **Ingredient** | | **Weight percent** | |
|---|---|---|---|
| | | | |
| Candy Gum from Example 37 | | | 94.00 |
| CPP-ACP | | | 6.00 |
| | | | |
| **Total** | | | **100.00** |
| Procedure: Candy Gum is prepared as in Example 37 with the CPP-ACP being added to the candy gum mixture during tempering. The thoroughly mixed candy gum is then introduced into a feeder for a nozzle other than the innermost nozzle of a multiple nozzle extruder. | | | |
| | | | |

| Liquid Center with calcium | | | |
|---|---|---|---|
| **Ingredient** | **Weight percent** | | |
| Glycerin | 63.00 | | |
| Lycasin™ | 29.26 | | |
| Sorbitol solution | 3.25 | | |
| Sodium carboxymethyl cellulose | 0.08 | | |
| Color | 0.004 | | |
| Flavors | 1.30 | | |
| Cooling agents | 0.06 | | |
| Calcium carbonate | 3.00 | | |
| Intense sweetener | 0.05 | | |
| | | | |
| The liquid fill composition is prepared by first preparing a pre-mix of the sodium carboxymethyl cellulose, glycerine, and polyols. This pre-mix is then combined with the colors, flavors, cooling agents, calcium carbonate, and intense sweeteners and mixed. | | | |
| | | | |
| The candy gum region and liquid-fill compositions are then extruded together and formed into individual pieces. The pieces each have a total weight of approximately 2.2g. In the final pieces, the candy gum region is about 70% by weight and the liquid-fill is about 30% by weight. Optionally, pieces may be coated. | | | |

### Coated Examples

Coating can involve treating the exterior surface of a confectionery composition. Various methods can be used to treat the exterior surface and apply a coating composition. These methods can include panning, extrusion, enrobing, and tumble coating, fold coating, as well as applying a coating composition around a confectionery composition as it proceeds through a rope sizer or batch former.

### Example 65 - Center Filled Candy Gum with Hard Coating

| **Center Filled Candy gum composition with Cooling Agents in the Coating** | |
|---|---|
| | |

| **Ingredient** | **Weight percent** |
|---|---|
| | |
| Center Filled Candy Gum from Example 60 | 67.00 |
| Isomalt | 32.50 |
| Cooling agents | 0.50 |
| | |
| **Total** | **100.00** |
| Procedure: Candy Gum is prepared as in Example 60 and individual pieces are formed into a desired shape such as a pillow or ball shape. The individual pieces are then loaded into a coating pan and the isomalt coating is applied by repeatedly spraying a solution of isomalt onto the candy gum pieces with tumbling and drying air circulation. The cooling agents are added to one or more of the applications of isomalt syrup for incorporation into the coating layer. The coated candy gum pieces are removed from the coating pan once the desired amount of coating has been achieve. | |

### Example 66 - Candy Gum Enrobed with Hard Candy

| **Candy gum composition with Food Acids in the Enrobing Composition** | |
|---|---|
| | |

| **Ingredient** | **Weight percent** |
|---|---|
| | |
| Candy Gum from Example 51 | 67.00 |
| Isomalt | 32.50 |
| Food Acids | 0.50 |
| | |
| **Total** | **100.00** |
| Procedure: Candy Gum is prepared as in Example 51 and individual pieces are formed into a desired shape such as a stick or slab. The individual pieces are then placed onto a mesh conveying element and passed through a bottomer loaded with isomalt syrup. The bottomed pieces are vibrated to remove excess syrup and may be passed through a drying step before being conveyed through an enrober where the bottomed pieces pass through a curtain of isomalt syrup to which the food acids have been added. Excess isomalt syrup is blown off with air or shaken off with vibration and the enrobed pieces are then dried prior to packaging. | |

### Example 67 - Candy Gum Hard Candy Shell

| **Candy gum composition with a Second Flavor in the Candy Shell** | |
|---|---|
| | |

| **Ingredient** | **Weight percent** |
|---|---|
| | |
| Center Filled Candy Gum from Example 57 | 67.00 |
| Sugar | 19.50 |
| Corn Syrup | 13.00 |
| Second Flavor | 0.50 |
| | |
| **Total** | **100.00** |
| Procedure: Candy Gum is prepared as in Example 57 and loaded into a first hopper of a multiple hopper extruder. The sugar and corn syrup are mixed with enough water to dissolve the sugar and then cooked to a desired moisture content. The second flavor is added to the sugar/corn syrup mixture as it cools and the flavored sugar/corn syrup is loaded into a second hopper of the extruder. The candy gum and flavored sugar/corn syrup mixtures are co-extruded to form the finished product. | |

To prepare a confectionery product with a softer texture, texture modifying agents such as fat and hydrocolloids may be included. In Example 18, a fat blend of hydrogenated vegetable fats is added along with a hydrated gelatin blend. To prepare the confectionery product, the sugar and corn syrup are first dissolved in water and heated to 172°C. Separately, the gelatin is dissolved in hot water and added to the hot sugar syrup. Next, the fat is added to the cooked sugar syrup and the mass is placed on a cooling table. Once on the cooling table, fondant, color, and flavor are worked into the candy to form a candy mass. High intensity sweetener can be added to the candy on the cooling table or it can be added to the gum base component that forms an elastomeric portion. The gum base component is heated to 50-65°C and mixed with the candy mass to form a confectionery mass. Lastly, the confectionery mass is shaped into finished product pieces. One method of shaping is to pass the confectionery mass through a drop roller.

### Example 39 - Powdered Candy Gum with Erythritol

| **Ingredient** | **Weight percent** |
|---|---|
| | |
| Candy Gum from Example 30 | 90.00 |
| Erythritol | 10.50 |
| | |
| **Total** | **100.00** |
| Procedure: Candy Gum is prepared as in Example 30 and formed into strands which are ground in a Fitz mill to the desired particle size distribution using ambient conditions. The ground candy gum is then dry-mixed with the erythritol in a ribbon blender. The resulting powder blend can be packaged and consumer as-is or may be used as a powder center fill as in Example 63 above or it can be used on the exterior surface of a confectionery, chewing gum or candy gum product. | |

### Example 40

Solid gum base, amorphous isomalt and other ingredients are mixed and fed into pre-heated (85°C) twin screw Brabender extruder with the following process conditions:
Extruder temperature: 75 °C
Die Temperature: 80 °C
Die Pressure: 400 - 600 psig
Extruder speed: 20 - 35 RPM
Extruder Type: Twin-screw counter rotating.

Gum sticks having glossy and smooth surface with excellent chewing texture can be produced. The gloss measurements on conventional and extruded candy gums are given in Table 15.

**Table 15: Gloss measurements of extruded candy gum and conventional gums.**

| | **Gloss on a 60 degree meter** | **Comments** |
|---|---|---|
| **Extruded Candy Gum** | 48 | |
| **Conventional coated pellet gum** | 51 | |
| **Conventional Gum slab** | 39 | |
| **Eggshell (reference)** | 20-35 | |
| **Semi-gloss (Reference)** | 35-70 | |
| **Full-gloss (reference)** | >70 | Smooth with an almost mirror-like surface |

Obviously, numerous modifications and variations of the present invention are possible in light of the above teachings. It is therefore to be understood that within the scope of the appended claims, the invention may be practiced otherwise than as specifically described herein.

## Claims

1. A multi-layered edible composition comprising a saccharide-containing portion and a chewing gum base, the composition comprising one or more layers of each of the saccharide-containing portion and chewing gum base, wherein the saccharide-containing portion comprises a particulate texture modifying component, wherein the particulate texture modifying component is sucrose, polyols such as sorbitol, xylitol, mannitol, galactitol, lactitol, maltitol, erythritol, isomalt, hydrogenated starch hydrolysates and mixtures thereof, proteins, and combinations thereof.

2. A multi-layered edible composition comprising
a first portion comprising a layer of a saccharide component and having a first initial hardness intensity as measured by sensory evaluation methods, wherein at least a portion of the saccharide component is cooked, and the layer of a saccharide component further comprising a particulate texture modifying component; and
a second portion comprising a layer of an elastomeric component and having a second initial hardness intensity as measured by sensory evaluation methods;
wherein the initial first hardness intensity is similar to the second initial hardness intensity; and wherein the hardness intensity similarity is measured by a hardness intensity difference that is less than about one point on a 15 point sensory evaluation methods scale, wherein the particulate texture modifying component is sucrose, polyols such as sorbitol, xylitol, mannitol, galactitol, lactitol, maltitol, erythritol, isomalt, hydrogenated starch hydrolysates and mixtures thereof, starches, proteins, and combinations thereof.

3. The multi-layered edible composition of claim 1 or 2, wherein the particulate texture modifying component is mannitol.

4. The multi-layered edible composition of claim 1 or claim 2, wherein at least a part of the saccharide-containing portion or first portion is cooked.

5. The multi-layered edible composition of claim 1 or 2, wherein at least one of the saccharide-containing portion and the first portion comprises at least one hydrocolloid.

6. The multi-layered edible composition of claim 5, wherein the hydrocolloid is gelatin or gum arabic.

7. The multi-layered edible composition of claim 1 or 2, wherein at least one of the portions comprises at least one ingredient selected from the group consisting of sensates, flavors, sweeteners, sweetness potentiators, functional ingredients, food acids.

8. The multi-layered edible composition of claim 7, wherein the at least one ingredient is at least partially encapsulated.

9. The multi-layered edible composition of claim 1 or 2, wherein the saccharide-containing portion or first portion comprises maltitol or mannitol.

10. The multi-layered edible composition of claim 1 or 2, wherein the saccharide-containing portion or first portion comprises flavor and color.

11. The multi-layered edible composition of claim 1 or 2, wherein the saccharide-containing portion or first portion comprises fat.

## Patentansprüche

1. Mehrschichtige essbare Zusammensetzung, umfassend einen saccharidhaltigen Anteil und eine Kaugummigrundmasse, wobei die Zusammensetzung jeweils ein oder mehrere Schichten des saccharidhaltigen Anteils und der Kaugummigrundmasse umfasst, wobei der saccharidhaltige Anteil einen partikulären texturmodifizierenden Bestandteil aufweist, wobei es sich bei dem partikulären texturmodifizierenden Bestandteil um Saccharose, Polyole wie Sorbit, Xylit, Mannit, Galactit, Lactit, Maltit, Erythrit, Isomalt, hydrierte Stärkehydrolysate und Gemische derselben, Eiweiße und Kombinationen derselben handelt.

2. Mehrschichtige essbare Zusammensetzung, umfassend
einen ersten Anteil, der eine Schicht aus einem Saccharidbestandteil umfasst und eine mittels Verfahren zur sensorischen Beurteilung gemessene erste Anfangshärteintensität aufweist, wobei mindestens ein Anteil des Saccharidbestandteils gekocht ist, und die Schicht aus einem Saccharidbestandteil darüber hinaus einen partikulären texturmodifizierenden Bestandteil umfasst, und
einen zweiten Anteil, der eine Schicht aus einem elastomeren Bestandteil umfasst und eine mittels Verfahren zur sensorischen Beurteilung gemessene zweite Anfangshärteintensität aufweist,
wobei die anfängliche erste Anfangshärteintensität und die zweite Anfangshärteintensität ähnlich sind, und wobei die Ähnlichkeit der Härteintensität anhand einer Härteintensitätsdifferenz gemessen wird, die weniger als ungefähr einen Punkt auf einer 15-punktigen Skala von Verfahren zur sensorischen Beurteilung beträgt, wobei es sich bei dem partikulären texturmodifizierenden Bestandteil um Saccharose, Polyole wie Sorbit, Xylit, Mannit, Galactit, Lactit, Maltit, Erythrit, Isomalt, hydrierte Stärkehydrolysate und Gemische derselben, Eiweiße und Kombinationen derselben handelt.

3. Mehrschichtige essbare Zusammensetzung nach Anspruch 1 oder 2, wobei es sich bei dem partikulären texturmodifizierenden Bestandteil um Mannit handelt.

4. Mehrschichtige essbare Zusammensetzung nach Anspruch 1 oder 2, wobei mindestens ein Teil des saccharidhaltigen Anteils oder des ersten Anteils gekocht ist.

5. Mehrschichtige essbare Zusammensetzung nach Anspruch 1 oder 2, wobei mindestens der saccharidhaltige Anteil oder der erste Anteil mindestens ein Hydrokolloid umfasst.

6. Mehrschichtige essbare Zusammensetzung nach Anspruch 5, wobei es sich bei dem Hydrokolloid um Gelatine oder Gummi arabicum handelt.

7. Mehrschichtige essbare Zusammensetzung nach Anspruch 1 oder 2, wobei mindestens einer der Anteile mindestens einen Inhaltsstoff umfasst, der aus der Gruppe ausgewählt ist, die aus die Empfindung beeinflussenden Mitteln, Aromen, Süßungsmitteln, Süßkraftverstärkern, funktionellen Inhaltsstoffen, Genusssäuren besteht.

8. Mehrschichtige essbare Zusammensetzung nach Anspruch 7, wobei mindestens ein Inhaltsstoff mindestens teilweise eingekapselt ist.

9. Mehrschichtige essbare Zusammensetzung nach Anspruch 1 oder 2, wobei der saccharidhaltige Anteil oder der erste Anteil Maltit oder Mannit umfasst.

10. Mehrschichtige essbare Zusammensetzung nach Anspruch 1 oder 2, wobei der saccharidhaltige Anteil oder der erste Anteil Aroma und Farbstoff umfasst.

11. Mehrschichtige essbare Zusammensetzung nach Anspruch 1 oder 2, wobei der saccharidhaltige Anteil oder der erste Anteil Fett umfasst.

## Revendications

1. Composition comestible multicouche comprenant une partie contenant un saccharide et une base de gomme à mâcher, la composition comprenant une ou plusieurs couches de chacune de la partie contenant un saccharide et de la base de gomme à mâcher, dans laquelle la partie contenant un saccharide comprend un composant modifiant la texture particulaire, dans laquelle le composant modifiant la texture particulaire est du saccharose, des polyols tels que le sorbitol, le xylitol, le mannitol, le galactitol, le lactitol, le maltitol, l'érythritol, l'isomalt, les hydrolysats d'amidon hydrogénés et les mélanges de ceux-ci, des protéines et leurs combinaisons.

2. Composition comestible multicouche comprenant
une première partie comprenant une couche d'un composant saccharide et ayant une première intensité de dureté initiale telle que mesurée par les procédés d'évaluation sensorielle, dans laquelle au moins une partie du composant saccharide est cuite, et la couche de composant saccharide comprenant en outre un composant modifiant la texture particulaire ; et
une deuxième partie comprenant une couche de composant élastomère et ayant une deuxième intensité de dureté initiale telle que mesurée par les procédés d'évaluation sensorielle ;
dans laquelle la première intensité de dureté initiale est similaire à la deuxième intensité de dureté initiale ; et dans laquelle la similarité d'intensité de dureté est mesurée par une différence d'intensité de dureté qui est inférieure à environ un point sur une échelle des procédés d'évaluation sensorielle à 15 points, dans laquelle le composant modifiant la texture particulaire est du sucrose, des polyols tels que le sorbitol, le xylitol, le mannitol, le galactitol, le lactitol, le maltitol, l'érythritol, l'isomalt, les hydrolysats d'amidon hydrogénés et les mélanges de ceux-ci, des protéines et leurs combinaisons.

3. Composition comestible multicouche selon la revendication 1 ou 2, dans laquelle le composant modifiant la texture particulaire est du mannitol.

4. Composition comestible multicouche selon la revendication 1 ou 2, dans laquelle au moins une partie de la partie contenant un saccharide ou de la première partie est cuite.

5. Composition comestible multicouche selon la revendication 1 ou 2, dans laquelle au moins l'une de la partie contenant un saccharide et de la première partie comprend au moins un hydrocolloïde.

6. Composition comestible multicouche selon la revendication 5, dans laquelle l'hydrocolloïde est de la gélatine ou de la gomme arabique.

7. Composition comestible multicouche selon la revendication 1 ou 2, dans laquelle au moins l'une des parties comprend au moins un ingrédient choisi parmi le groupe comprenant des agents de sensation, des arômes, des édulcorants, des renforçateurs de sucrosité, des ingrédients fonctionnels, des acides alimentaires.

8. Composition comestible multicouche selon la revendication 7, dans laquelle l'au moins un ingrédient est au moins partiellement encapsulé.

9. Composition comestible multicouche selon la revendication 1 ou 2, dans laquelle la partie contenant un saccharide ou la première partie comprend du maltitol ou du mannitol.

10. Composition comestible multicouche selon la revendication 1 ou 2, dans laquelle la partie contenant un saccharide ou la première partie comprend un arôme et un colorant.

11. Composition comestible multicouche selon la revendication 1 ou 2, dans laquelle la partie contenant un saccharide ou la première partie comprend de la graisse.
